# EUROPEAN PATENT APPLICATION

(11) **EP 4 282 424 A1**
(43) Date of publication of application: **29.11.2023**
(21) Application number: 21905583.7
(22) Date of filing: 08.12.2021
(51) Int. Cl.: A61K 38/00, C07D 277/56, C07D 495/04, A61P 11/00, A61P 13/12, A61P 1/16, A61P 9/00

(54) **ANTI-FIBROSIS PHARMACEUTICAL COMPOSITION AND APPLICATION THEREOF**

(30) Priority: 16.12.2020 CN 202011482603
(71) Applicant: West China Second University Hospital, Chengdu, Sichuan 610044 (CN)
(72) Inventor: DING, Bisen, Chengdu, Sichuan 610044 (CN); CAO, Zhongwei, Chengdu, Sichuan 610044 (CN)
(74) Representative: Dolphin, Kirsty Mairi
(86) International application number: PCT/CN2021/136240
(87) International publication number: WO 2022/127654

(57) **Abstract**

An anti-fibrosis pharmaceutical composition and application thereof. The anti-fibrosis pharmaceutical composition specifically comprises: (a) an NRP1 inhibitor and/or an HIF2α inhibitor; and (b) an EPCR pathway activator that promotes the activity of an EPCR pathway. The anti-fibrosis pharmaceutical composition helps reduce organ fibrosis to a certain extent and promotes corresponding organ repair and regeneration.

## Description

### Technical Field

. The present invention belongs to the field of biomedicine, and particularly relates to an anti-fibrosis pharmaceutical composition and application thereof.

### Background

. Fibrosis can occur in a wide range of organs and is the ultimate pathological outcome of many common chronic inflammatory, immune-mediated metabolic diseases, as well as a major cause of morbidity and mortality in these diseases. A variety of noxious stimuli (including toxins, infectious pathogens, autoimmune reactions and mechanical stress) are able to induce a fibrotic cellular response. Fibrosis can affect all tissues of the body, and left unchecked, can result in organ failure and death.

. Fibrosis is a repair response to protect the relative integrity of tissues and organs after tissue damage. In response to tissue damage, myofibroblasts-derived from a number of sources including resident fibroblasts, mesenchymal cells, circulating fibrocytes, and the transdifferentiation of other cell types-initiate a wound healing response by remodeling the extracellular environment to restore tissue integrity and promote the replacement of parenchymal cells. Normally, this pro-fibrotic program is turned off as the tissue heals. However, persistent insult and injury results in dysregulation of this process, leading to pathologically excessive deposition of ECM proteins (including collagen, laminin, and fibronectin) and, in concert with upregulated myofibroblast activity, creates a chronic inflammatory environment with macrophage (and monocyte) and immune cell infiltration. Although this "excessive deposition" repairs the damage, it does not possess the structure and function of parenchymal cells of organs. Instead, it would cause fibrosis and dysfunction of organs.

. Mammalian organs (e.g., liver, lung, and kidney) can undergo facultative regeneration to replace damaged tissues. However, regenerative capacity in most mammals is limited by the aging process. Damaged tissue in aged organs is often replaced by excessive scar, resulting in fibrosis and organ dysfunction. Whereas different mammalian organs possess distinct capacity to undergo self-repair, regenerative capacity seems to be similarly suppressed in aged organs, and these aged organs are all susceptible to fibrosis after injury.

. There are currently many studies on the mechanism of fibrosis. Among them, members of the transforming growth factor-β (TGF-β) family are considered to play a major role in the fibrotic process, and TGF-β is the main pro-fibrotic cytokine. It is believed that many fibrotic diseases are mediated by the TGF pathway, which promotes the differentiation of fibroblasts into myofibroblasts. Myofibroblasts increase the production and secretion of ECM proteins, causing ECM deposition, which leads to structural changes and sclerosis of organs. Integrin is considered to enhance signals from soluble pro-fibrotic growth factors (e.g., TGF-β1). Therefore, targeting integrin and targeting TGF-β are popular research directions. However, continuous systemic inhibition of TGF-β1 and targeting integrin are ineffective in practice due to strong side effects or safety concerns, and the effects of other drugs for the treatment of fibrosis are unsatisfactory.

. Fibrosis has long been considered irreversible. Currently existing drugs for the treatment of fibrosis can only inhibit fibrosis to a certain extent, but are difficult to prevent or reverse fibrosis. Therefore, there is still a need to develop new means that can promote the regeneration of damaged organs while combating fibrosis.

### Summary

. The present invention provides an anti-fibrosis pharmaceutical composition, comprising: (a) an NRP1 inhibitor and/or an HIF2α inhibitor; (b) an EPCR pathway activator, the EPCR pathway activator promotes activity of the EPCR pathway.

. Further, the NRP1 inhibitor comprises one or more of a substance for inhibiting NRP1 protein activity, a substance for degrading NRP1 protein activity, and a genetic tool for reducing NRP1 protein level; the HIF2α inhibitor comprises one or more of a substance for inhibiting HIF2α protein activity, a substance for degrading HIF2α protein activity, and a genetic tool for reducing HIF2α protein level.

. Further, the NRP1 inhibitor comprises EG00229 or a derivative thereof; the HIF2α inhibitor comprises HIF-2α-IN-1 or a derivative thereof.

. Further, the EPCR pathway activator comprises activated protein C.

. Further, the fibrosis comprises organ fibrosis, and the organ fibrosis is optionally aging-related organ fibrosis.

. Further, the organ fibrosis comprises one or more of liver fibrosis, kidney fibrosis, pulmonary fibrosis, and cardiac fibrosis, and the pulmonary fibrosis is optionally fibrosis caused by immunotherapy-induced pneumonitis.

. Further, the genetic tool for reducing NRP1 protein level comprises one or more of RNA interference, microRNA, gene editing, and gene knockout; the genetic tool for reducing HIF2α protein level comprises RNA interference, microRNA, gene editing, and gene knockout.

. Further, the composition further comprises a pharmaceutically acceptable carrier.

. In another aspect, the present invention provides use of a pharmaceutical composition in the preparation of an anti-fibrotic agent, the pharmaceutical composition comprising: (a) an NRP1 inhibitor and/or an HIF2α inhibitor; (b) an EPCR pathway activator, the EPCR pathway activator promotes activity of the EPCR pathway.

. Further, the use comprises reducing fibrosis degree of damaged organ and/or promoting regeneration capacity of damaged organ, the regeneration capacity comprises one or more of cell proliferation capacity, tissue repair capacity, and function restoration capacity; the tissue repair capacity is optionally fibrosis-free repair, and the organ optionally comprises one or more of liver, kidney, lung, and heart.

. Further, the use comprises reducing formation of platelet-macrophage rosette; and/or reducing expression of one or more of platelet IL-1α, SDF1, and TIMP1.

. Further, the EPCR pathway activator is used in combination with an NRP 1 inhibitor and/or an HIF2α inhibitor to prepare the anti-fibrotic agent, and the NRP1 inhibitor and/or HIF2α inhibitor are/is used for promoting expression of EPCR.

. Further, the use comprises reducing Rho pathway activity; and/or reducing deposition of inflammatory monocytes; and/or promoting pro-regenerative function of endothelial niche.

### Beneficial technical effects

. The present invention provides an anti-fibrosis pharmaceutical composition and use thereof. The EPCR (endothelial cell protein C receptor) targeted by the technical solution of the present invention is a transmembrane glycoprotein located on the cell membrane specifically expressed by endothelial cells. Vascular endothelial cells are distributed in almost all organs and are present in large numbers in the organisms. Through a series of experiments, experimenters of the present invention discovered that inhibition of the EPCR pathway is an important node in the transition from organ regeneration to fibrosis. Experimenters of the present invention utilize the interaction among multiple cell types, including vascular endothelial cells, platelets, and macrophages to simultaneously intervene in the upstream of EPCR inhibition (i.e., inhibition of NRP1 and/or HIF2α) and EPCR to upregulate EPCR to a certain extent, thereby inhibiting the downstream of EPCR inhibition (i.e., IL-1α, TIMP1, etc.), which promotes fibrosis-free organ repair to a certain extent. Prior art often only targets a single target and have poor therapeutic effects; while directly targeting integrin or TGF-β results in safety and other problems. Combined with the feature that the circulatory system is readily accessible, the anti-fibrosis pharmaceutical composition provided by the present invention can regulate various molecules (such as EPCR, IL-1α, TIMP1, etc.) produced by multiple cell types (i.e., vascular endothelial cells, platelets, and macrophages in the circulatory system) to regulate fibrosis of damaged organs and promote regeneration of damaged cells from multiple perspectives. The technical solution of the present invention can inhibit pro-fibrotic platelet IL-1α and macrophage TIMP1, and macrophage TIMP1 has been proven to be able to stimulate the activation of fibroblasts through integrins (it can also be understood that integrins are downstream of EPCR). Therefore, the technical solution of the present invention has an obvious therapeutic effect in anti-fibrosis, which is specifically reflected in: reducing the expression of IL-1α, SDF1, and TIMP1, alleviating the pro-fibrotic "platelet-macrophage" rosette, and inhibiting the Rho-activation in the endothelial cells, and reducing deposition of inflammatory monocytes to reduce fibrosis degree of damaged organs and promote regeneration (including parenchymal cell proliferation, tissue repair, function restoration etc.) of damaged organs, and promote fibrosis-free repair of damaged organs. It should be noted that the technical solution of the present invention can further inhibit the activation of fibroblasts by reducing the expression of TIMP1 in macrophages. The above therapeutic effects have been verified in the regeneration and fibrosis models of lung, liver, kidney, and their corresponding aging models.

. Fibrosis has long been considered irreversible. Prior art is often only able to inhibit fibrosis to a certain extent, but is difficult to "reverse fibrosis". By targeting EPCR (i.e., EPCR pathway activator + NRP1 inhibitor and/or HIF2α inhibitor, thereby targeting EPCR), the present invention regulates the expression of EPCR to affect the expression of platelet ILla and macrophage TIMP1 in the circulatory system (i.e., normalization of the aberrant platelet-macrophage-vascular endothelial cell interactions) to inhibit fibrosis and restores regenerative capacity of damaged organs to a certain extent, achieving "reversal of fibrosis" (i.e., fibrosis-free repair of damaged organs, thereby transforming the damaged organ from fibrosis to regeneration). In addition, the pharmaceutical composition provided by the present invention can alleviate immunotherapy-induced pneumonia to a certain extent.

. In summary, the anti-fibrosis pharmaceutical composition and use thereof provided by the technical solution of the present invention can facilitate systematically alleviation or treatment, or even reversal of organ fibrosis to a certain extent.

. In another aspect, the present invention provides use of an EPCR pathway activator in the preparation of an anti-fibrotic agent, the EPCR pathway activator promotes activity of the EPCR pathway.

. Further, the EPCR pathway activator comprises activated protein C.

. Further, the fibrosis comprises organ fibrosis, and the organ fibrosis is optionally aging-related organ fibrosis.

. Further, the organ fibrosis comprises one or more of liver fibrosis, kidney fibrosis, pulmonary fibrosis, and cardiac fibrosis, and the pulmonary fibrosis is optionally fibrosis caused by immunotherapy-induced pneumonitis.

. Further, the use comprises reducing fibrosis degree of damaged organ and/or promoting regeneration capacity of damaged organ, the regeneration capacity comprises one or more of cell proliferation capacity, tissue repair capacity, and function restoration capacity.

. Further, the use comprises reducing formation of platelet-macrophage rosette; and/or reducing expression of one or more of platelet IL-1α, SDF1, and TIMP1.

. Further, the EPCR pathway activator is used in combination with an NRP1 inhibitor and/or an HIF2α inhibitor to prepare the anti-fibrotic agent, and the NRP1 inhibitor and/or HIF2α inhibitor are/is used for promoting expression of EPCR.

. Further, the NRP1 inhibitor comprises one or more of a substance for inhibiting NRP1 protein activity, a substance for degrading NRP1 protein activity, and a genetic tool for reducing NRP 1 protein level; the HIF2α inhibitor comprises one or more of a substance for inhibiting HIF2α protein activity, a substance for degrading HIF2α protein activity, and a genetic tool for reducing HIF2α protein level.

. Further, the NRP1 inhibitor comprises EG00229 or a derivative thereof; the HIF2α inhibitor comprises HIF-2α-IN-1 or a derivative thereof.

. Further, the substance for inhibiting NRP1 protein activity comprises a small molecule drug and/or an antibody; the substance for inhibiting HIF2α protein activity comprises a small molecule drug and/or an antibody.

. Further, the genetic tool for reducing NRP1 protein level comprises one or more of RNA interference, microRNA, gene editing, and gene knockout; the genetic tool for reducing HIF2α protein level comprises RNA interference, microRNA, gene editing, and gene knockout.

. In another aspect, the present invention also provides a marker group for evaluating organ fibrosis, wherein the marker group comprises one or more of NRP1, HIF2α, and EPCR; expression level of the marker group can be used for evaluating fibrosis degree of organ and/or function impairment of organ.

. Further, the marker group further comprises one or more of PIGF, SDF1, IL-1α, CXCR4, and TIMP.

. Further, the organ fibrosis comprises one or more of liver fibrosis, kidney fibrosis, pulmonary fibrosis, and cardiac fibrosis.

. Further, the organ fibrosis is aging-related organ fibrosis.

. In another aspect, the present invention also provides a kit for evaluating organ fibrosis, wherein comprising a marker group, and the marker group comprises one or more of NRP1, HIF2α, and EPCR

. Further, the marker group further comprises one or more of P1GF, SDF1, IL-1α, CXCR4, and TIMP.

. Further, the kit is used for detecting gene expression level of the marker in a biological sample, the biological sample comprises one or more of blood sample, serum sample, plasma sample, tissue sample, organ sample, and cell sample.

. Further, the organ fibrosis comprises one or more of liver fibrosis, kidney fibrosis, pulmonary fibrosis, and cardiac fibrosis.

. Further, the organ fibrosis is aging-related organ fibrosis.

### Beneficial technical effects

. The present invention provides use of an EPCR pathway activator in the preparation of an anti-fibrotic agent. The EPCR (endothelial cell protein C receptor) targeted by the technical solution of the present invention is a transmembrane glycoprotein located on the cell membrane specifically expressed by endothelial cells. Vascular endothelial cells are distributed in almost all organs and are present in large numbers in the organisms. Through a series of experiments, experimenters of the present invention discovered that inhibition of the EPCR pathway is an important node in the transition from organ regeneration to fibrosis. Experimenters of the present invention utilize the interaction among multiple cell types, including vascular endothelial cells, platelets, and macrophages to intervene in the upstream of EPCR inhibition (i.e., inhibition of NRP1 and/or HIF2α) to facilitate upregulation of EPCR to a certain extent, thereby inhibiting the downstream of EPCR inhibition (i.e., IL-1α, TIMP1, etc.). Similarly, directly intervening in EPCR or intervening in the upstream of EPCR inhibition while directly intervening in EPCR can also be used to promote fibrosis-free organ repair to a certain extent. Prior art often only targets a single target and have poor therapeutic effects; while directly targeting integrin or TGF-β results in safety and other problems. Combined with the feature that the circulatory system is readily accessible, the anti-fibrosis pharmaceutical composition provided by the present invention can regulate various molecules (such as EPCR, IL-1α, TIMP1, etc.) produced by multiple cell types (i.e., vascular endothelial cells, platelets, and macrophages in the circulatory system) to regulate fibrosis of damaged organs and promote regeneration of damaged cells from multiple perspectives. The technical solution of the present invention can inhibit pro-fibrotic platelet IL-1α and macrophage TIMP1, and macrophage TIMP1 has been proven to be able to stimulate the activation of fibroblasts through integrins (it can also be understood that integrins are downstream of EPCR). Therefore, the technical solution of the present invention has an obvious therapeutic effect in anti-fibrosis, which is specifically reflected in: reducing the expression of IL-1α, SDF1, and TIMP1, alleviating the pro-fibrotic "platelet-macrophage" rosette, and inhibiting the Rho-activation in the endothelial cells, and reducing deposition of inflammatory monocytes to reduce fibrosis degree of damaged organs and promote regeneration (including parenchymal cell proliferation, tissue repair, function restoration etc.) of damaged organs, and promote fibrosis-free repair of damaged organs. It should be noted that the technical solution of the present invention can further inhibit the activation of fibroblasts by reducing the expression of TIMP1 in macrophages. The above therapeutic effects have been verified in the regeneration and fibrosis models of lung, liver, kidney, and their corresponding aging models.

. Fibrosis has long been considered irreversible. Prior art is often only able to inhibit fibrosis to a certain extent, but is difficult to "reverse fibrosis". By targeting EPCR (or in combination with EPCR pathway activator + NRP1 inhibitor and/or HIF2α inhibitor, thereby targeting EPCR), the present invention regulates the expression of EPCR to affect the expression of platelet ILla and macrophage TIMP1 in the circulatory system (i.e., normalization of the aberrant platelet-macrophage-vascular endothelial cell interactions) to inhibit fibrosis and restores the regenerative capacity of damaged organs to a certain extent, achieving "reversal of fibrosis" (i.e., fibrosis-free repair of damaged organs, thereby transforming the damaged organ from fibrosis to regeneration). In addition, the use provided by the present invention can alleviate immunotherapy-induced pneumonia to a certain extent.

. In summary, use of an EPCR pathway activator in the preparation of an anti-fibrotic agent provided by the technical solution of the present invention can facilitate systematically alleviation or treatment, or even reversal of organ fibrosis to a certain extent.

### Summary of main acronyms used in the present invention

. EPCR: Endothelial protein C receptor; HIF2α: Hypoxia-inducible-factor 2α; NRP1: Neuropilin 1; TIMP1: Tissue inhibitor of metalloproteinases 1; IL-1α: Interleukin-1α; ECs: Endothelial cells; PCECs: Pulmonary capillary endothelial cells; P1GF: Placental growth factor; SDF1: stromal-derived factor 1; HDAC: Histone deacetylase; VPA: Valproic acid; DNMT: DNA methyltransferase; AZA: 5-azacitidine; PNX: Pheumonectomy; PH: Partial hepatectomy; I/R: Ischemia-reperfusion.

. As used herein, "promote" refers to further improvement of the described object at the existing level, the existing level comprises one or more of quantitative level, expression level, function level, and capacity level.

. As used herein, "reverse fibrosis" refers to transforming an organ in a fibrotic state to regeneration, including reducing fibrosis level, reducing severity of symptoms caused by fibrosis, and enhancing regeneration capacity.

. As used herein, "promote activity of EPCR pathway" refers to enhancing EPCR signal and/or increasing expression level of EPCR that allow EPCR to interact with its downstream molecules.

### Description of Drawings

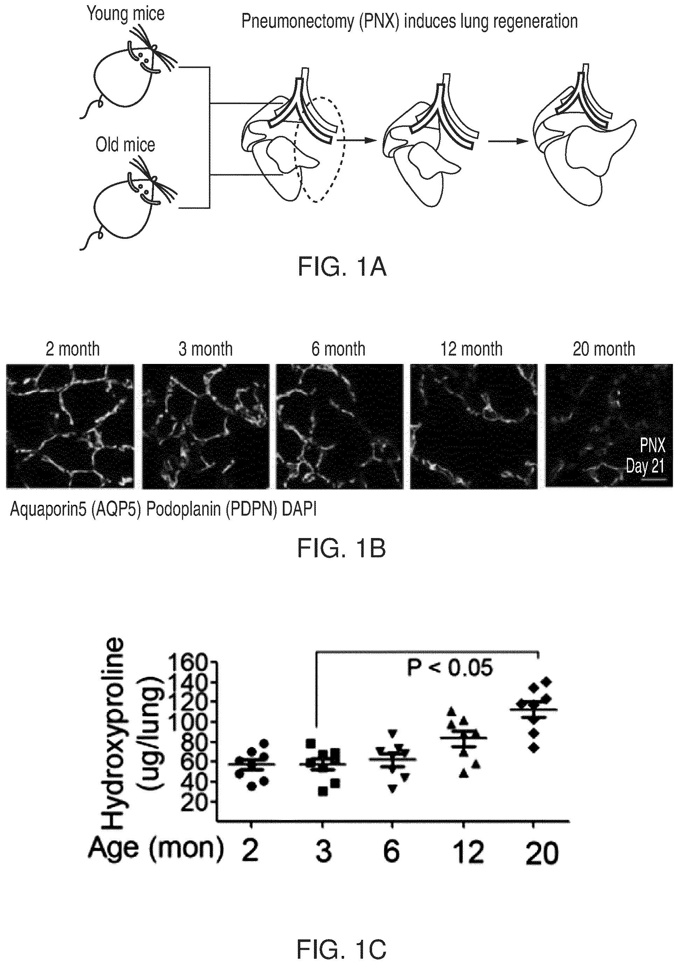

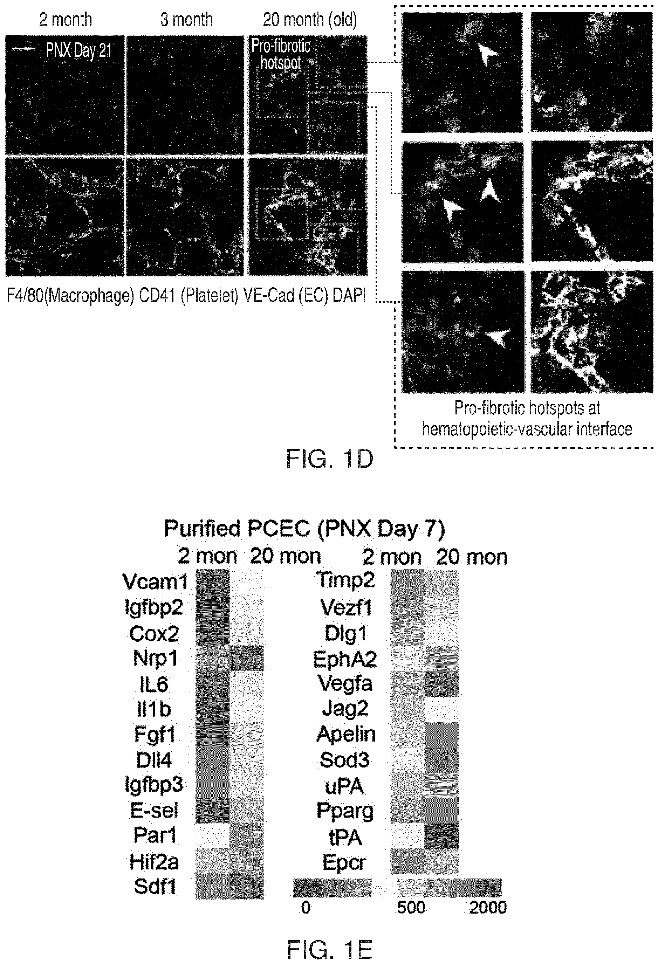

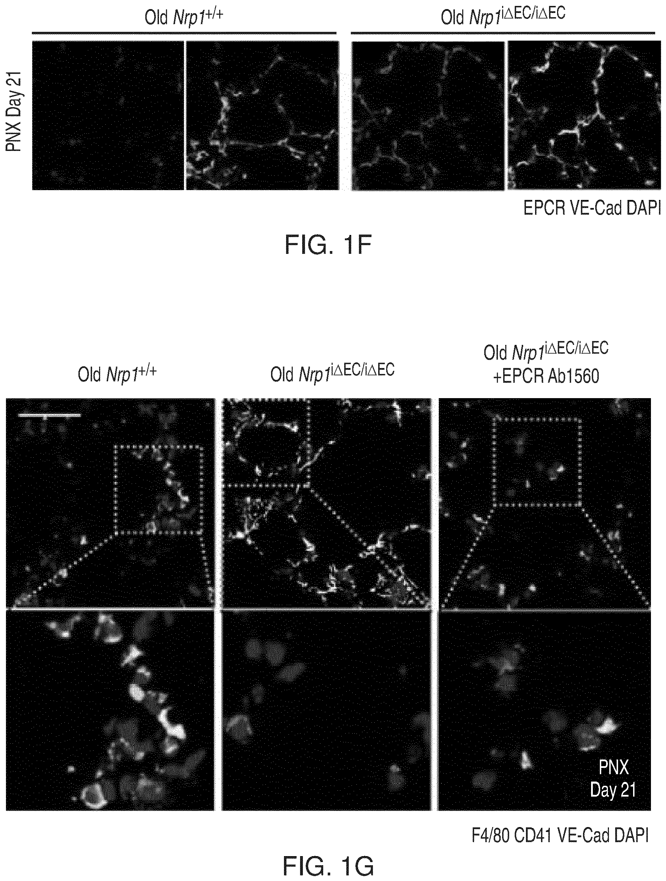

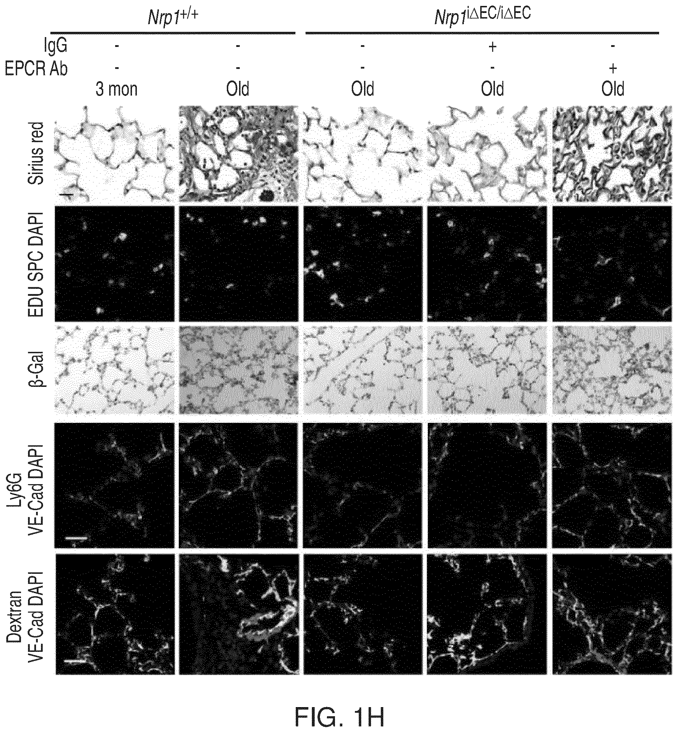

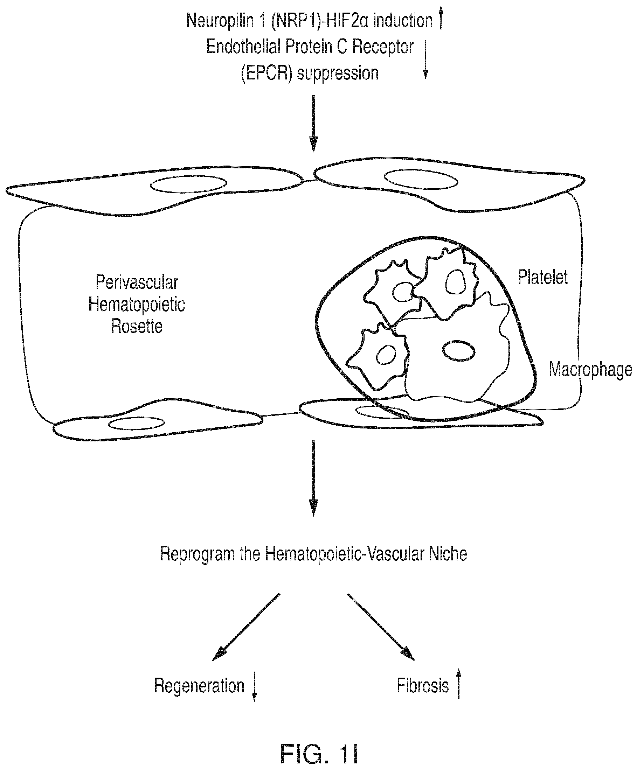

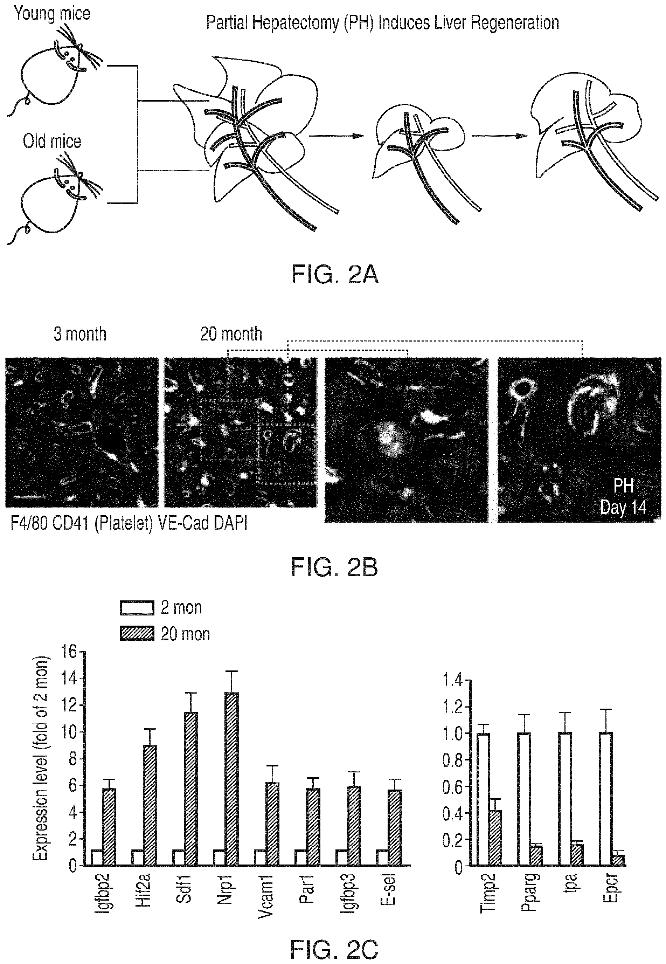

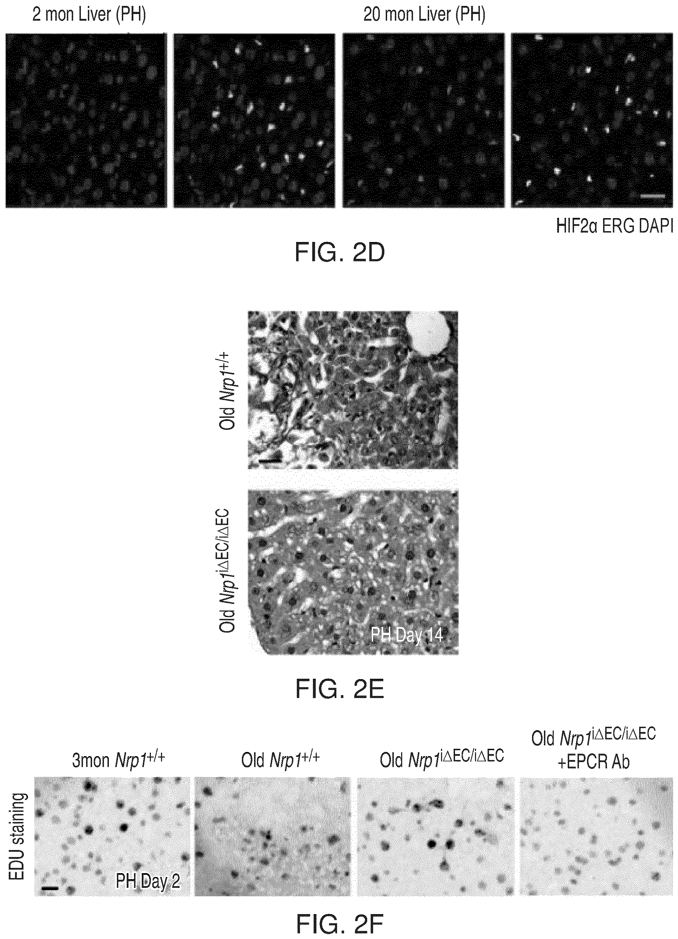

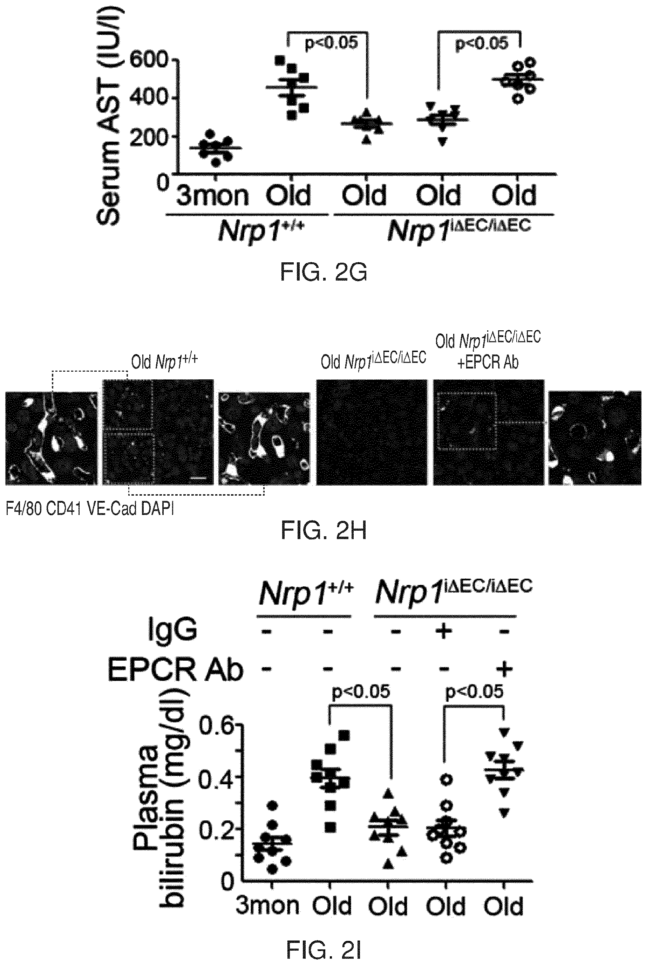

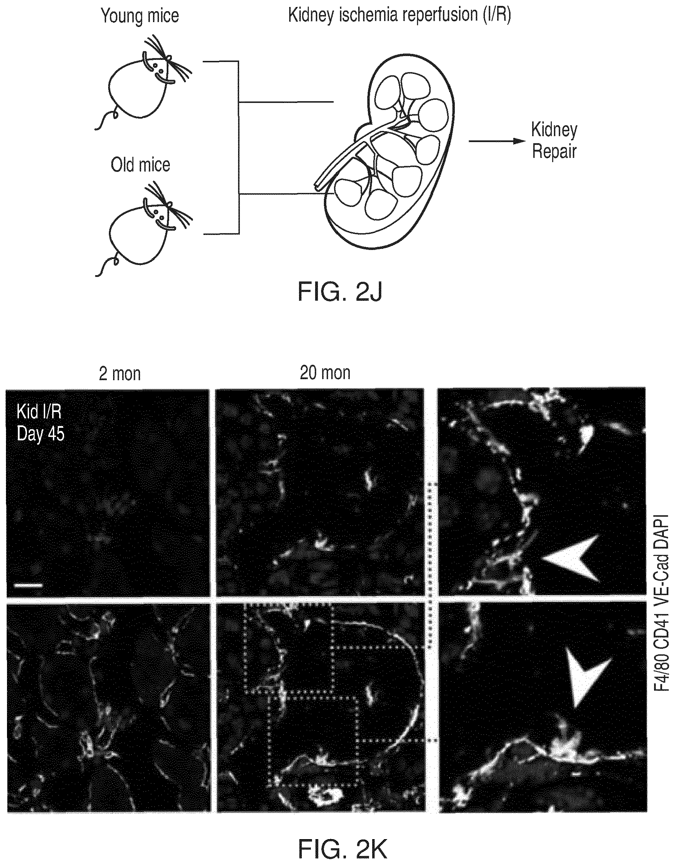

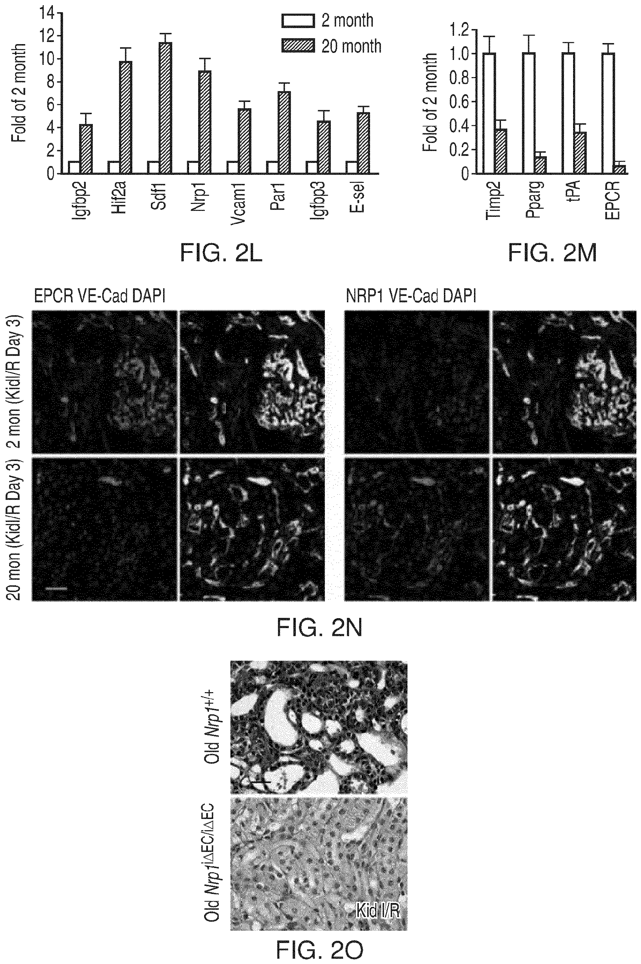

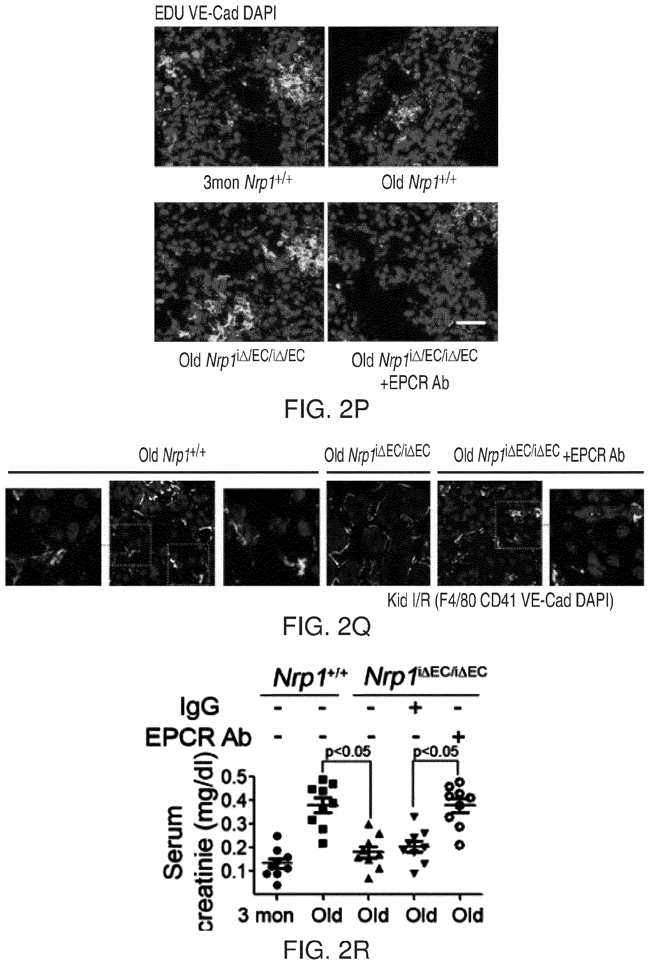

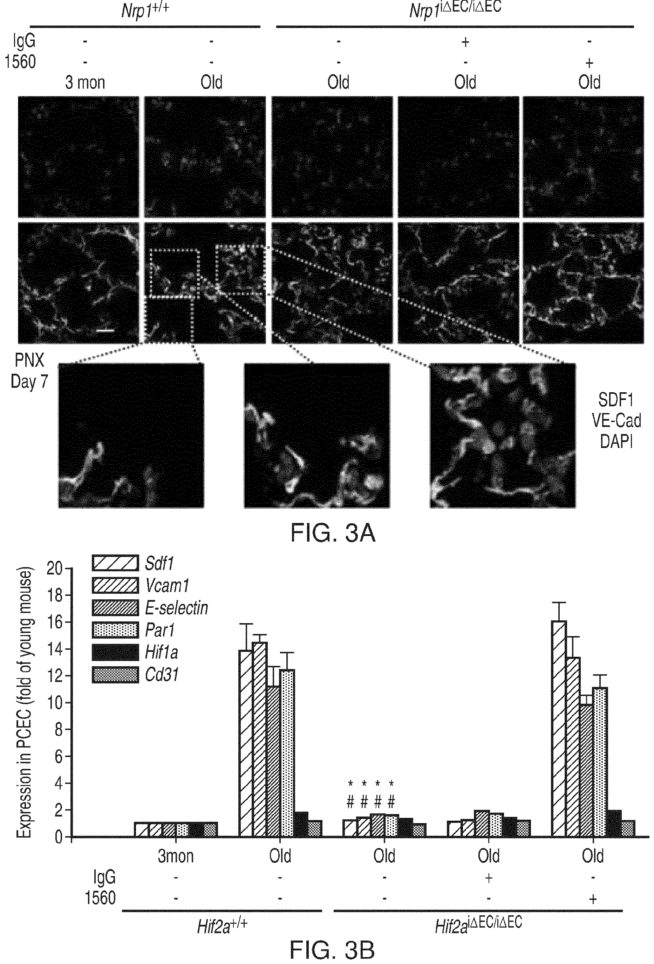

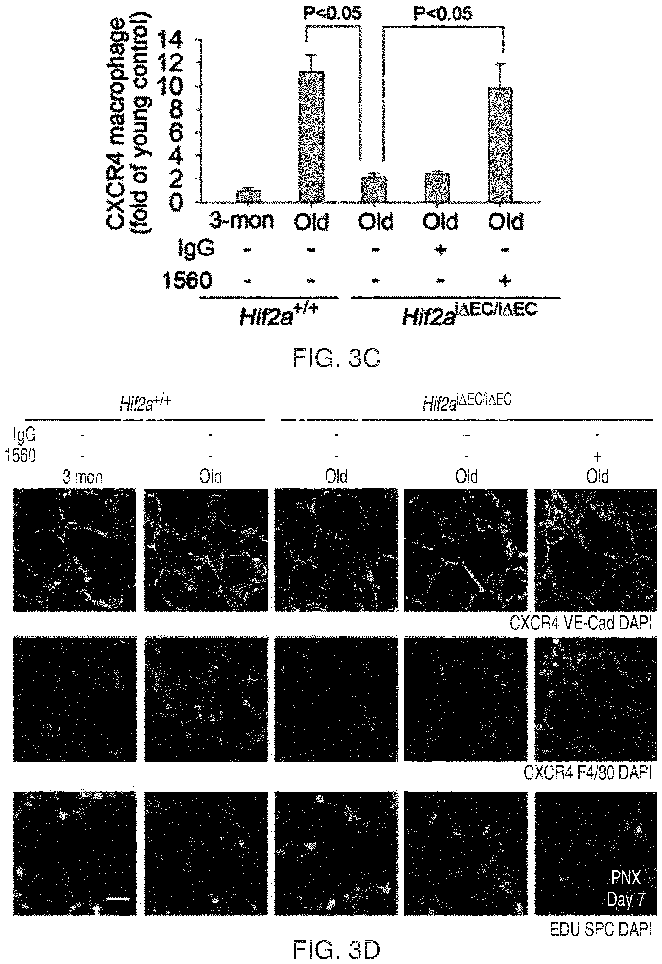

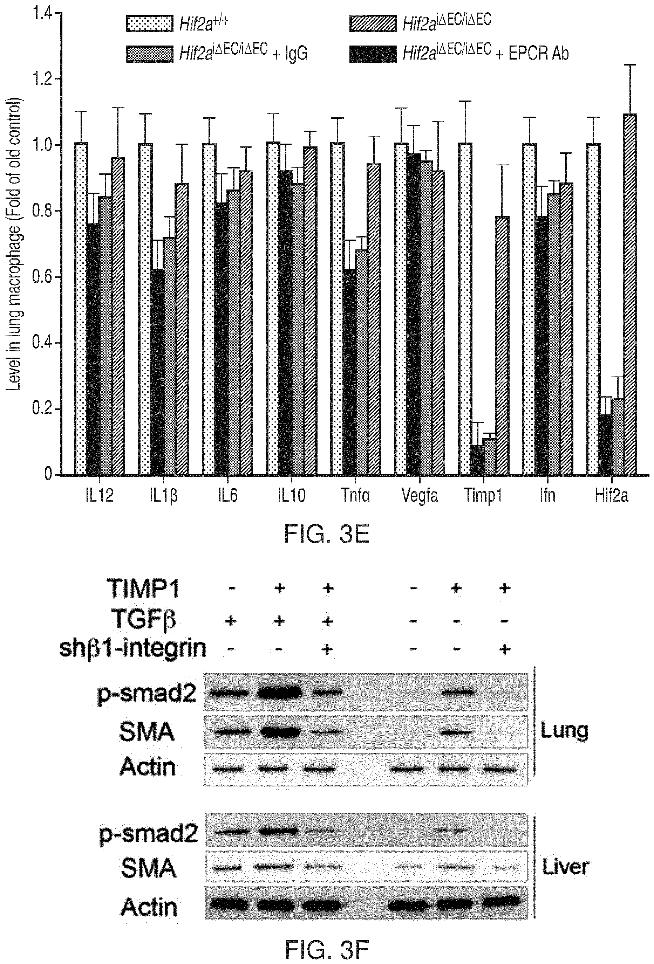

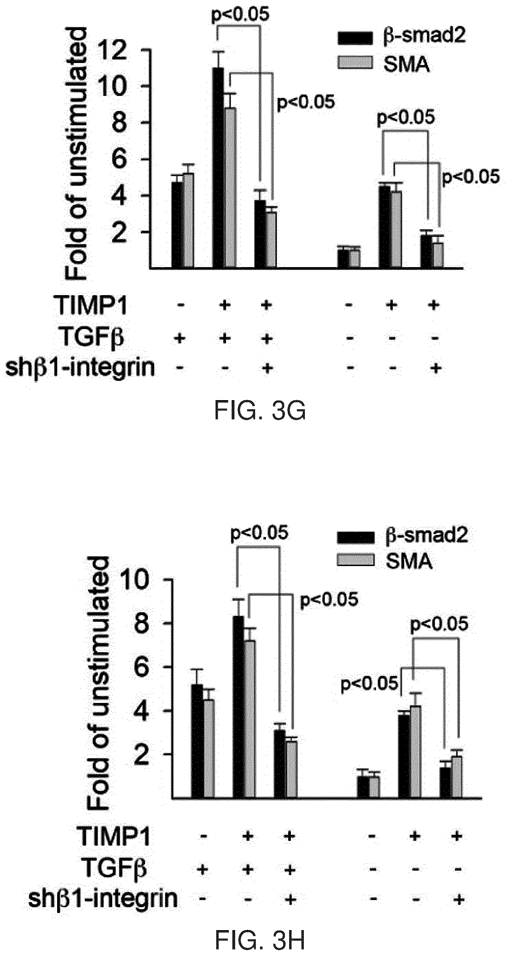

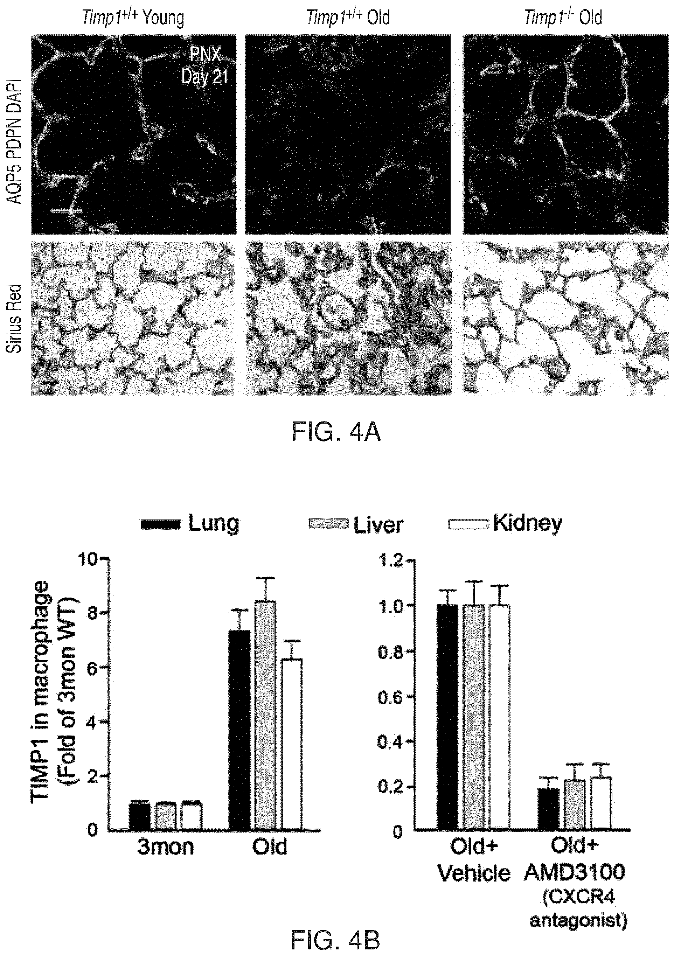

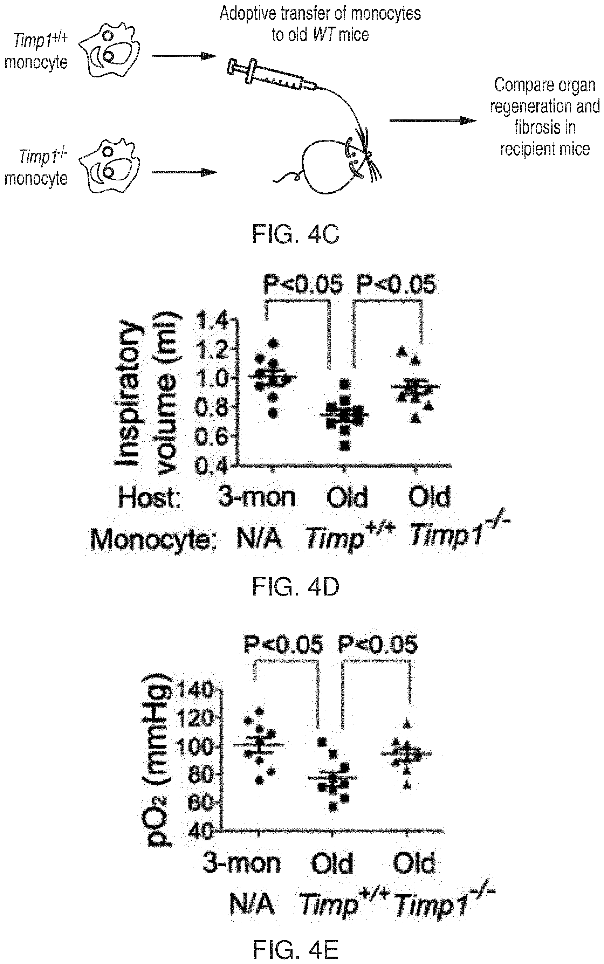

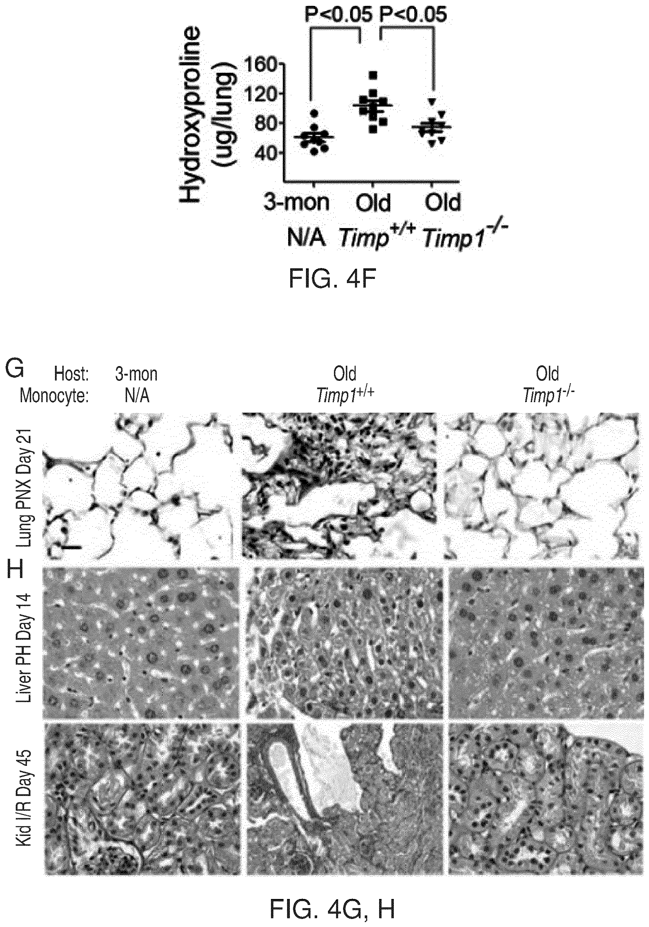

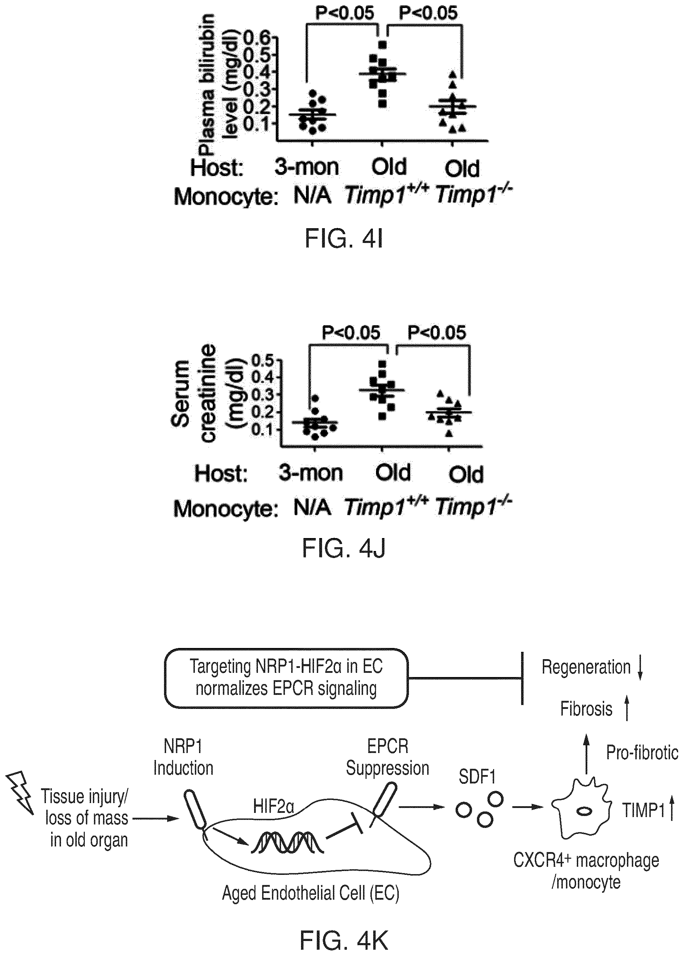

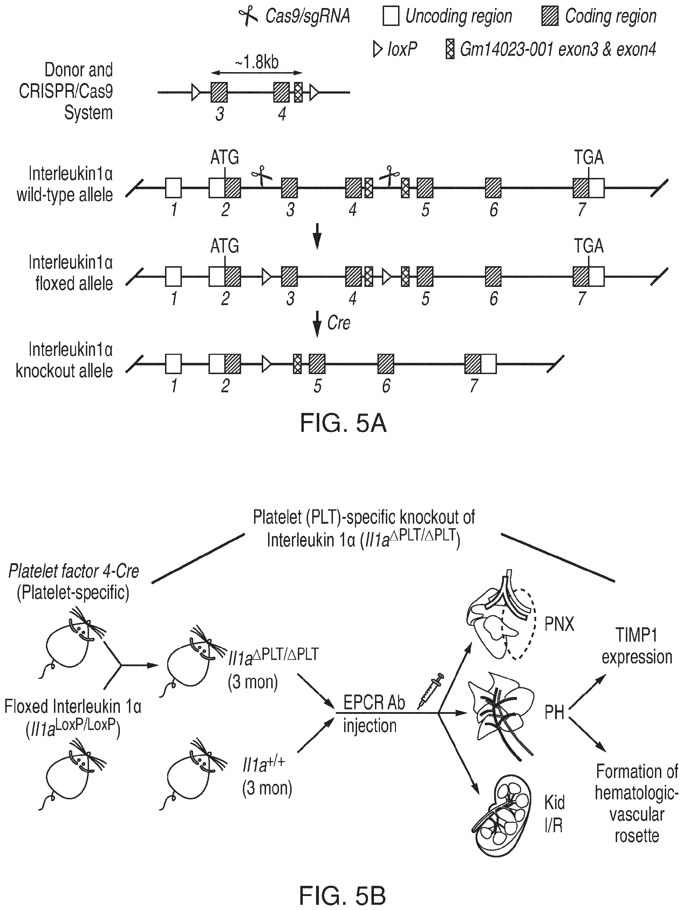

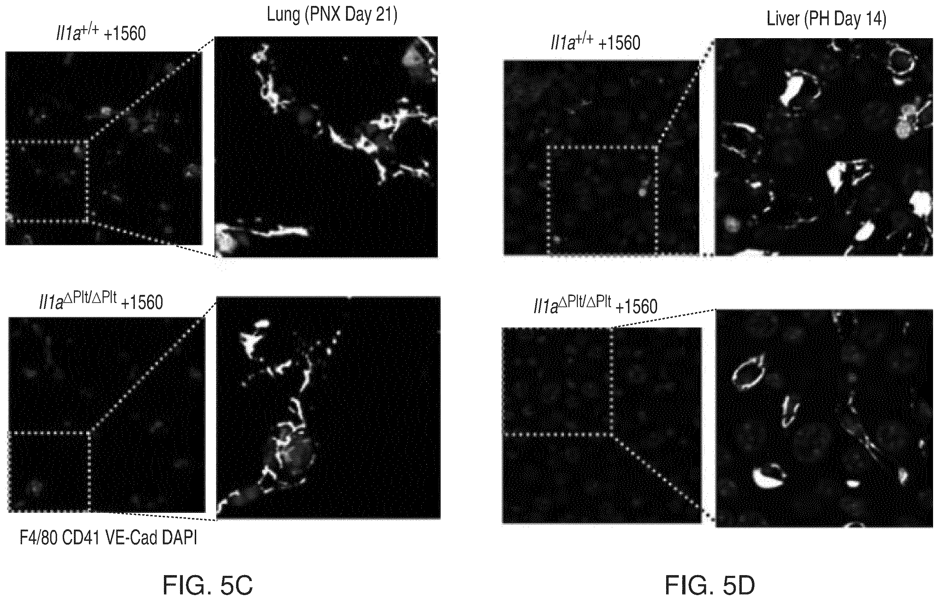

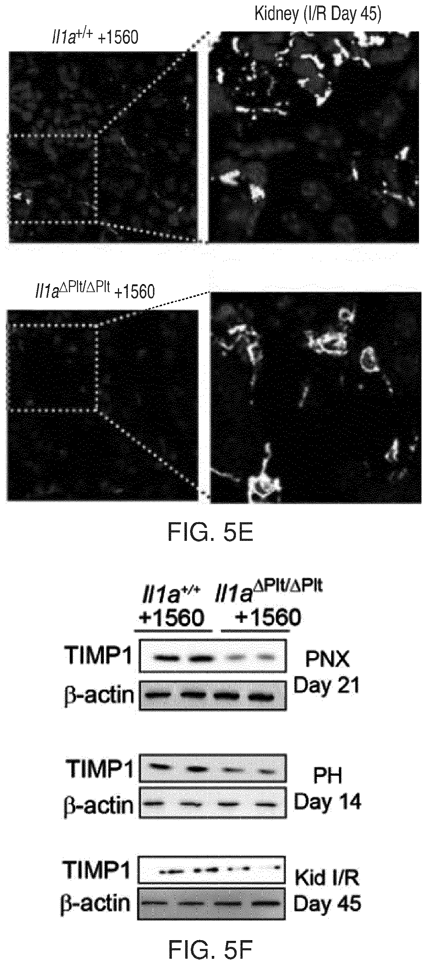

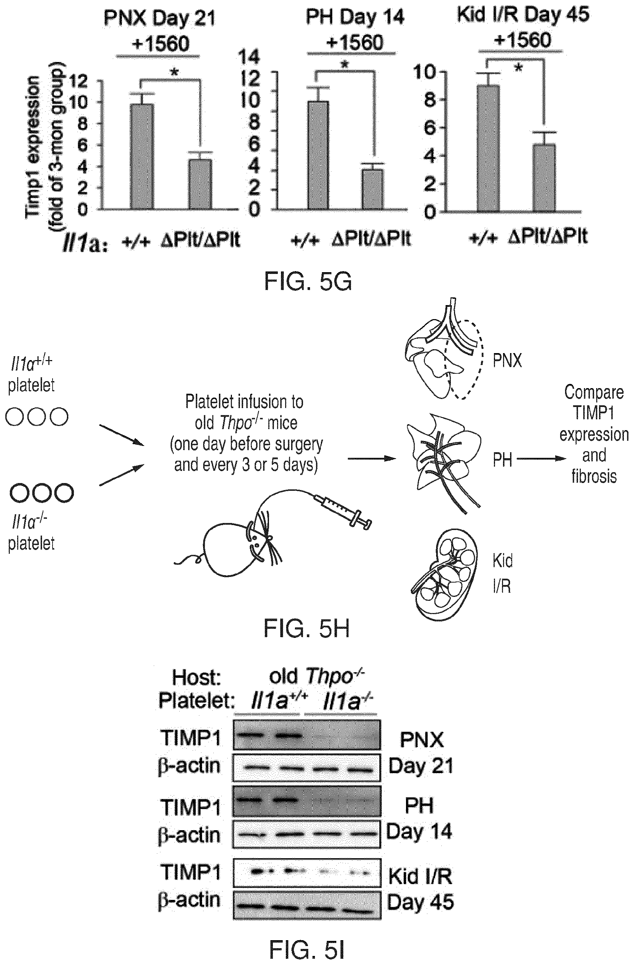

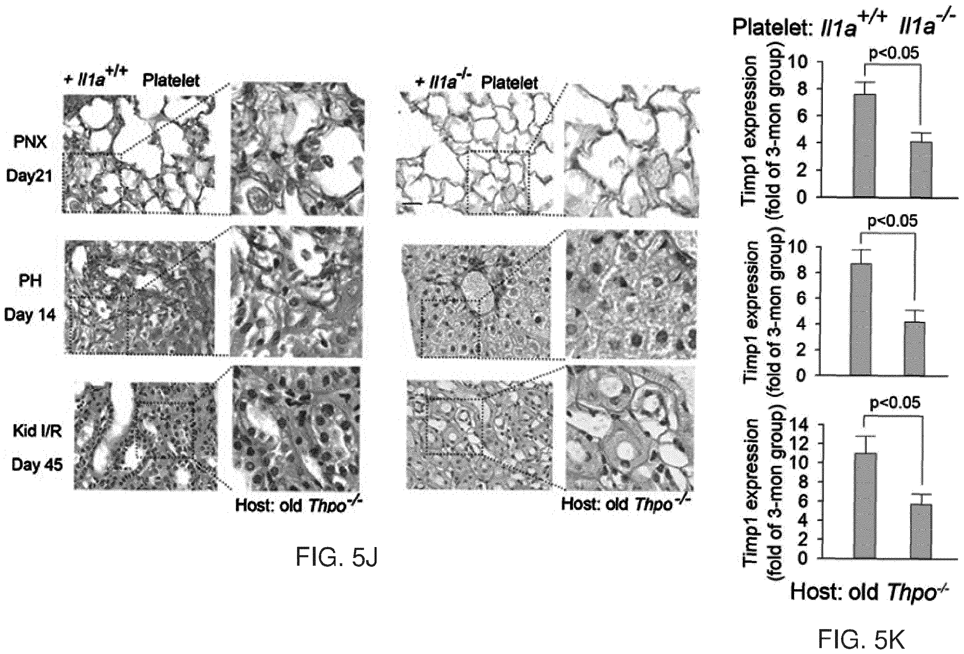

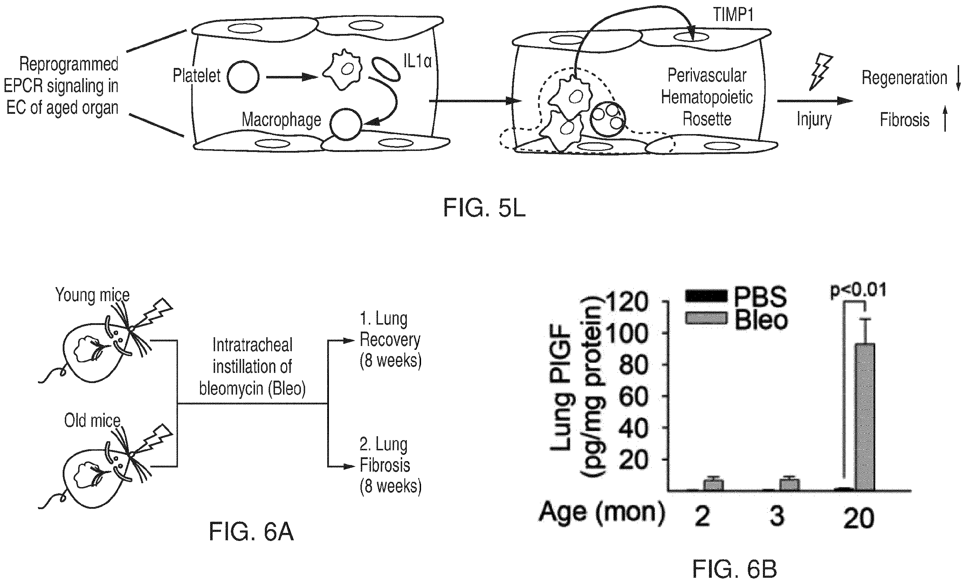

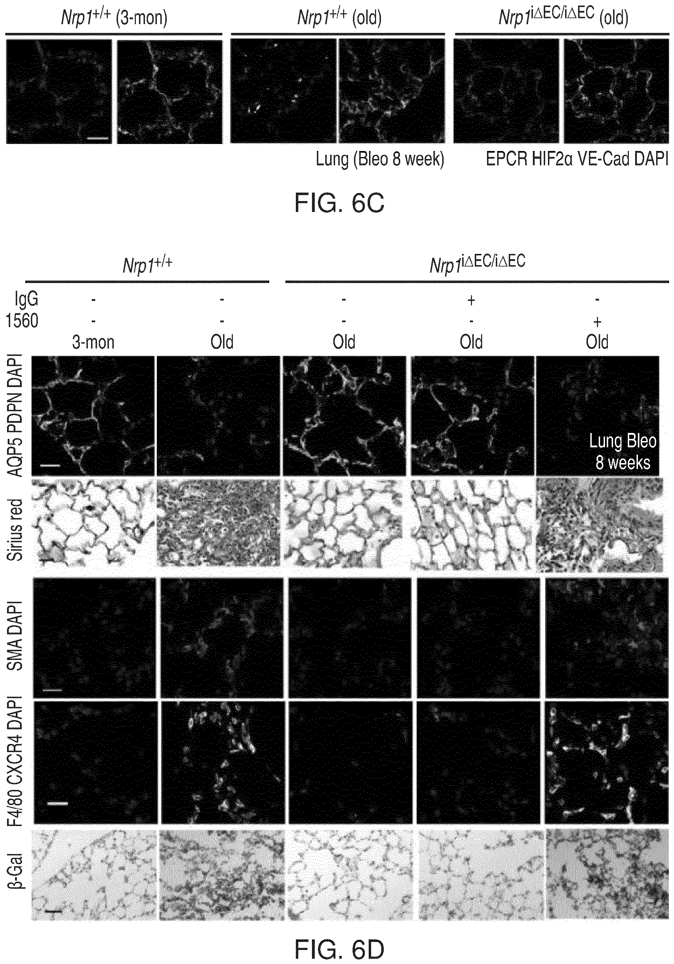

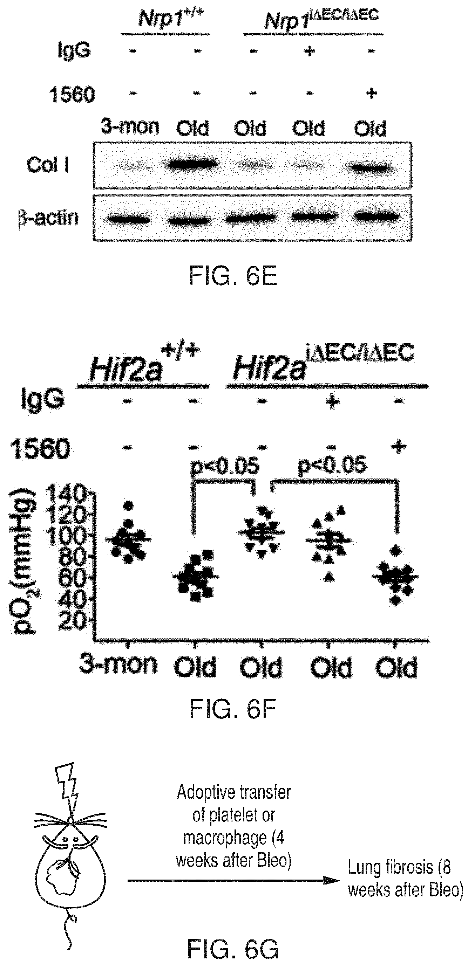

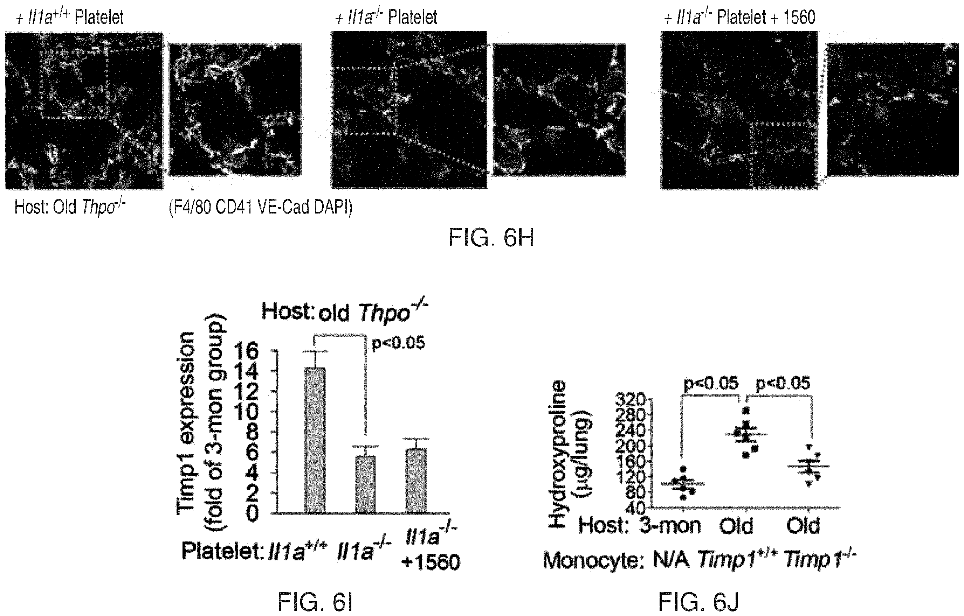

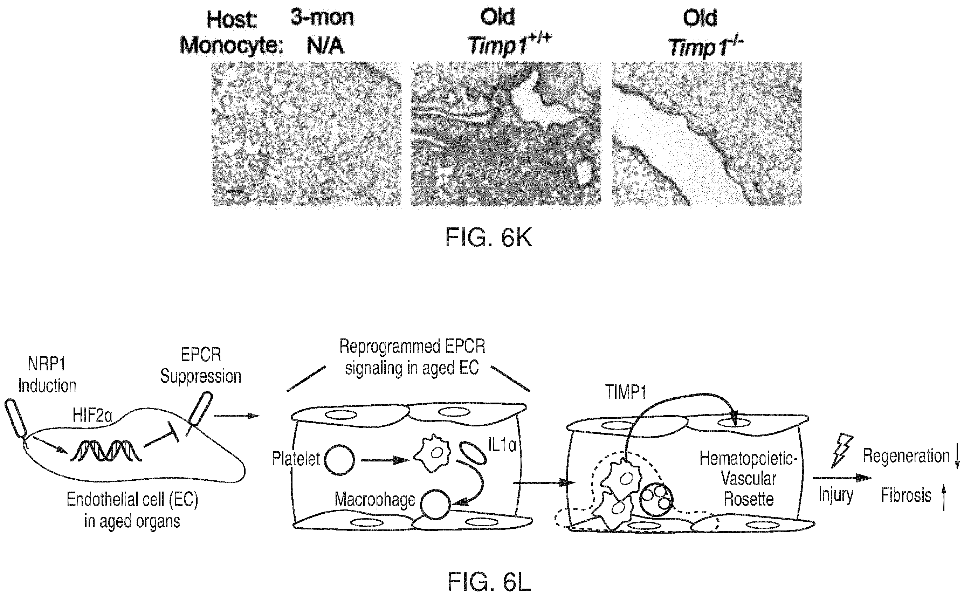

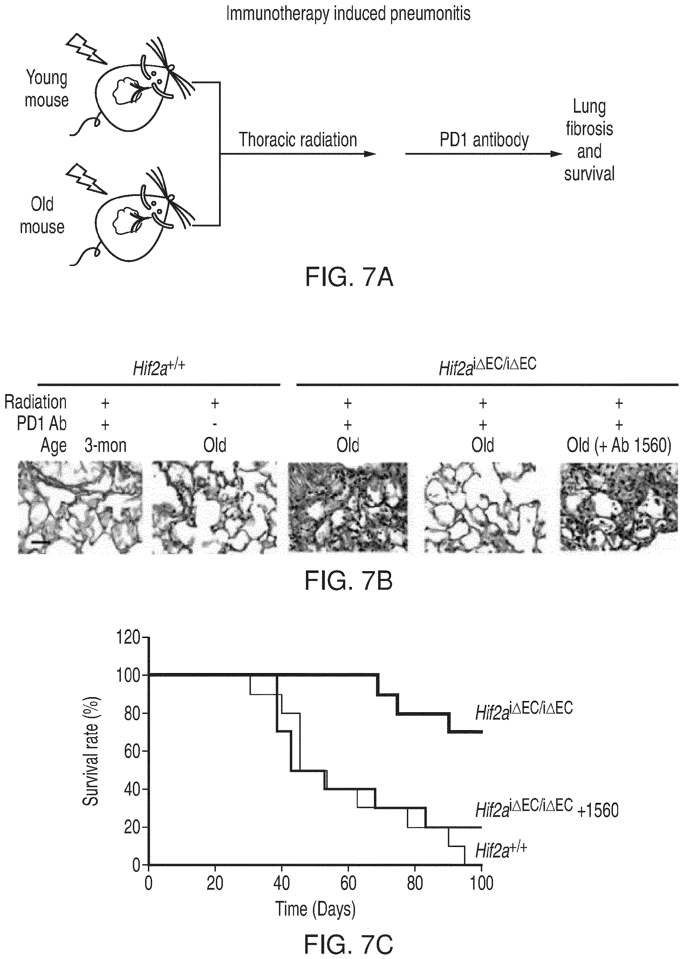

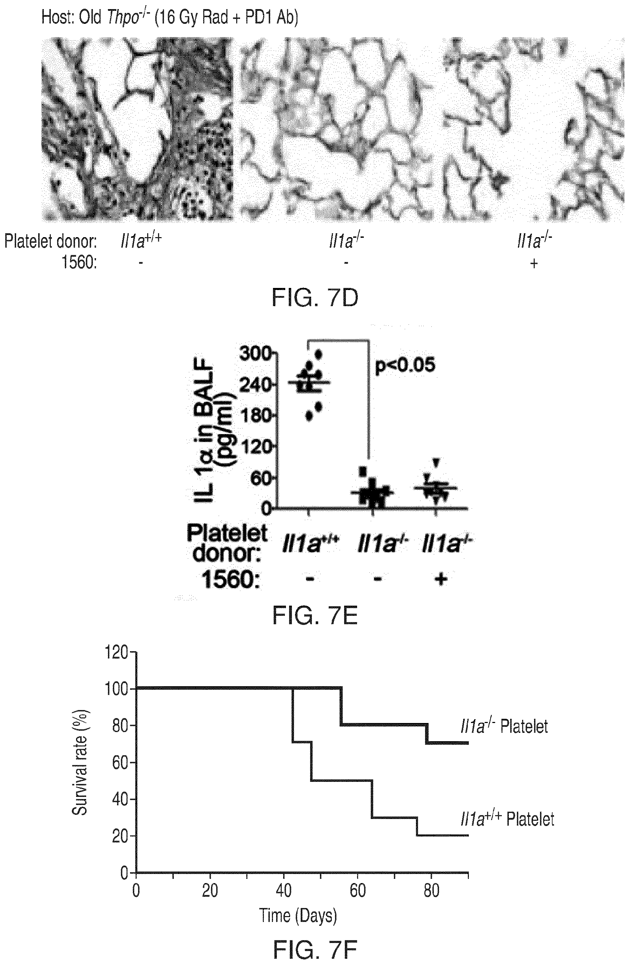

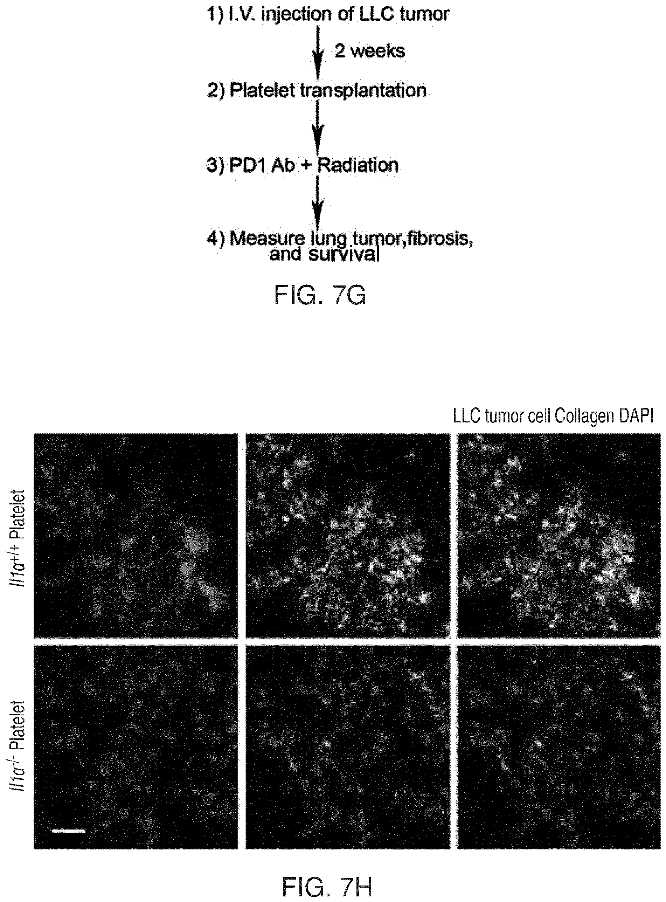

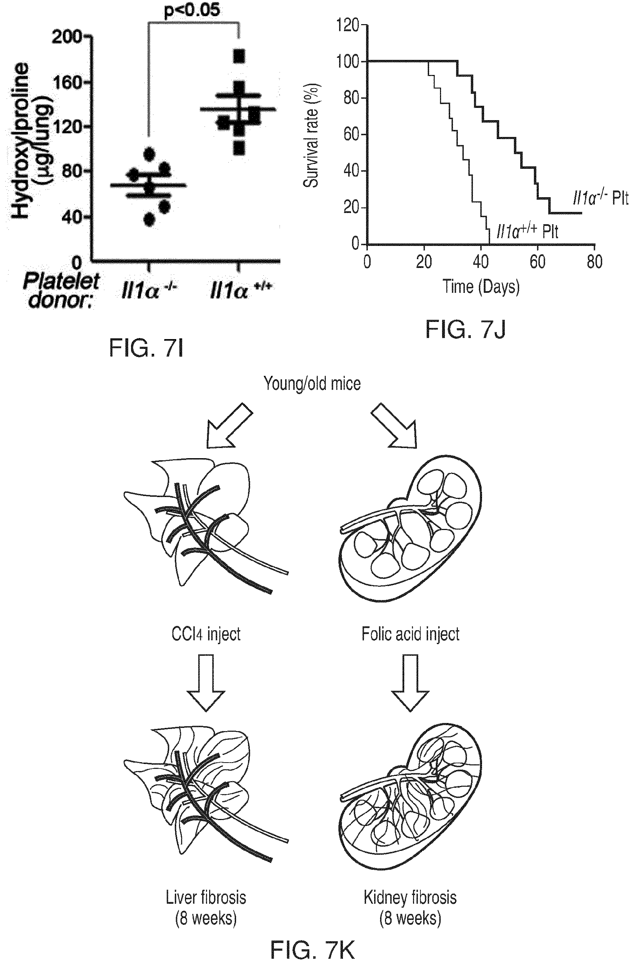

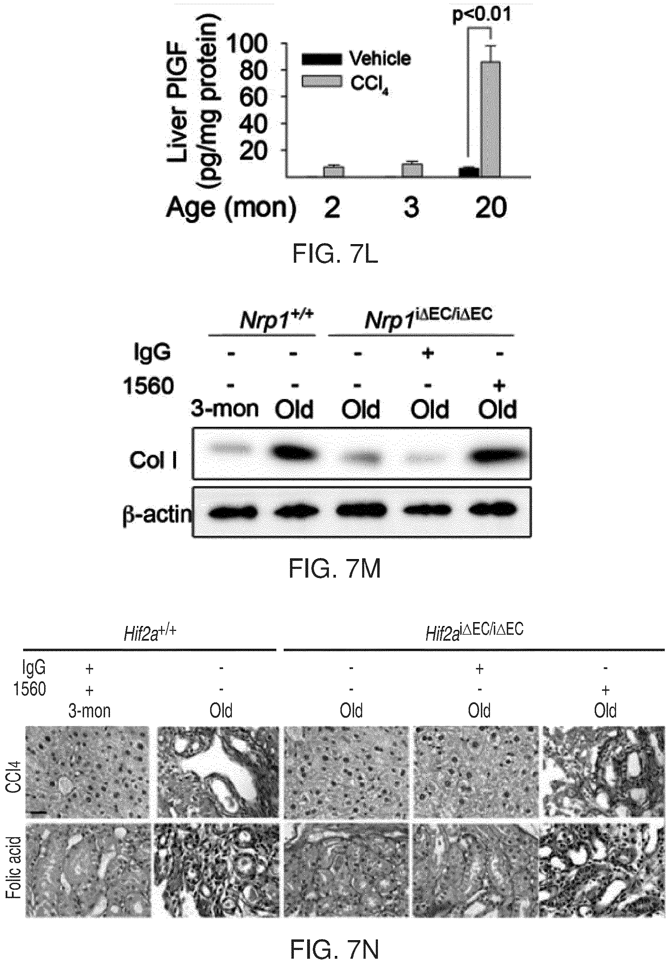

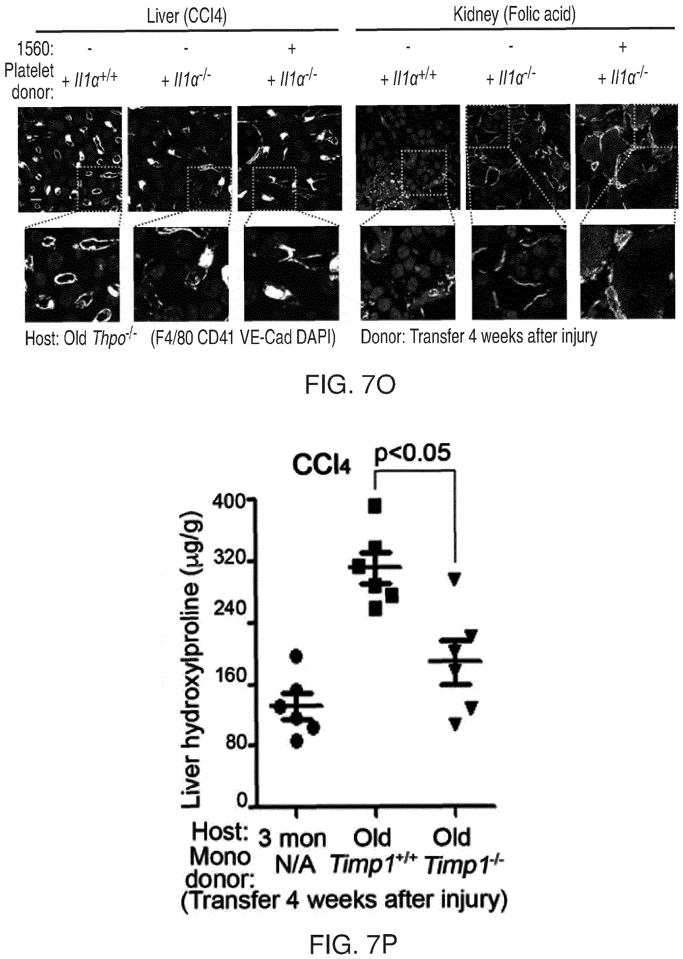

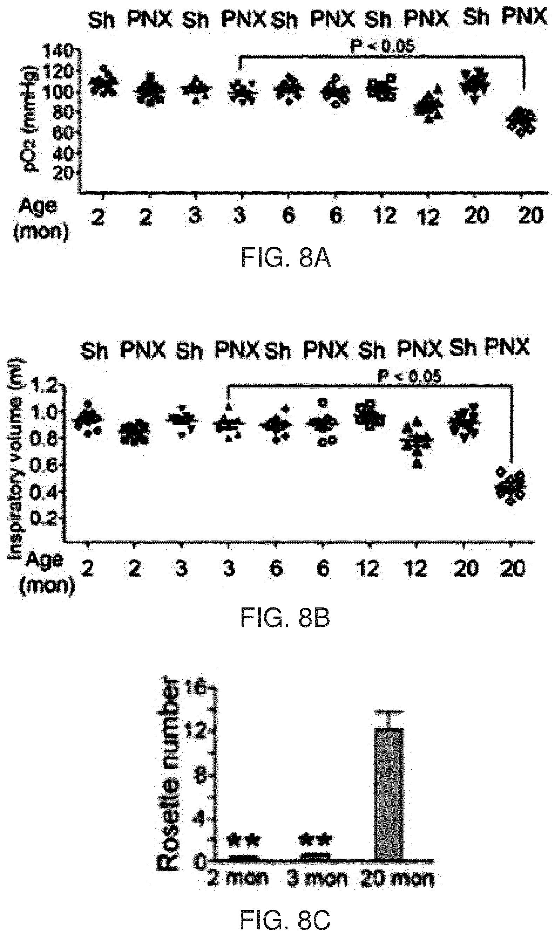

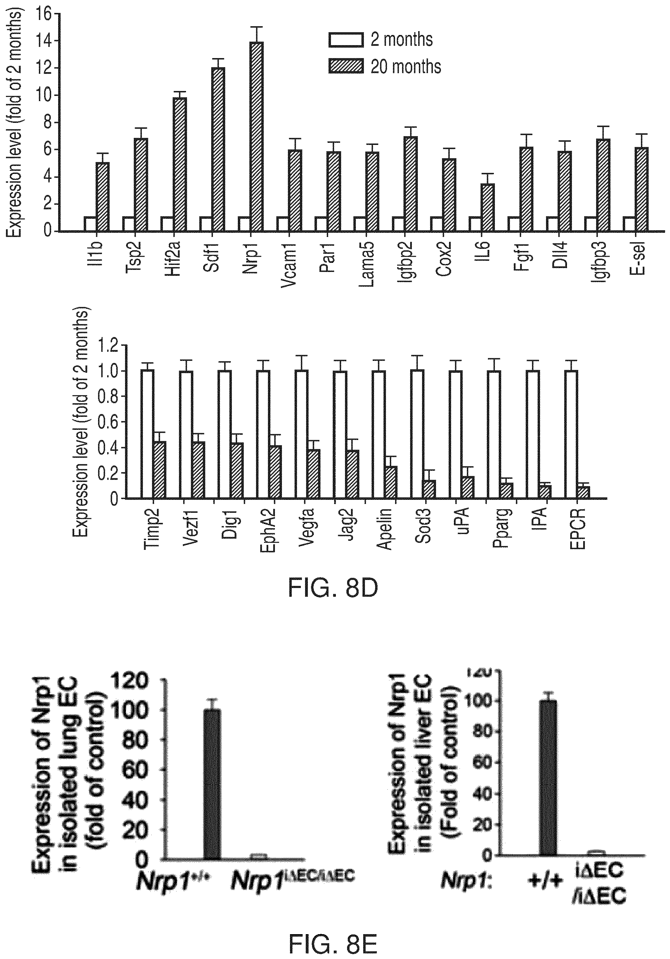

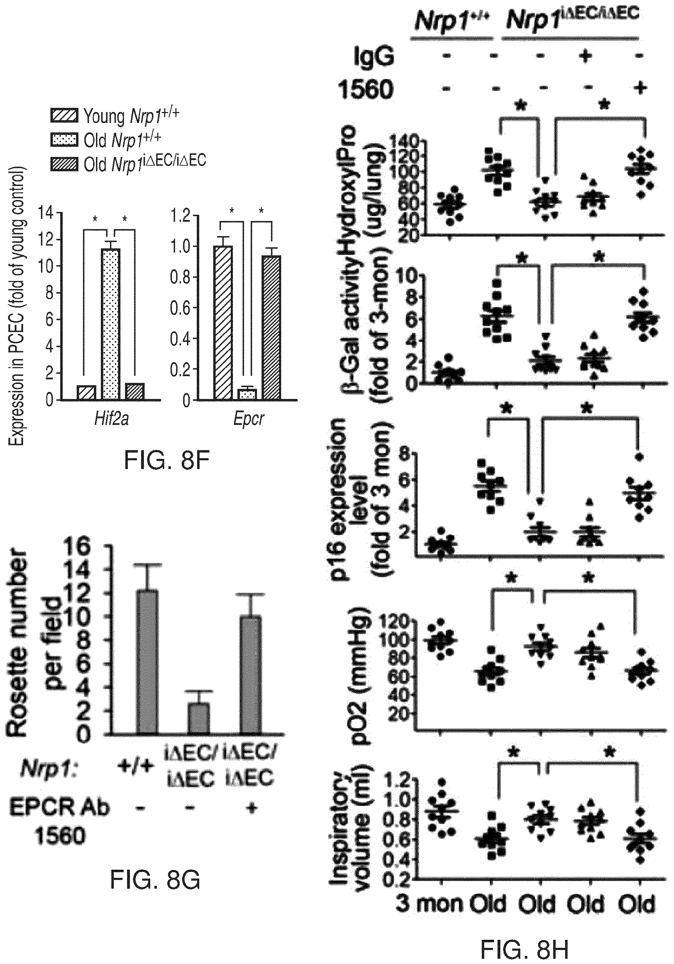

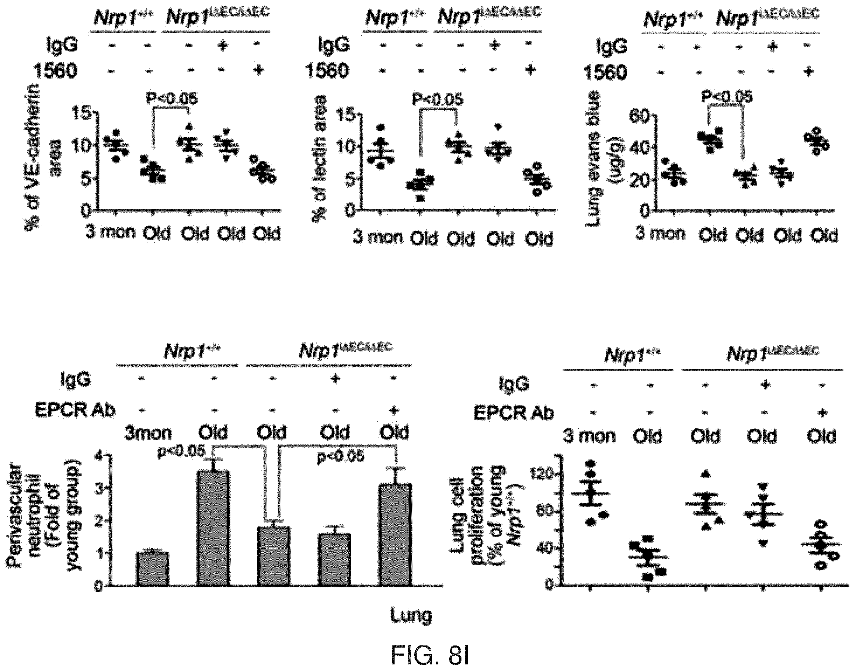

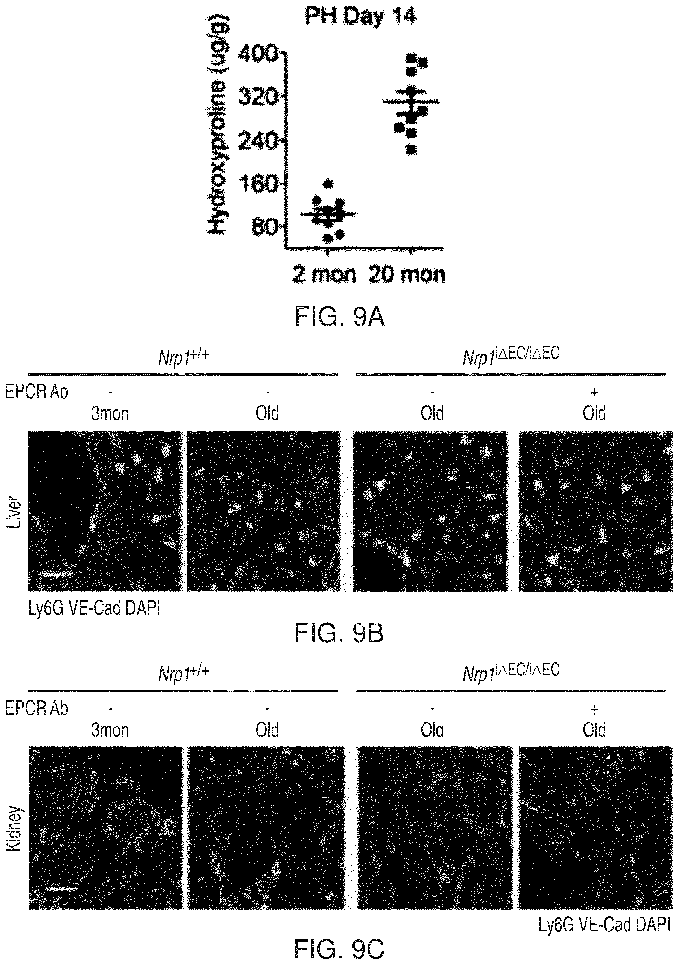

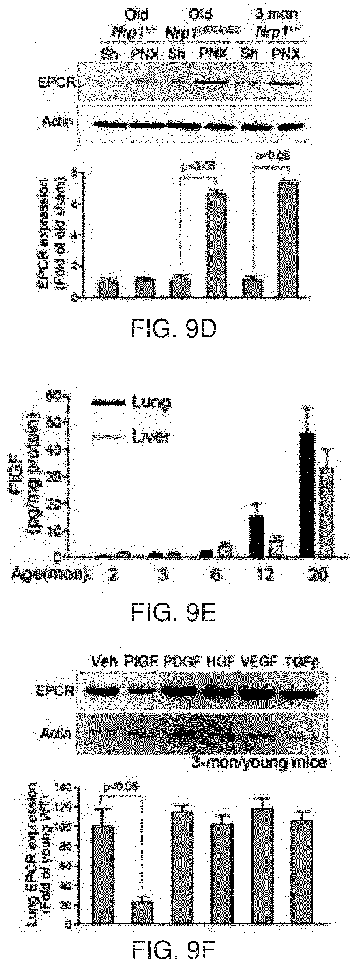

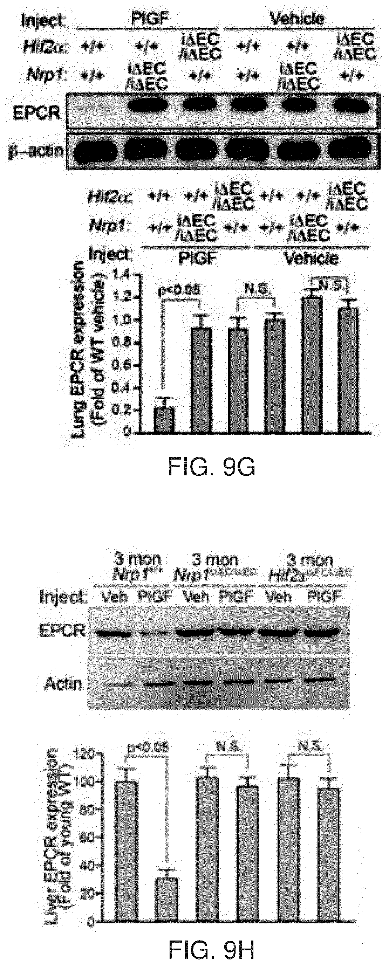

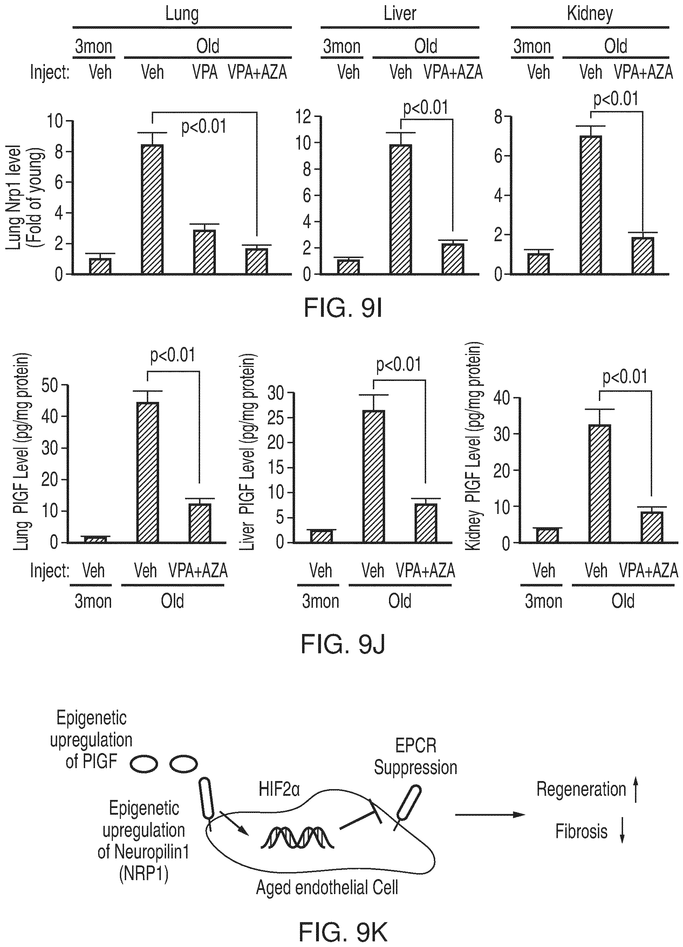

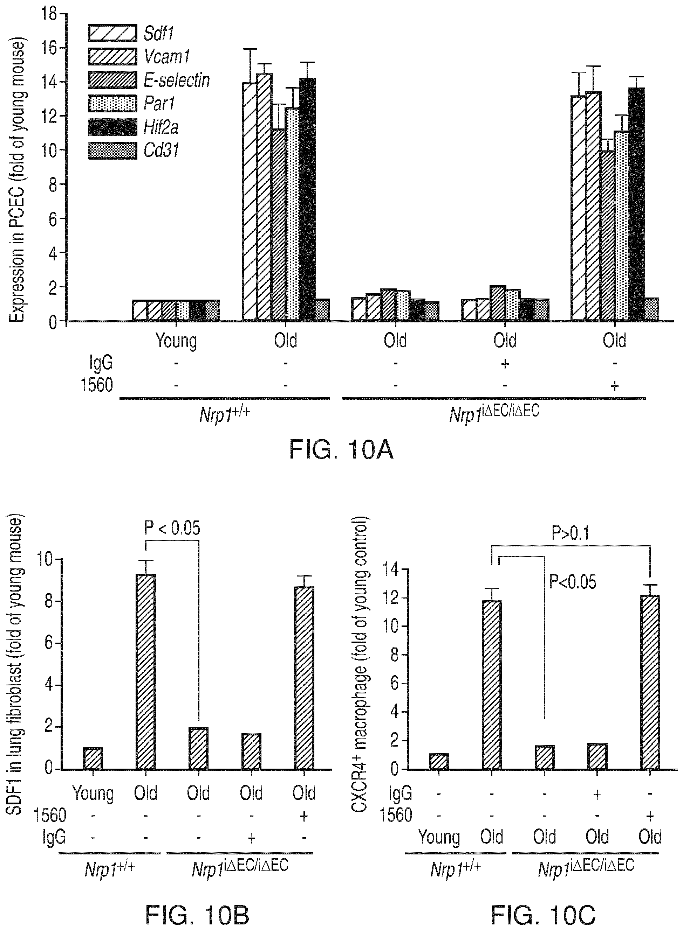

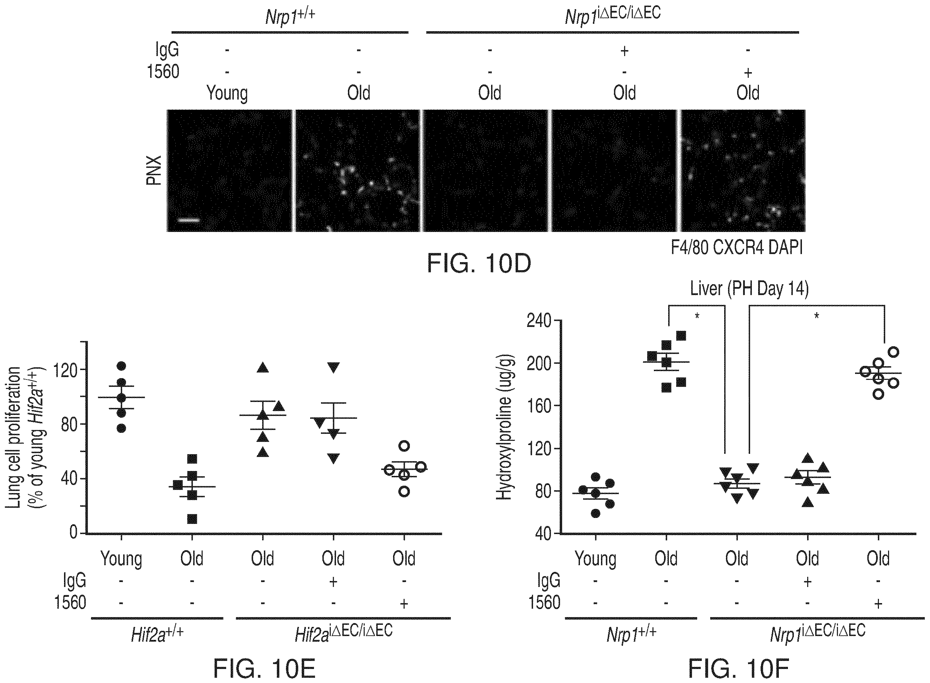

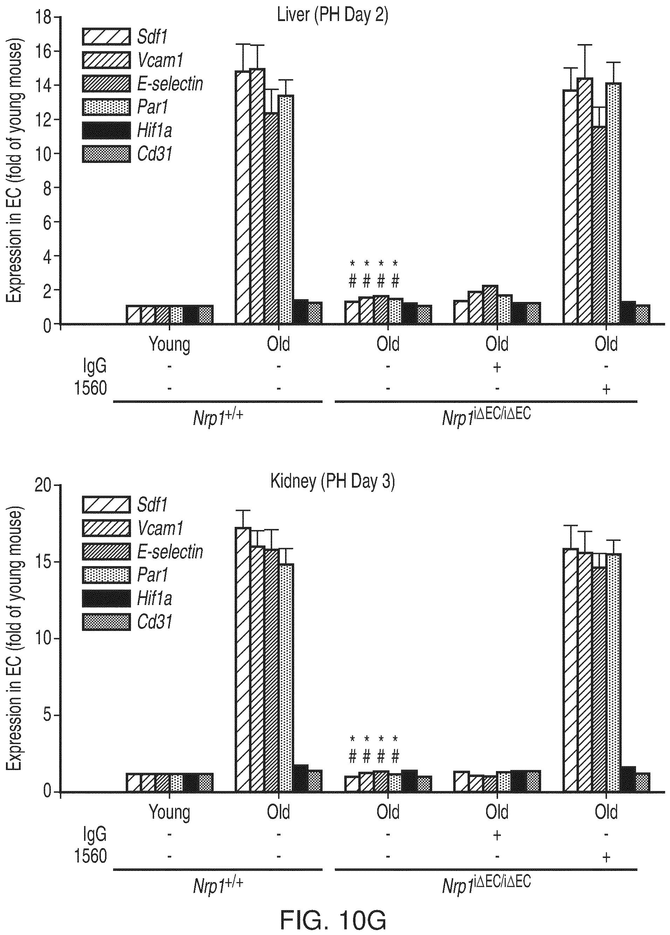

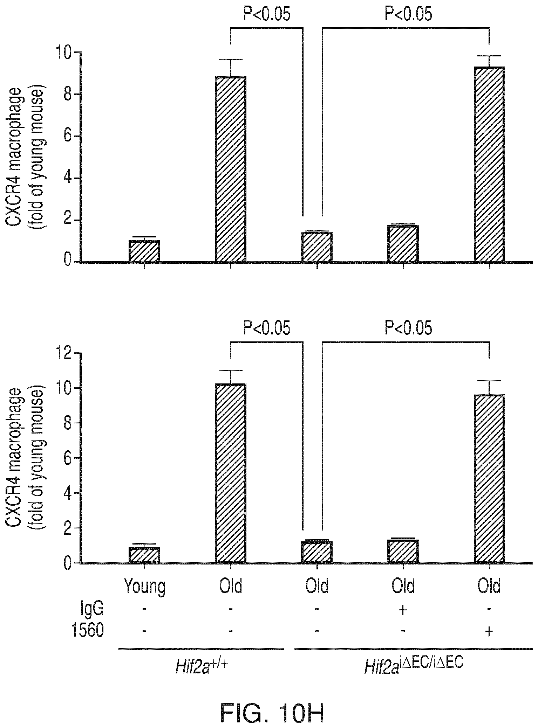

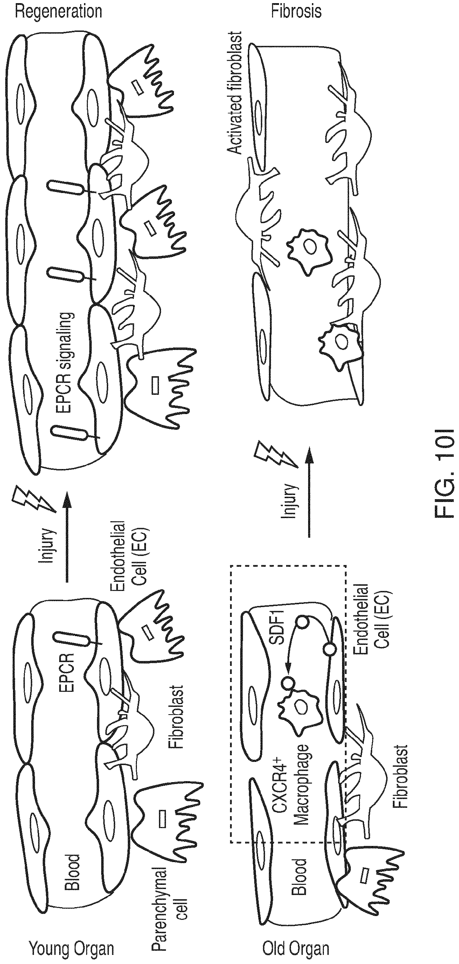

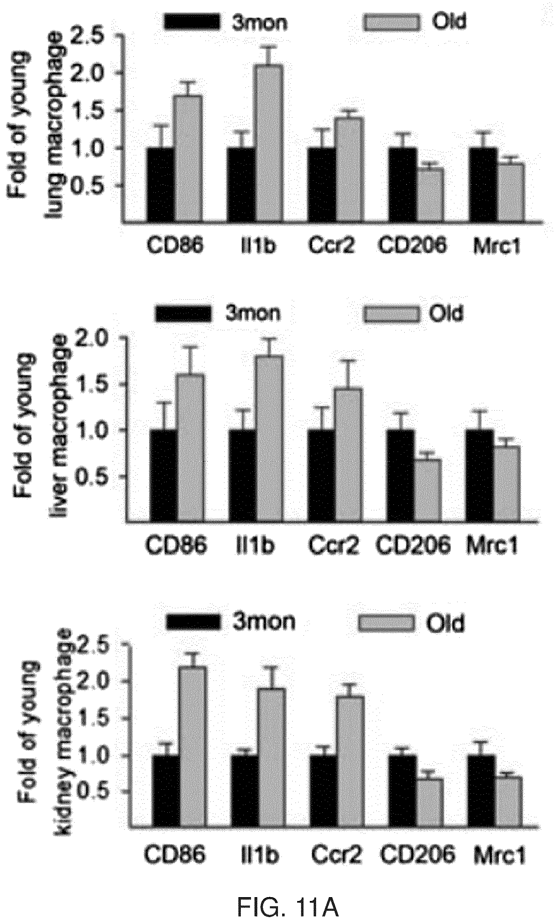

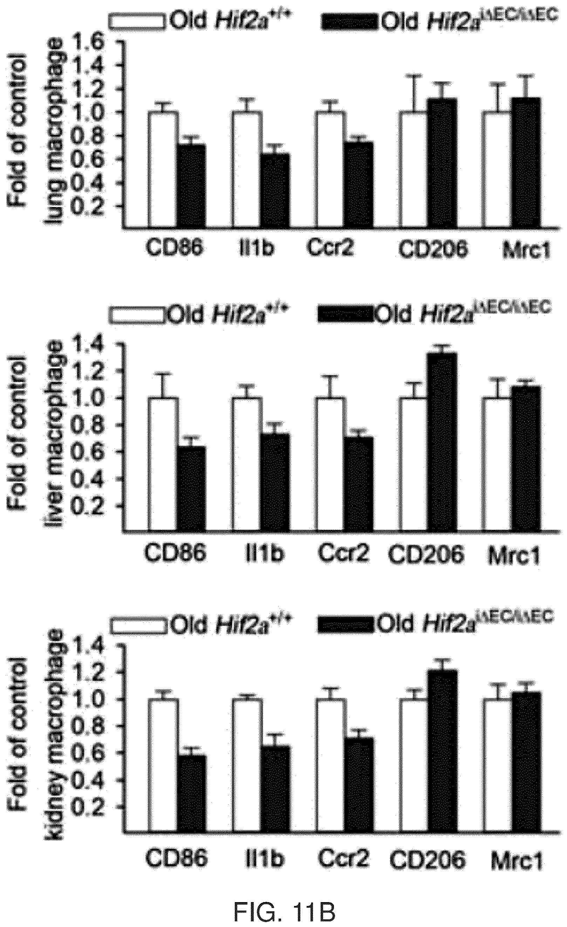

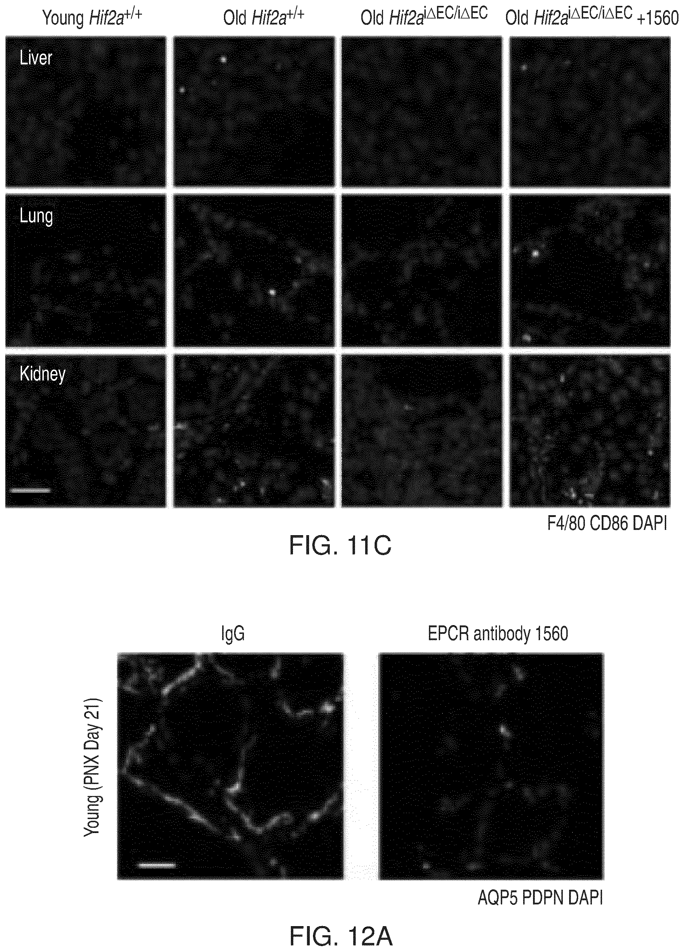

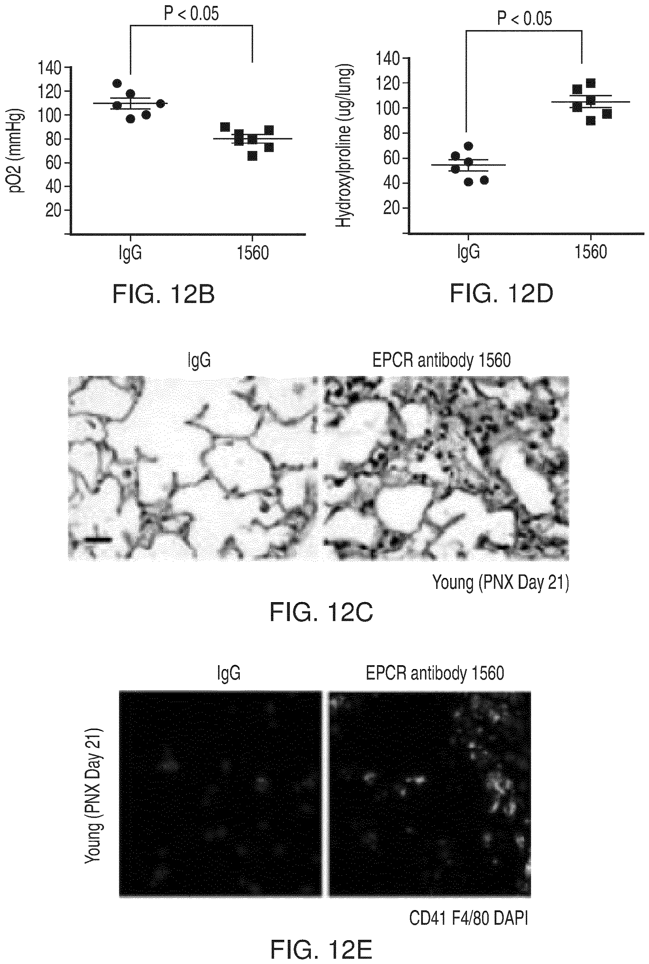

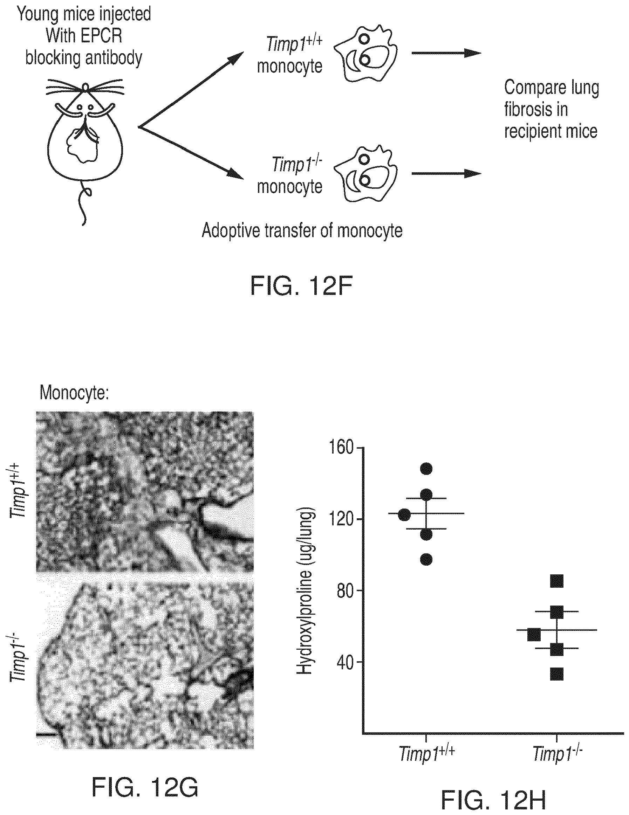

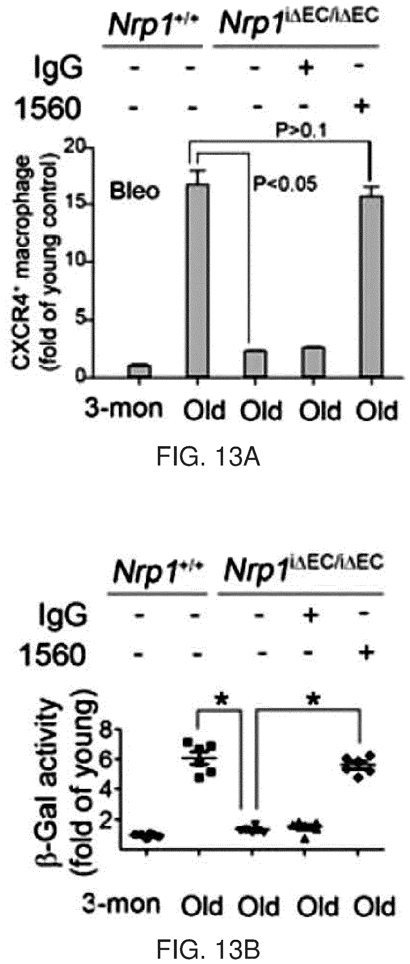

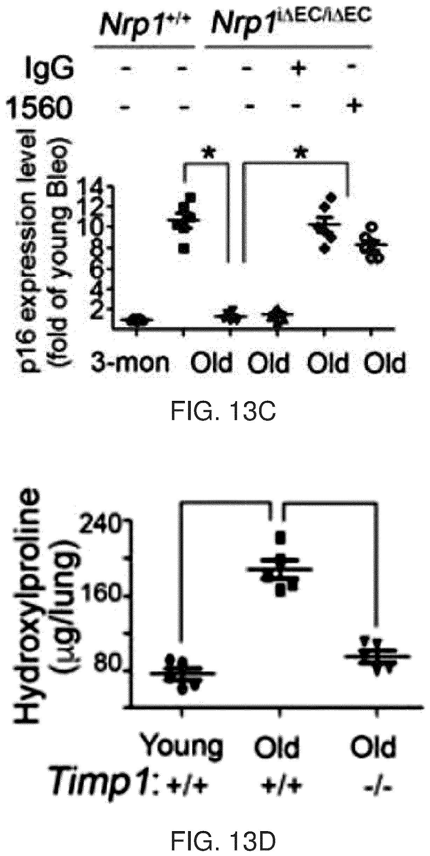

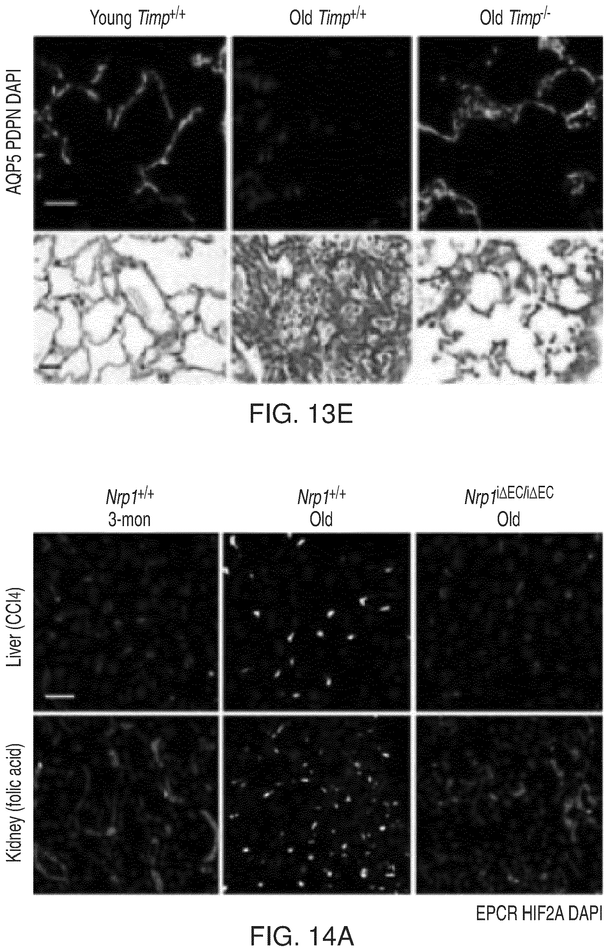

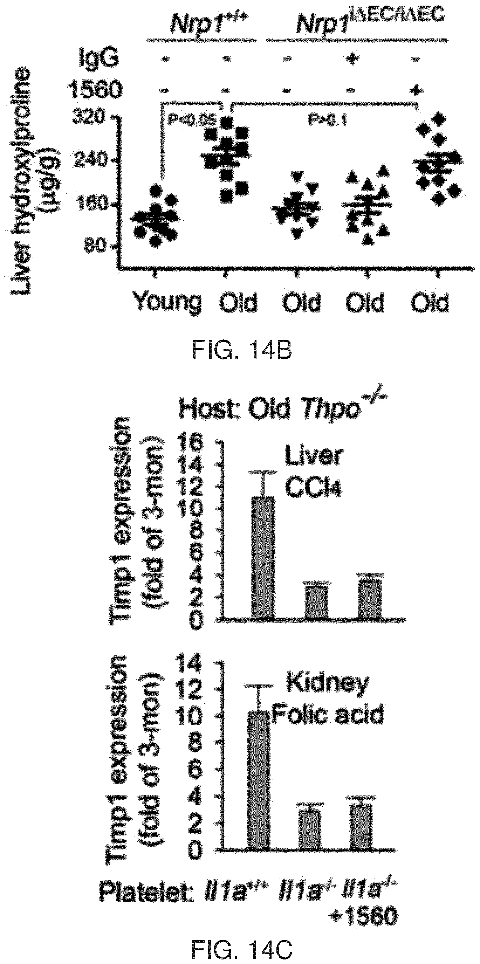

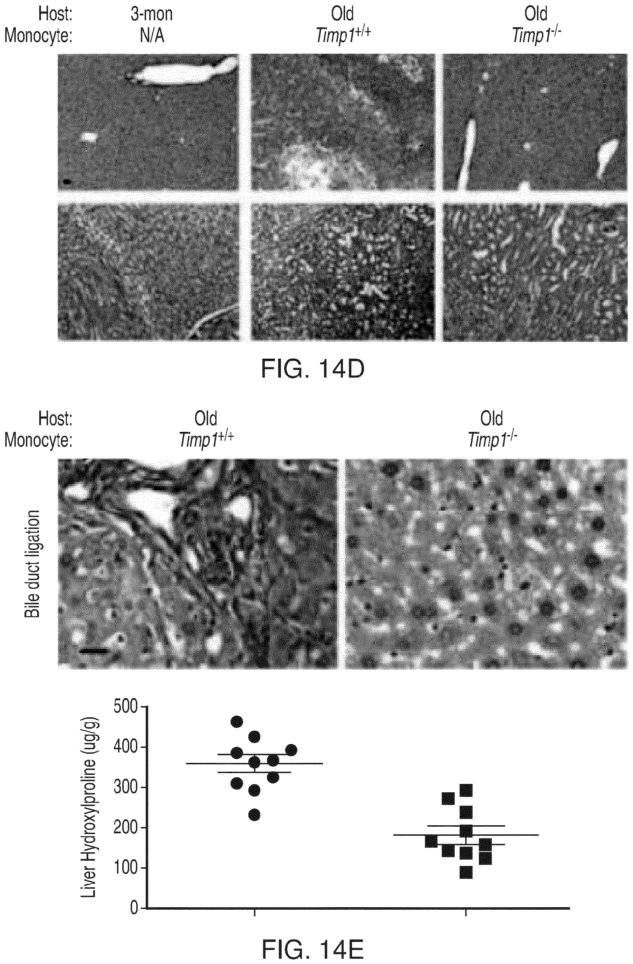

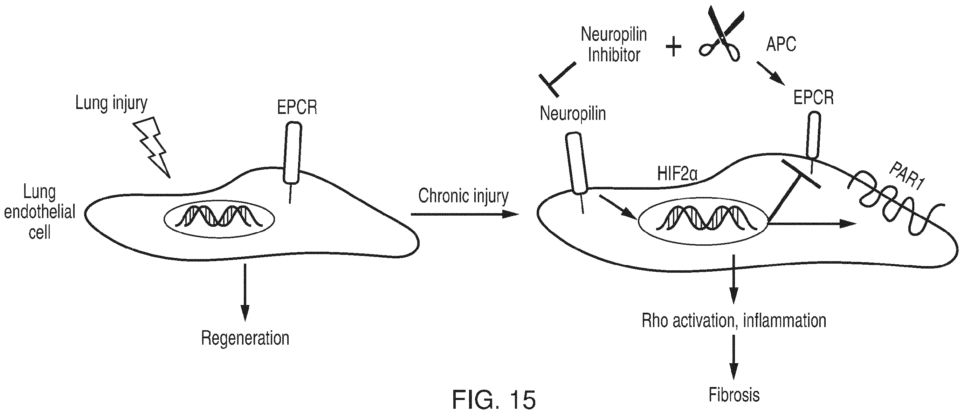

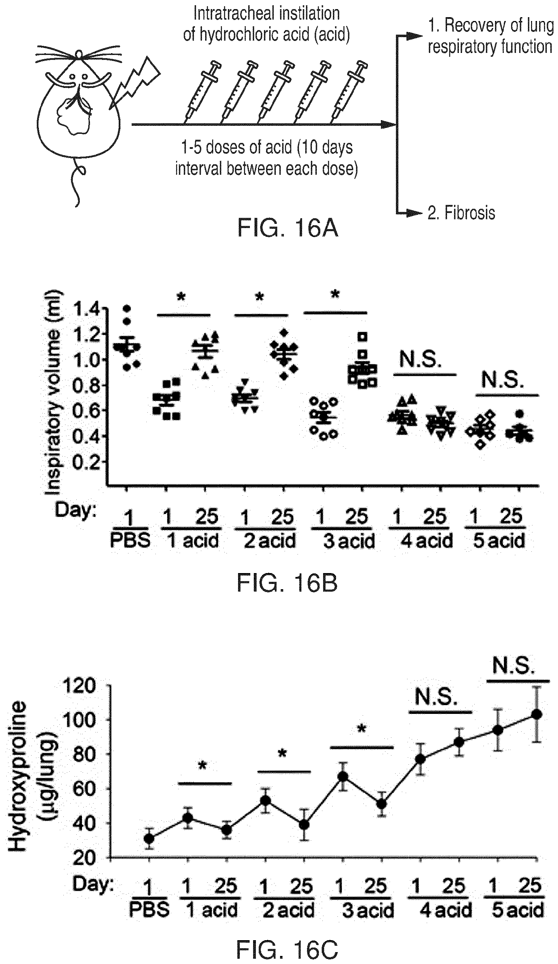

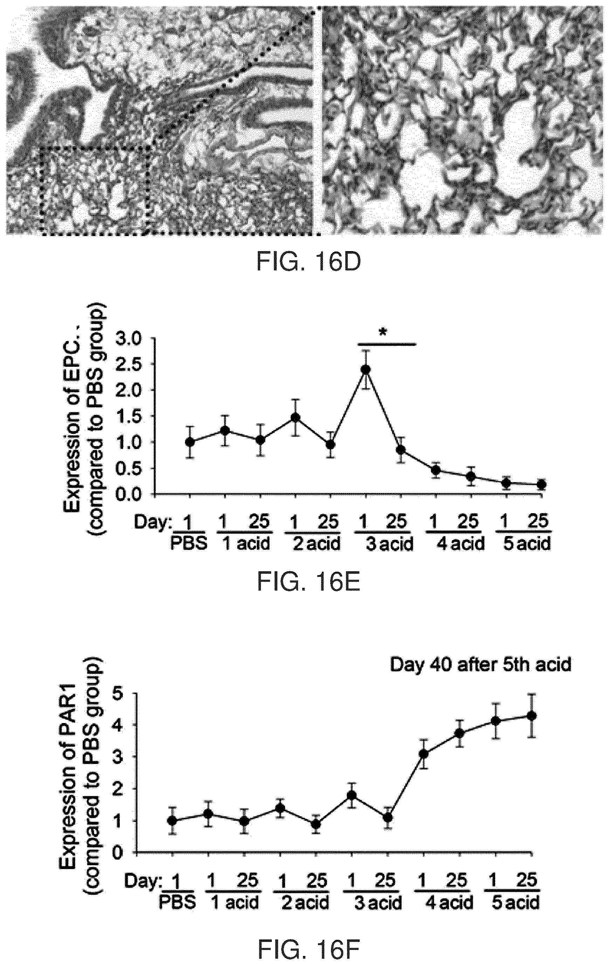

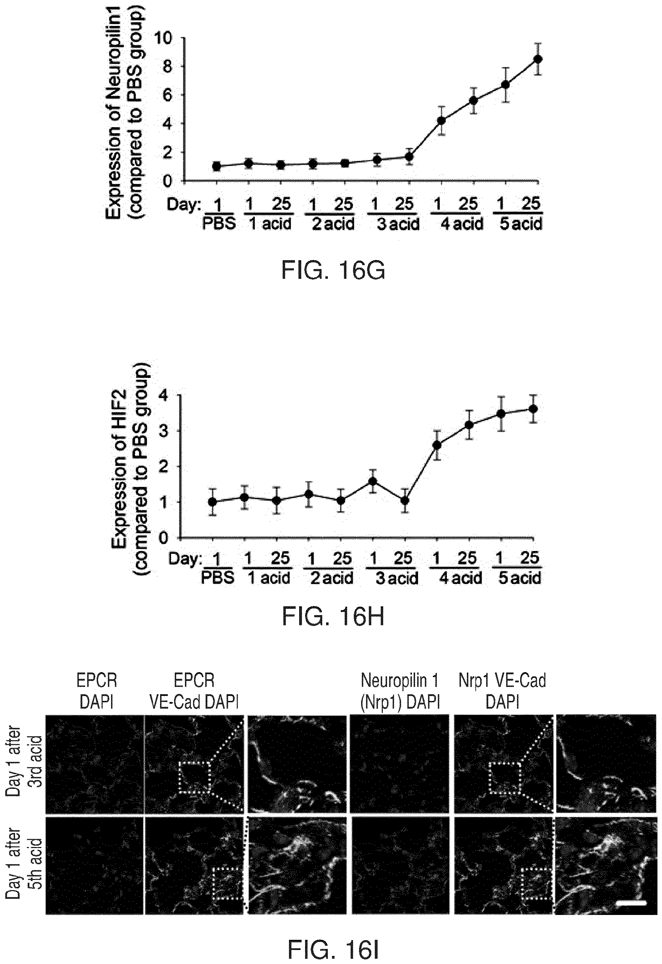

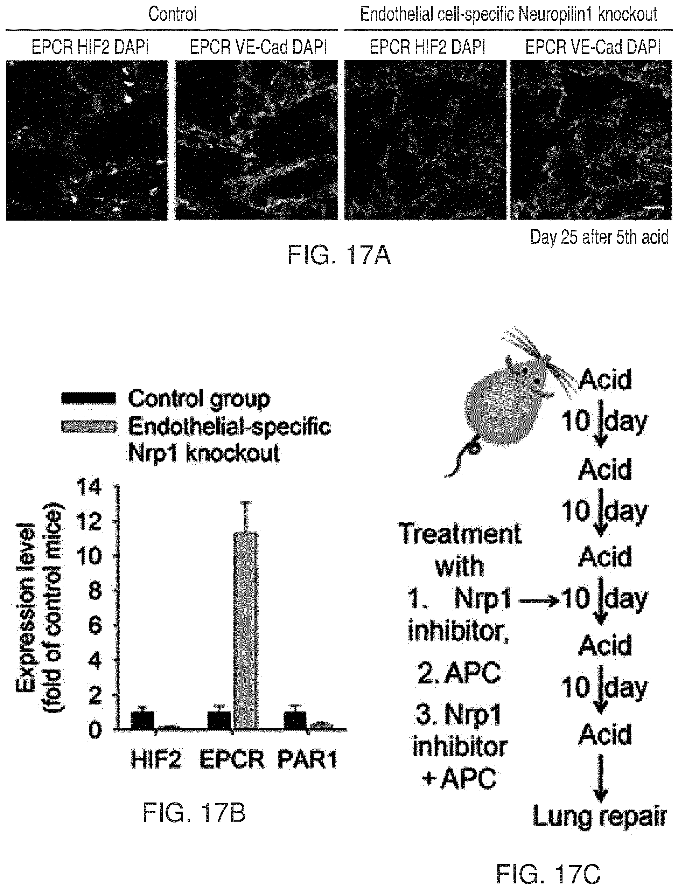

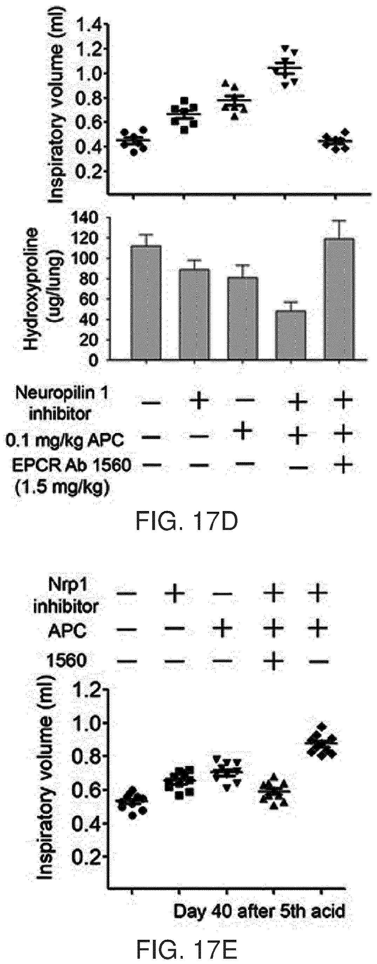

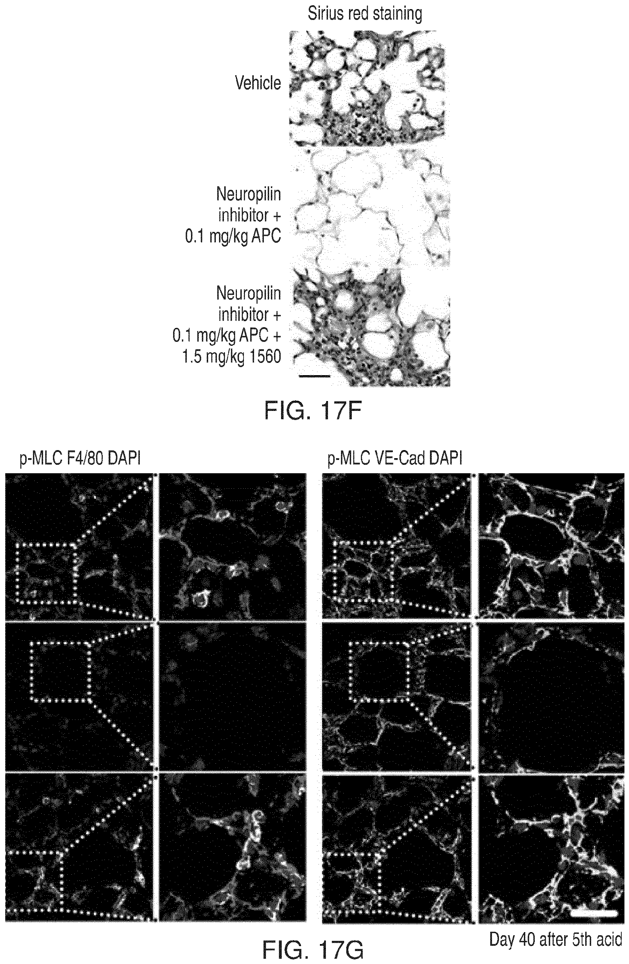

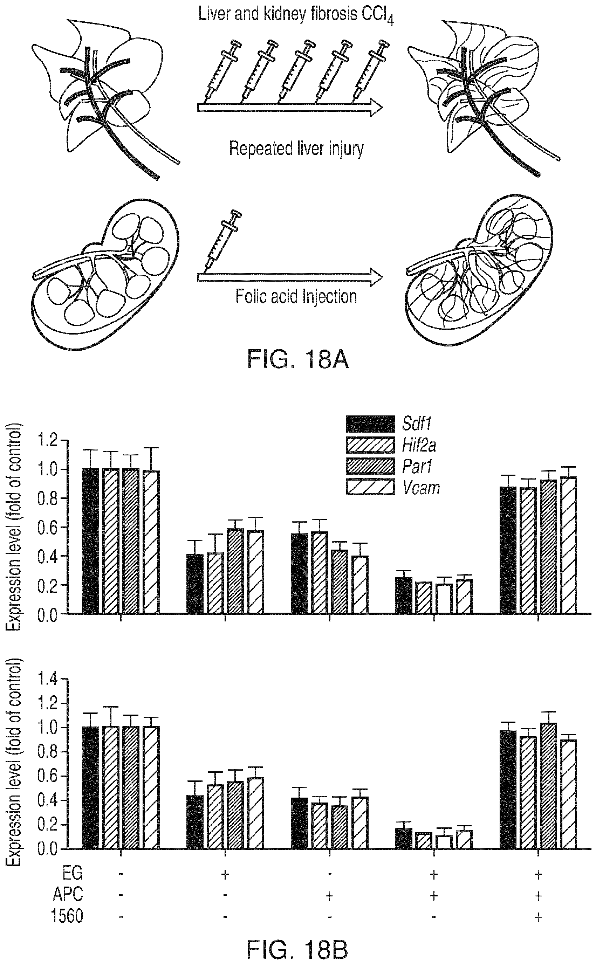

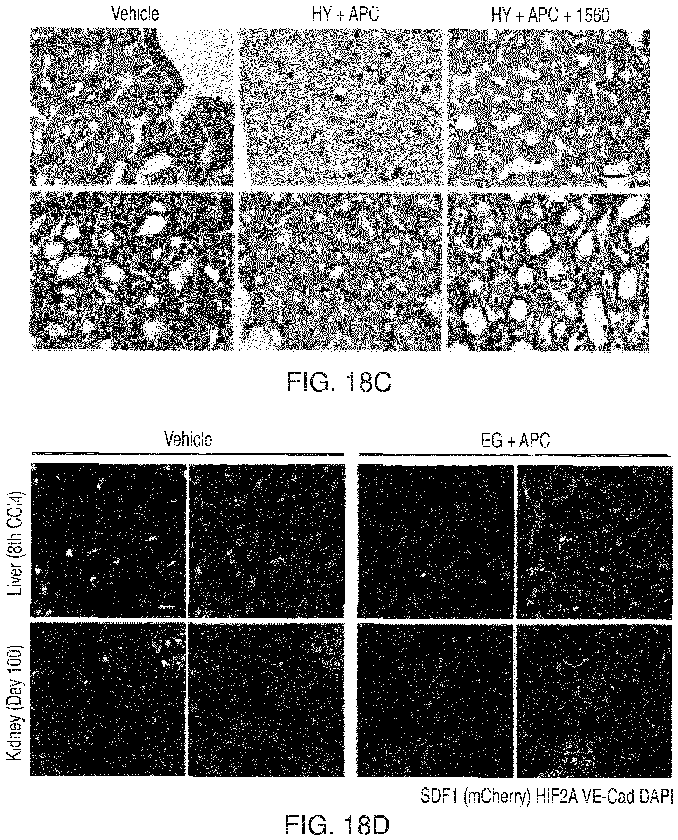

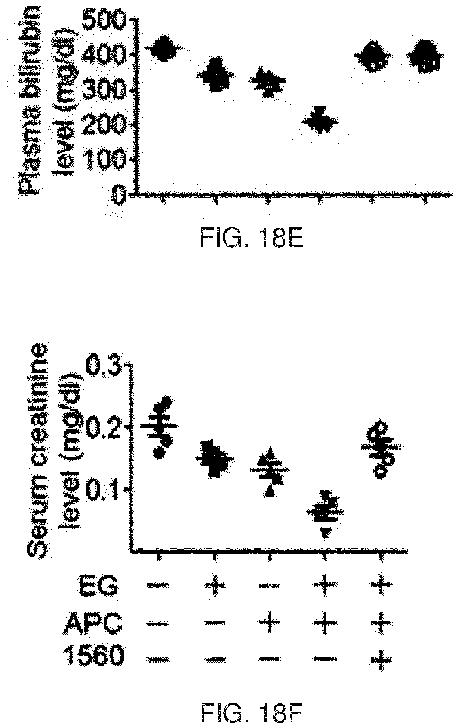

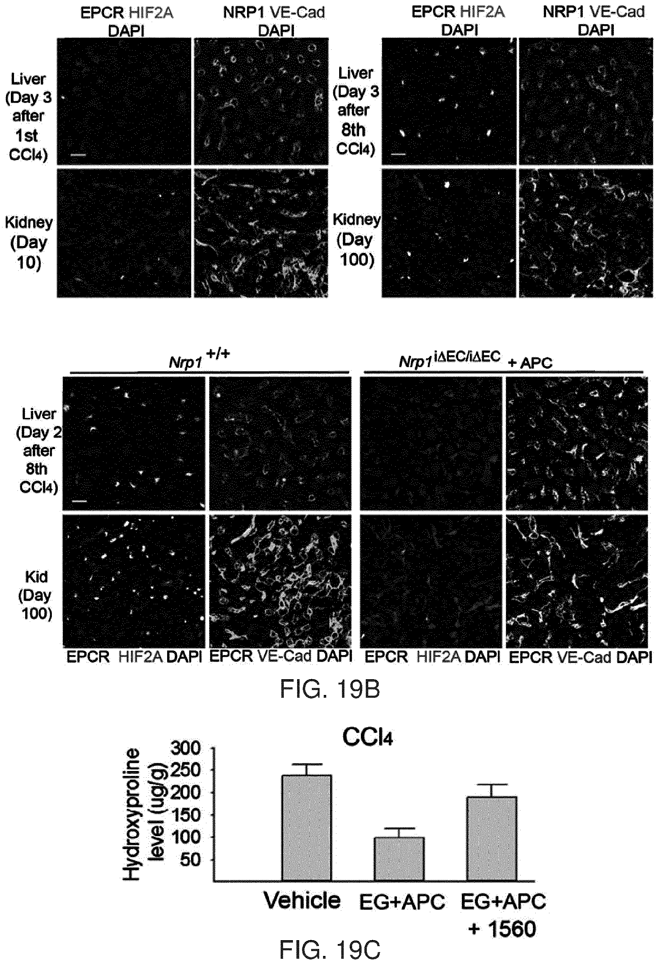

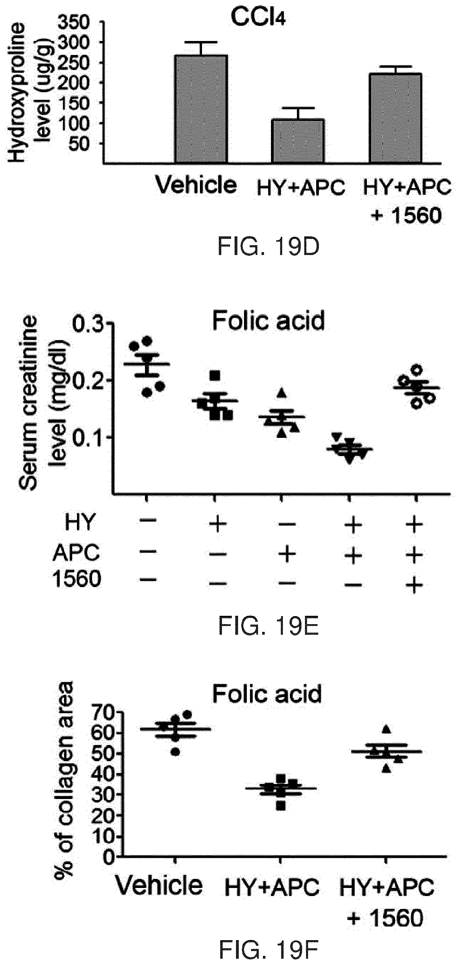

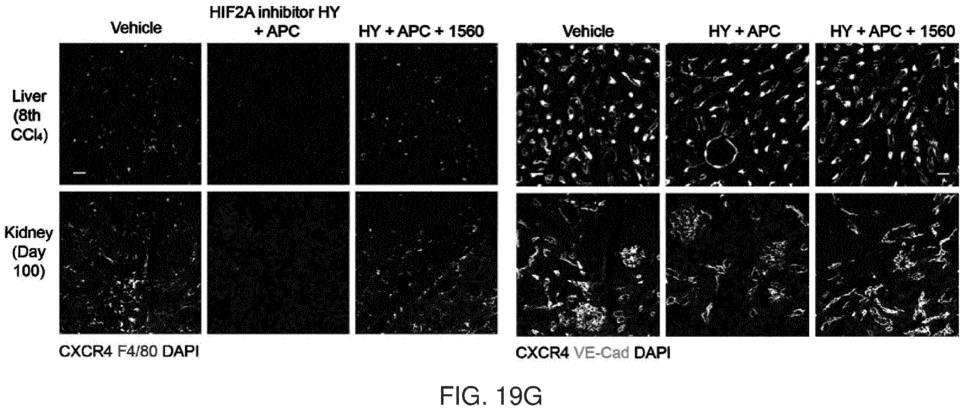

. To make the embodiments of the present invention or the technical solutions in the prior art clearer, the drawings required to be used in the description of the embodiments or the prior art will be briefly introduced below. It is obvious that the drawings described below are some embodiments of the present invention, and that other drawings can be obtained from these drawings for those of ordinary skill in the art without making inventive effort.
. FIG. 1 (FIG. 1A - FIG. 1I) is an experiment plot showing aberrant induction of NRP1 in aged ECs subverts regeneration to fibrosis in the lung;
. FIG. 2 (FIG. 2A - FIG. 2R) is an experiment plot showing reprogrammed EPCR signaling in the ECs of aged liver and kidney prohibits regeneration and stimulates fibrosis;
. FIG.3 (FIG. 3A - FIG. 3H) is an experiment plot showing suppression of EPCR by endothelial NRP1 upregulates stromal-derived factor 1 (SDF1) to recruit pro-fibrotic macrophages in old mouse organs;
. FIG.4 (FIG. 4A - FIG. 4K) is an experiment plot showing tissue inhibitor of metalloproteinases from macrophages/monocytes promotes fibroblast activation and enhances fibrosis;
. FIG. 5 (FIG. 5A - FIG. 5L) is an experiment plot showing platelet-derived Il-1α interacts with macrophages to form pro-fibrotic hematopoietic rosette;
. FIG. 6 (FIG. 6A - FIG. 6L) is an experiment plot showing genetic targeting of endothelial NRP1 or platelet Il-1α attenuates fibrosis in aged lung after bleomycin injury;
. FIG. 7 (FIG. 7A-FIG. 7P) is an experiment plot showing blockage of endothelial HIF2α or platelet IL-1α reduces immunotherapy-induced pneumonitis, liver fibrosis, and kidney fibrosis in aged organs;
. FIG. 8 (FIG. 8A - FIG. 8I) is an experiment plot showing impaired lung regeneration, aberrant induction of Neuropinin-1 (NRP1)-HIF2α pathway, and suppressed Endothelial Protein C Receptor (EPCR) in the endothelial cells (ECs) of aged mouse lungs after pneumonectomy (PNX);
. FIG. 9 (FIG. 9A - FIG. 9K) is an experiment plot showing activation of NRP1-HIF2α pathway reduces EPCR expression in EC of aged liver and kidney;
. FIG. 10 (FIG. 10A - FIG. 10I) is an experiment plot showing endothelial NRP1 in old organs recruits pro-fibrotic macrophages after injury;
. FIG. 11 (FIG. 11A - FIG. 11C) is an experiment plot showing macrophage phenotypes in described mouse groups;
. FIG. 12 (FIG. 12A - FIG. 12H) is an experiment plot showing effect of EPCR neutralizing antibody and macrophage TIMP1 on lung regeneration and fibrosis in young mice;
. FIG. 13 (FIG. 13A - FIG. 13E) is an experiment plot showing aberrant NRP1 signaling in EC promotes lung fibrosis and senescence by recruiting pro-fibrotic macrophages expressing TIMP1;
. FIG. 14 (FIG. 14A - FIG. 14E) is an experiment plot showing aberrant NRP 1-HIF2α signaling in EC and pro-fibrotic function of TIMP1 in old liver and kidney after injury;
. FIG. 15 is a schematic diagram showing a model of role of the APC-EPCR pathway in promoting fibrosis-free organ repair;
. FIG. 16 (FIG. 16A - FIG. 16I) is an experiment plot showing EPCR suppression and Neuropilin1 induction in endothelial cells is associated with lung fibrosis after repeated hydrochloric acid aspiration in mice;
. FIG. 17 (FIG. 17A - FIG. 17G) is an experiment plot showing Neuropilin-1 (Nrp1) blockage restores EPCR expression in the endothelial niche, which synergistically acts with APC to promote a fibrosis-free lung repair after acid injury;
. FIG. 18 (FIG. 18A - FIG. 18F) is an experiment plot showing targeting Nrp1/HIF2α and EPCR pathway attenuates fibrosis in liver and kidney;
. FIG. 19 (FIG. 19A - FIG. 19G) is an experiment plot showing NRP1-HIF2α inhibition and APC treatment promote liver and kidney adaptive repair after chronic injury.

### Detailed Description

. To make the objective, the technical solutions and advantages of the embodiments of the present invention clearer, the technical solutions of the embodiments of the present invention will be clearly and completely described below in combination with drawings. It is obvious that the described embodiments are some of the embodiments of the present invention, not all of the embodiments. Based on the embodiments of the present invention, all other embodiments obtained by those of ordinary skill in the art without making inventive effort shall belong to the protection scope of the present invention.

. It should be noted that the term "include", "comprise" or any variant thereof is intended to encompass nonexclusive inclusion so that a process, method, article or device including a series of elements includes not only those elements but also other elements which have been not listed definitely or an element(s) inherent to the process, method, article or device. Moreover, the expression "comprising a(n) ... " in which an element is defined will not preclude presence of an additional identical element(s) in a process, method, article or device comprising the defined element(s) unless further defined.

. As used herein, the term "about" (for example, used for indicating dose rate of ionizing irradiation or irradiation time values), typically means +/-5% of the stated value, more typically +/-4% of the stated value, more typically +/-3% of the stated value, more typically, +/-2% of the stated value, even more typically +/-1% of the stated value, and even more typically +/-0.5% of the stated value.

. Throughout this application, embodiments of this invention may be presented with reference to a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as "from 1 to 6" should be considered to have specifically disclosed subranges such as "from 1 to 3", "from 1 to 4", "from 1 to 5", "from 2 to 4", "from 2 to 6", "from 3 to 6", etc.; as well as individual numbers within that range, for example, 1, 2, 3, 4, 5, and 6. This applies regardless of the breadth of the range.

### DETAILED DESCRIPTION OF DRAWINGS

. FIG. 1: (A) Schema illustrating the strategy to test lung alveolar regrowth in young and old mice after pneumonectomy (PNX). **(B-C)** Recovery of alveolar epithelial structure (B) and hydroxyproline amounts (C) in mice at indicated ages. Immunostaining of type 1 alveolar epithelial cell (AEC) marker aquaporin5 (AQP5) and podoplanin (PDPN) and Sirius red staining were performed. 20-month-old mice (designated as "old" mice) exhibited lower extent of alveolar regeneration and higher degree of fibrosis; n = 8 mice per group. Scale bars represent 50 µm unless specified. **(D)** Pulmonary deposition of platelets and macrophages in mice after PNX. Platelet marker CD41, macrophage marker F4/80, and vascular endothelial marker VE-cadherin were co-stained. In the old mouse lung, platelets, and macrophages form cellular rosette (arrowhead). **(E)** Heatmap of altered paracrine/angiocrine genes in the pulmonary capillary endothelial cells (PCECs) from 2-month-old (2-mon) and 20-month-old "old" mice after PNX. **(F)** Expression of endothelial protein C receptor (EPCR) and hypoxia inducible factor 2α (HIF2α) in old control mice and mice with inducible EC-specific deletion of *Nrp1* (*Nrp1*^{iΔEC/iΔEC}) after PNX. **(G)** Formation of platelet-macrophage rosette after PNX was attenuated in old *Nrp1*^{iΔEC/iΔEC} mice compared with control mice, which was reversed by EPCR neutralizing antibody 1560. **(H)** Regenerative and fibrotic responses in *Nrp1*^{iΔEC/iΔEC} and Nrp1^{+/+} mice at indicated ages. Expansion of type 2 alveolar epithelial progenitor cell was assessed after PNX. 5-ethynyl-2'-deoxyuridine (EDU) incorporation was co-stained with surfactant protein C (SPC). Senescence was examined with β-galactosidase activity staining. Neutrophil accumulation was assessed by immunostaining of Ly6G. Vascular permeability was determined via injection of high molecular weight dextran (F). **(I)** Upregulation of NRP1 stimulates HIF2α and suppress anti-inflammatory and anti-thrombotic EPCR in aged ECs. EPCR suppression activates platelets and macrophages to form pro-fibrotic hematopoietic rosette. Data are presented as mean ± standard error of mean (SEM). Statistical difference was determined by one-way analysis of variance (ANOVA) followed by Tukey's test as post hoc analysis. ^{∗}p < 0.05.

. FIG. 2: (A) Liver regeneration in young and old mice was tested after PH. **(B-C)** Deposition of hematopoietic rosette (B) and transcriptional level of indicated genes in ECs (C) were assessed (n = 8-9 mice per group). **(D)** HIF2α protein in the liver ECs of described mouse groups after PH. HIF2α expression was tested by co-immunostaining with endothelial nuclear marker ETS-related gene (ERG) (E). **(E-I)** Accumulation of platelet-macrophage rosette, liver cell proliferation, and fibrosis in the liver of old *Nrp1*^{iΔEC/iΔEC} and control mice after PH. Sirius red staining was carried out (E). EDU incorporation was measured to assess hepatocyte proliferation (F). Serum concentration of aspartate aminotransferase (AST) (G) (n = 7 mice per group), distribution of platelet-macrophage aggregates (H), and plasma bilirubin concentration (I) (n = 9 mice per group) in indicated mice. **(J)** Kidney repair after ischemia reperfusion (I/R) was tested in young and old mice. **(K-N)** Formation of platelet-macrophage rosette (K), transcriptional level of indicated genes (L and M), and expression of EPCR and NRP1 proteins in the kidney ECs (N) of 2-mon and 20-mon "old" mice after I/R (n = 6 mice per group). **(O-R)** Distribution of hematopoietic rosette, kidney fibrosis, cell propagation, and restoration of kidney function in old *Nrp1*^{iΔEC/iΔEC} and control mice after I/R (O). EDU incorporation was assessed to measure kidney cell proliferation (P). Platelets, macrophages, and ECs were co-stained in the kidney slides (Q), and serum creatinine was determined in described mice (R) (n = 9 mice per group). Data are presented as mean ± SEM. Statistical difference was determined by one-way ANOVA followed by Tukey's post hoc test.

. FIG. 3: (A) Expression of SDF1 in the PCECs of SDF1 reporter mice or reporter mice lacking endothelial *Nrp1.* SDF1 expression (red fluorescent protein) was co-stained with VE-cadherin in the lung sections. **(B)** Expression of indicated genes in the PCECs of old control and EC-specific *Hif2a* knockout (*Hif2a*^{iΔEC/iΔEC}) mice after PNX (n = 6 mice per group). *p < 0.05 versus old control group, #p < 0.05, compared with old *Hif2a*^{iΔEC/iΔEC} mice treated with 1560 antibody. **(C-D)** Recruitment of CXCR4⁺ macrophages and lung alveolar type 2 progenitor cell propagation in indicated mice. Quantification of macrophage is shown in (C) (n = 6 mice per group). CXCR4 was co-stained with F4/80, and EDU incorporation was measured to assess cell proliferation (n = 6 mice per group) (D). **(E)** Cytokine expression in the pulmonary macrophages of indicated mice (n = 6 animals per group). **(F-H)** Smad2 phosphorylation (p-Smad2) and α-SMA protein expression in mouse fibroblast or stellate cells. β1 integrin was silenced in fibroblast cells with shRNA (sh β1-integrin). Representative blot image is shown in (F), and protein quantification is shown in (G) and (H) (n = 5 individual samples for each group). Data are presented as mean ± SEM. Statistical difference was assessed by one-way ANOVA followed by Tukey's post hoc test.

. FIG.4: (A) Recovery of alveolar epithelial cell architecture and collagen deposition in old mice lacking *Timp1* (Timp1^{-/-}) and young and old control mice after PNX. **(B)** *Timp1* expression in the macrophage of described mice. Old mice were also treated with CXCR4 antagonist AMD3100 or vehicle (n = 5 mice per group). **(C)** Schema describing approach to test the contribution of macrophage TIMP1 to promoting aging-associated fibrosis. **(D-F)** Restoration of pulmonary function (D), arterial oxygenation (E), and hydroxyproline amounts (F) in young and old pneumonectomized mice with or without transfer of described monocytes (n = 9 mice per group). N/A: no monocyte transferred. **(G-H)** Sirus red staining in the lung (G) and liver and kidney (H) of indicated mouse groups. **(I-J)** Concentrations of plasma bilirubin (I) and serum creatinine (J) in mice with indicated treatments (n = 9 mice per group). N/A: no monocyte transferred. **(K)** After lung, liver, and kidney injury, NRP1 is induced in aged ECs to upregulate HIF2α and suppress EPCR induction. Resulting endothelial niche expresses SDF1 to enhance deposition of CXCR4⁺TIMP1^{high} macrophages. These macrophages might contribute to the pro-fibrotic function of platelet-macrophage rosette, overturning regenerative responses to fibrosis in aged lung, liver, and kidney. Statistical difference was determined by one-way ANOVA followed by Tukey's test as post hoc analysis. Data are presented as mean ± SEM.

. FIG. 5: **(A-B)** Platelet-specific deletion of *Il1α* in mice (*Il1a*^{ΔPlt/ΔPlt}). Floxed *Il1a* (*Il1a*^{loxP/loxP}) mice were bred with mice expressing platelet-specific Pf4-cre to generate *Il1a*^{ΔPlt/ΔPlt} mice (A). Expression of TIMP1 and formation of platelet-macrophage rosette were compared in described groups after PNX, PH, or kidney I/R (B). **(C-E)** Deposition of platelet-macrophage rosette in the lung (C), liver (D), and kidney (E) in young control and *Il1a*^{ΔPlt/ΔPlt} mice after indicated treatments (n = 4 mice per group). **(F-G)** TIMP1 protein expression was detected (F) and quantified (G) in indicated mouse groups (n = 4 mice per group). **(H)** Strategy to examine the influence of platelet-derived IL-1α on fibrosis in aged organs. Il1a^{+/+} and Il1a^{-/-} platelets were infused into old Thpo^{-/-} mice. Expressions of TIMP1 and collagen deposition in recipient mice were assessed. **(I-K)** Il1a1^{-/-} but not Il1a^{+/+} platelets suppressed expression of TIMP1 (I) and attenuated regeneration to fibrotic transition (J) in aged organs of recipient mice. TIMP1 expression was quantified (K) in different groups (n = 4 mice per group). **(L)** Reprogrammed endothelial EPCR signaling in aged organs leads to platelet activation. IL-1α supplied by platelets stimulates recruited macrophages, promotes formation of pro-fibrotic platelet-macrophage rosette, and subverts regeneration to fibrosis after injury. Data are presented as mean ± SEM. Statistical difference was determined by one-way ANOVA followed by Tukey's test as post hoc analysis.

. FIG. 6: (A) The experimental procedure for inducing lung injury and fibrosis by intratracheal injection of bleomycin (Bleo) to old mice. **(B)** PIGF protein expression in old mouse lung after Bleo injury (n = 6 mice per group). **(C)** HIF2α and EPCR protein expression in indicated mouse groups after Bleo injury. **(D)** Alveolar epithelial cell distribution, SMA expression, CXCR4⁺ macrophage accumulation, and senescence-associated β-galactosidase in the lung after Bleo injury. **(E-F)** Collagen I protein level (E) and restoration of pulmonary function (F) after bleomycin (Bleo) injury were tested in indicated mice (n = 10 mice per group). **(G)** Schema describing adoptive transfer of platelets and monocytes into mice after Bleo injury. **(H-1)** Deposition of platelet-macrophage aggregate (H) and expression of lung TIMP1 (I) in described mice. Transplantation of Il1a^{-/-} platelets suppressed the formation of hematopoietic-vascular rosette and TIMP1 expression in Bleo-injured mice (n = 4 mice per group). **(J-K)** Hydroxyproline amount measurement (J) and sirius red staining (K) were performed in old recipient mice transplanted with indicated monocytes after Bleo injury. N/A: no monocyte transferred. (n = 6 mice per group). **(L)** Suppression of anti-thrombotic EPCR signaling in aged ECs leads to production of IL-1α from platelets, which interacts with perivascular macrophages expressing pro-fibrotic TIMP1. Data are presented as mean ± SEM, and statistical difference was determined by ANOVA followed by Tukey's post hoc test.

. FIG. 7: (A) Mouse model of immunotherapy-induced pneumonitis. Young and old mice were treated with anti-programed cell death protein 1 (PD1) antibody and radiation. Animal morbidity and lung fibrosis were measured. **(B-C)** Lung collagen deposition (B) and animal survival (C) of indicated mice (n = 10 mice per group). **(D-F)** Lung collagen deposition (D), IL-1α concentration in bronchioalveolar lavage fluid (BALF) (E), and animal survival (F) of indicated groups. Old mice were transplanted with platelets and subjected to indicated treatments (n = 8-10 mice per group). **(G)** Approach testing the influence of platelet Il1α to fibrosis and tumor progression in the lungs after immunotherapy. Old mice were injected with LLC cells, transplanted with indicated platelets, and subjected to described treatments. **(H-I)** Pulmonary tumor load, collagen deposition (H), and lung hydroxyproline amounts (I) were assessed after indicated treatments (n = 10-12 mice per group). **(J)** Survival curve of mice transplanted with platelets (n = 10 mice per group). **(K)** Liver and kidney fibrosis induced by injection of carbon tetrachloride (CCl₄) and folic acid injection, respectively. **(L)** PIGF protein amounts in mouse liver after CCl₄ injury (n = 8 mice per group). **(M)** Collagen I protein amounts in the liver of described mice. (N) Sirius red staining of liver and kidney of indicated mouse groups. **(O)** Formation of hematopoietic rosette in old recipient mice transplanted with indicated platelets after CCl₄ or folic acid treatment. **(P)** Liver hydroxyproline of old recipient mice injected with described monocytes after CCl₄ injury (n = 6 mice per group). Data are presented as mean ± SEM, and statistical difference was determined by ANOVA followed by Tukey's post hoc test.

. FIG. 8: FIG. 8 is a supplement to the experiment shown in FIG. 1 and is related to it. **(A-C)** Recovery of pulmonary function and deposition of collagen and platelet-macrophage rosette in mice at denoted ages after PNX. Blood oxygenation and inspiratory volume were measured. Sirius red staining and Collagen I immunoblot were performed, and formation of platelet-macrophage aggregate was measured. 20-month-old (old) mice exhibited lower alveolar regeneration and higher fibrosis. In all drawings, each dot in the dot plot denotes a datum from an individual animal. Sh, sham-operated groups; PNX, pneumonectomy group. **(D)** Altered paracrine-related genes in pulmonary capillary endothelial cells (PCECs) from aged mouse lungs after PNX, compared with that of 2-month-old mouse lungs. N = 5-6 per group. Scale bar = 50 µm in FIG. 8. **, P < 0.05 versus old group. Statistical difference was determined by one-way analysis of variance (ANOVA) followed by Tukey's post hoc test. **(E)** Expression of neuropilin1 in lung and liver ECs was compare between old control and mice with inducible endothelial cell (EC)-specific deletion of *Nrp1* (*Nrp1*^{iΔEC/iΔEC})*.* **(F)** Expression of EPCR and HIF2α in old *Nrp1*^{iΔEC/iΔEC} and control mice after PNX. 3-month-old (young) and 20-month-old (old) mice were compared, respectively. N = 5 mice per group. **(G)** Formation of platelet-macrophage rosette was attenuated in old *Nrp1*^{iΔEC/iΔEC} mice compared with that in control mice, which was reversed by EPCR neutralizing antibody 1560. N = 6 mice per group. **(H)** Hydroxyproline amounts, β-Gal activity, and p16 expression in the lungs of indicated mouse groups were quantified. * *P* < 0.05, statistical difference was determined by ANOVA followed by Tukey's post hoc test. **(I)** Vascular growth and perfusion, perivascular neutrophil distribution, vascular leak, and lung type 2 alveolar progenitor proliferation in indicated mouse groups after PNX. B4-isolectin and Evans blue was intravenously injected to mice. Isolectin⁺ vessels and residual Evans blue in the lung were quantified. Scale bars = 50 µm.

. FIG. 9: FIG. 9 is a supplement to the experiment shown in FIG. 2 and is related to it. (**A**) Liver regeneration in young and old mice was tested after partial hepatectomy (PH). Hydroxyproline amounts in the liver were measured in indicated groups. (**B-C**) Perivascular neutrophil distribution in indicated groups after PH or kidney ischemia-reperfusion (I/R). (**D**) EPCR protein level in the lung ECs of young *Nrp1*^{+/+}, old *Nrp1*^{+/+}, and old *Nrp1*^{iΔEC/iΔEC}mice after sham and PNX. EPCR protein quantification is shown in the bar graph. N = 4-5 per group. (**E**) PlGF protein quantity in the lung and liver from indicated mouse groups after PNX and PH. N = 4 per group. (**F**) EPCR protein level in the lung ECs of indicated mouse groups after injection of PIGF and equal amounts of platelet-derived growth factor-BB (PDGF), hepatocyte growth factor (HGF), vascular endothelial growth factor 165 (VEGF), tumor growth factor β1 (TGFβ1). N = 4. (**G**) PlGF-mediated EPCR protein suppression in lung ECs from 3-mon control, *Nrp1*^{iΔEC/iΔEC}mice and mice with EC-specific inducible knockout of *Hif2a* (*Hif2a*^{iΔEC/iΔEC})*.* N = 4 per group. (**H**) EPCR suppression by PIGF in the liver ECs from indicated mice. N = 4 per group. (**I**) Expression of NRP1 protein in ECs of indicated mice after PNX, PH, or Kidney I/R. Old mice were treated with histone deacetylase (HDAC) inhibitor (VPA), DNA Methyltransferase (DNMT) inhibitor azacitidine (AZA), or co-injection of VPA and AZA (VPA+AZA). Veh: vehicle. N = 5. (**J**) PlGF protein quantity in indicated mouse groups. Veh: vehicle; N = 5-6 per group. (**K**) In old mouse organs, epigenetically upregulated PlGF-NRP1 pathway suppresses endothelial EPCR induction after injury.

. FIG. 10: FIG. 10 is a supplement to the experiment shown in FIG. 3 and is related to it. (**A**) Expression of indicated genes in PCECs of old *Nrp1*^{iΔEC/iΔEC} and control mice after PNX. N = 5. (**B**) Transcriptional expression of SDF1 in fibroblasts from the lungs of indicated mice after PNX. N = 6. (**C, D**) Recruitment of CXCR4⁺ macrophages in mouse lung after PNX. CXCR4-expressing macrophages were detected by co-staining of CXCR4 and F4/80. N = 6. (**E**) Lung cell proliferation in described mouse groups. (**F**) Amounts of hydroxyproline in the indicate mouse livers after PH. Statistical difference was determined by one-way ANOVA followed by Tukey's post hoc test. (**G**) Expression of indicated gene in EC of old *Nrp1*^{iΔEC/iΔEC} mice and young and old control mice. N = 5. **P* < 0.05 versus old *Nrp1*^{+/+} group, # *P* < 0.05, compared with old *Nrp1*^{iΔEC/iΔEC}mice treated with 1560. (**H**) Quantity of CXCR4⁺ macrophages in denoted mice. Liver and kidney sections were stained for CXCR4 and F4/80 after PH and kidney I/R. N = 5. (**I**) Normalizing EPCR signaling in old PCECs blocks the pro-fibrotic function of vascular niche. NRP1-HIF2α signaling in old mouse PCECs suppresses EPCR to induce SDF1 expression after PNX. Expression of SDF1 in PCECs recruits pro-fibrotic TIMP1 perivascular macrophages to impede alveolar regeneration and promote fibrosis in old pneumonectomized lungs.

. FIG. 11: FIG. 11 is a supplement to the experiment shown in FIG. 4 and is related to it. **(A)** Expression of individual markers in the described macrophages. Macrophages were isolated from the lung, liver, and kidney from 3-mon (young) and old mice after PNX, PH, or kidney I/R. **(B)** Expression of indicated genes in old control and *Hif2α*^{iΔEC/iΔEC} mouse macrophages from aged organs. **(C)** Co-staining of F4/80 and CD86 in the lung, liver, and kidney of indicated mouse groups. Scale bar = 50 um.

. FIG. 12: FIG. 12 is a supplement to the experiment shown in FIG. 5 and is related to it. (**A-B**) 3-month old mice (young) were injected with EPCR neutralizing antibody 1560 after PNX. Restoration of type 1 alveolar epithelial cell and blood oxygenation were measured. (**C-D**) Collagen deposition and hydroxyproline level in the young lungs after PNX and 1560 injection. Yong mice were at 3-month-old age. N = 6, and scale bars = 50 µm. (**E**) Enhanced accumulation of platelet-macrophage aggregates in the young mouse lungs after EPCR antibody injection, relative to mice treated with isotype-matched IgG. (**F**) Strategy to test the contribution of macrophage TIMP1 to lung fibrosis after EPCR blockage. Monocytes were isolated from *Timp1 - null (Timp1*^{-/-} ) and control (*Timp1*^{+/+}) mice and transferred to young mice after EPCR antibody injection. (**G-H**) Transplantation of *Timp1*^{*-*/*-*} but not *Timp1*^{+/+}monocytes reduced the increase of lung fibrosis after EPCR antibody blockage. Collagen deposition and hydroxyproline amounts in the mouse lungs were analyzed. N = 5, and scale bars = 50 µm.

. FIG. 13: FIG. 13 is a supplement to the experiment shown in FIG. 6 and is related to it. (**A**) Recruitment of CXCR4⁺ macrophage in mouse groups with indicated genotypes and treatment after intratracheal injection of Bleomycin (Bleo). (**B-C**) β-galactosidase and p16 expression level in indicated mouse groups after Bleo injury. N = 5-6 mice per group. (**D-E**) Amounts of hydroxyproline, alveolar epithelial cell regeneration, collagen I deposition in old *Timp1*^{-/*-*}, young and old control *Timp1*^{+/+}mice after Bleo injury. Yong mice were at 3-month-old age, and scale bars = 50 µm.

. FIG. 14: FIG. 14 is a supplement to the experiment shown in FIG. 7 and is related to it. **(A)** Expression of HIF2α and EPCR in the liver and kidney of mouse groups with indicated genotypes after CCl₄ or folic acid injury. (**B**) Hydroxyproline amounts in the liver of indicated mouse groups after CCl₄ injury. (**C**) Expression of pro-fibrotic TIPM1 in old host/recipient *Thpo*^{*-*/*-*} mice transplanted with *Il1a*^{*-*/*-*} or *Il1a*^{+/+}platelets after CCl₄ or folic acid treatment. TIMP1 expression in either liver or kidney was not affected by EPCR antibody in *Thpo*^{*-*/*-*} mice transferred with *Il1a*^{*-*/*-*}platelets after liver or kidney injury, implying that IL1α production by platelets is downstream of EPCR suppression. (**D**) Collagen deposition in old *WT* mice transplanted with *Timp1*^{*-*/*-*} or *Timp1*^{+/+} monocytes after CCl₄ or folic acid treatment. (**E**) Liver hydroxyproline and Sirius red staining of old *WT* mice transferred with indicated monocytes after bile duct ligation (BDL), a cholestatic liver injury model. Scale bars = 50 µm.

. FIG. 16: (A) The experimental scheme for inducing lung injury by single or multiple intratracheal instillations of hydrochloric acid (acid). (**B-C)** Restoration of respiratory function (inspiratory volume) and fibrosis (hydroxyproline) in the injured lungs were assessed after acid instillation. Each dot denotes data from individual mouse. (**D**) Collagen protein deposition was tested by Sirius red staining in indicated mouse lungs. (**E-H**) Expression of EPCR, PAR1, Neuropilin1, and HIF2 in lung endothelial cells after each acid injection. Lung endothelial cells were isolated as previously described, and gene expression was tested by quantitative PCR. N = 6-8 mice per group. (**I**) EPCR and Neuropilin1 (Nrp1) proteins in the lungs of indicated mouse groups were tested by Immunostaining. Scale bar = 50 um.

. FIG. 17: (A) Expression of EPCR and HIF2 proteins in VE-cadherin⁺ lung endothelial cells of mice with endothelial cell-specific *Nrp1* knockout. Floxed Nrp1 mice were crossed with mice expressing tamoxifen-responsive endothelial cell-specific VE-cadherin-Cre^{ERT2}. Treatment with resulting mice with tamoxifen induced specific deletion of Nrp1 in endothelial cells. Mice with endothelial haplodeficiency of *Nrp1* were used as control. Expression of HIF2 and EPCR was tested by immunostaining in mice at indicated time after acid injury. (**B**) Transcriptional expression of HIF2 and EPCR in lung endothelial cells of control mice and mice with endothelial-specific Nrp1 knockout. N = 6 mice per group. (**C**) Schema depicting strategy to test the therapeutic effect of Nrp1 inhibitor, activated protein C (APC), and combination of Nrp1 inhibitor of APC (Nrp1 inhibitor + APC). (**D**) Nrp1 inhibitor + APC treatment promoted lung repair and bypasses fibrosis. Restoration of respiratory function and deposition of hydroxyproline were tested in the mouse lungs at indicated time. Of note, therapeutic efficacy of Nrp1 inhibitor + APC was attenuated by EPCR blocking antibody 1560. (**E**) Restoration of pulmonary function (inspiratory volume) in acid-injured mice after treatment with Nrp1 inhibitor, APC, or APC + Nrp1 inhibitor. (**F-G**) Co-treatment of Nrp1 inhibitor and APC decreased Collagen I deposition (F) and reduced recruitment of F4/80⁺ macropage/mococytes (G) in acid-injured mouse lungs. Meanwhile, Nrp1 inhibitor + APC decreased Rho activation in lung endothelial cell, as demonstrated by co-staining of phosphorylated myosin light chain (p-MLC) and VE-cadherin. Scale bar = 50 µm.

. FIG. 18: (**A**) Liver and kidney fibrosis induced by repeated injection of carbon tetrachloride (CCl₄) and folic acid injection, respectively. Mouse liver fibrosis was induced by eight intraperitoneal injection of CCl₄ every three days, and kidney fibrosis was induced by folic acid injection. (**B**) Expression of EPCR suppression and Nrp1 induction in EC is associated with liver and kidney fibrosis. (**C**) Combination of HIF2α inhibitor HY and APC blocks liver and kidney fibrosis. Therapeutic effects of double hit strategy targeting Nrp1 (or HIF2α) and EPCR were tested in mouse liver and kidney fibrosis models. Mice were treated with APC, Nrp1 inhibitor EG, HIF2α inhibitor HY, or EPCR neutralizing antibody 1560. (**D**) Expression of HIF2, EPCR, Nrp1 in liver and kidney at indicated time point after injury and described treatments. EC markers VE-cadherin and ERG were also stained to test vascular distribution of tested molecules. (**E-F**) Recovery of hepatic and renal functions after injections of CCl₄ and folic acid and indicated treatments.

. FIG. 19: (**A**) EPCR suppression and NRP1-HIF2α activation in ECs are associated with liver and kidney fibrosis. EC marker VE-cadherin was stained to determine vascular distribution of tested molecules. Scale bar = 50 um in FIG. 19. (**B**) Expression of HIF2α and EPCR in liver and kidney of *Nrp1*^{1ΔEC/1ΔEC} mice after injury and described treatments. (**C-D**) Hepatic hydroxyproline amounts in the injured liver at day 20 after 8th CCl₄ with indicated treatments. N=8-9 mice per group. HY, HIF2α inhibitor, HIF-2α-IN-1(HY-19949). (**E**) Serum creatinine quantity in kidney at day 100 after folic acid injection and described treatments. N = 8 mice per group. (**F**) Percentage of fibrotic area in the injured mouse kidney at day 100 after folic acid injection with described treatments. N = 6 mice per group. (**G**) Distribution of CXCR4⁺ macrophages in injured mouse liver and kidney with described treatments. Liver and kidney sections were stained with CXCR4, F4/80, and VE-cadherin after CCl₄ and folic acid injection and described treatments.

### Embodiment 1: Methods and Materials

.

**Table 1 Key Resources**

| REAGENT or RESOURCE | SOURCE | IDENTIFIER |
|---|---|---|
| Experimental Models: Organisms/Strains | | |
| Mouse: Floxed *Nrp1* | The Jackson Laboratory | Stock#005247 |
| Mouse: Floxed *Hif2α* | The Jackson Laboratory | Stock#008407 |
| Mouse: Timp1 knockout | The Jackson Laboratory | Stock#006243 |
| Mouse: Cdh5-Cre^{ERT2} | Reference 65 | N/A |
| Mouse: Pf4-Cre | The Jackson Laboratory | Stock#008535 |
| Mouse: Floxed *Il1a* | GemPhamatech | N/A |
| Mouse: SDF1 reporter | The Jackson Laboratory | Stock#022458 |
| Mouse: C57BL6/J | The Jackson Laboratory | N/A |

| Antibodies | | |
|---|---|---|
| Rabbit polyclonal anti-pSmad2 | CST | Cat#18338; RRID: AB_2798798 |
| Rabbit polyclonal anti-Collagen | Abeam | Cat#ab34710; RRID: AB_731684 |
| Rabbit polyclonal anti-SMA | Abeam | Cat#ab5694; RRID: AB_2223021 |
| Rabbit polyclonal anti-desmin | Abeam | Cat#ab15200; RRID: AB_301744 |
| Goat polyclonal anti-VE-Cadherin | R&D | Cat#AF1002; RRID: AB _2077789 |
| Rabbit polyclonal anti-SFTPC | Abeam | Cat#ab28744; RRID: AB_777472 |
| Rabbit polyclonal anti-Aquaporin | Abeam | Cat#ab78486; RRID: AB_1603410 |
| Goat polyclonal anti-Podoplanin | R&D | Cat#AF3244; RRID: AB_2268062 |
| Rat monoclonal anti-CD41 | BD Biosciences | Clone MWReg30; RRID: AB_395084 |
| Rabbit polyclonal anti-ERG | Abeam | Cat#Ab92513; RRID: AB_2630401 |
| EPCR blocking antibody | N/A | PMID 15978090 |
| AF647 donkey anti-rabbit IgG | Jackson ImmunoResearch Labs | Cat#711-605-152; RRID: |
| | | AB_2492288 |
| AF488 donkey anti-rabbit IgG | Jackson ImmunoResearch Labs | Cat#711-545-152; RRID: |
| | | AB_2313584 |
| AF647 donkey anti-goat IgG | Jackson ImmunoResearch Labs | Cat#705-605-147; RRID: |
| | | AB_2340437 |
| AF488 donkey anti-goat IgG | Jackson ImmunoResearch Labs | Cat#705-545-147; RRID: AB_2336933 |

| Deposited data | | |
|---|---|---|
| Affymetrix raw data | The present application | GEO (accession number: GSE159773) |

| Software | | |
|---|---|---|
| Image J 1.51 s | NIH | https://imagej.nih.gov/ij/ |
| Graphpad Prism 8.0 | Graphpad Software | https://www.graphpad.com/scientificso ftware/ prism/ |
| Photoshop | Adobe | https://www.adobe.com/product s/ photoshop.html |

| Chemicals, Peptides, and Recombinant Proteins | | |
|---|---|---|
| Tamoxifen | Sigma-Aldrich | Cat#T5648 |
| Corn Oil | Sigma-Aldrich | Cat#C8267 |
| Collagenase I | Roche | Cat#11088793001 |
| Dispase II | Roche | Cat#04942078001 |
| RIPA Lysis Buffer | Santa Cruz | Cat#sc-364162 |
| Phosphatase Inhibitor Cocktail | Roche | Cat#04906837001 |
| Protease Inhibitor Cocktail | Roche | Cat#04693159001 |
| Lipofectamine^{™} | Invitrogen | Cat#13778150 |
| Bleomycin | Millipore | Cat#203401 |
| PlGF | Novoprotein | Cat#CW90 |
| HGF | Novoprotein | Cat#CJ72 |
| VEGF165 | Novoprotein | Cat#C083 |
| TGF-b1 | Abeam | Cat#Ab86482 |
| TIMP1 | Abeam | Cat#Ab92486 |
| Azacitidine | Meilun Biotechnology | Cat#MB1022 |
| VPA | Meilun Biotechnology | Cat#MB1627 |
| Carbon Tetrachloride | Sigma-Aldrich | Cat#319961 |
| EndoGRO-VEGF Complete Culture Media Kit | Millipore | Cat#SCME002 |
| DMEM | Gibco | Cat#11965092 |
| DAPI | Invitrogen | Cat#D1306 |
| Ketamine | Akorn | NDC:59399-114-10 |
| Xylazine | Akorn | NDC:59399-110-20 |
| PFA | Electron Microscopy Sciences | Cat# 50-980-487 |
| Tween80 | Sigma-Aldrich | Cat#P1754 |
| Goat Anti-Rabbit IgG Magnetic Beads | New England Biolabs | Cat#S1432S |
| Dynabeads^{™} Sheep-Anti Mouse IgG | Invitrogen | Cat# 11031 |

| Critical Commercial Assays | | |
|---|---|---|
| Mouse Il1a Elisa Kit | R&D | Cat# DLA50 |
| Hydroxyproline (Hyp) Kit | Abeam | Cat# ab222941 |
| RNeasy Mini Kit | QIAGEN | Cat# 74104 |
| EDU Proliferation Kit | BD Biosciences | Cat# 565456 |

| Oligonucleotides | | |
|---|---|---|
| shRNA targeting sequence: b1 integrin | The present application | N/A |
| 5'- GGGGA ATTCA AAAC CAAGTTTCAA GGGCC AACTTCTCGAG AAGTT GGCCCTTGAA ACTTG GCCGGTGTTT CGTCC TTTC -3' | | |
| | | |
| | | |

. **Animal Care and Mouse lines used.** Floxed Neuropilin1 (*Nrp1*) and HIF2α (*Hif2α*) mice and mice deficient of tissue inhibitor of metalloprotease-1 (*Timp1*), platelet factor-Cre (*Pf4-Cre*) mice, were obtained from Jackson lab. Mice expressing EC-specific Cdh5-(PAC)-Cre^{ERT2}/VE-Cadherin-Cre^{ERT2} were provided by Dr. Ralf Adams. This mouse line was crossed with floxed *Nrp1* and *Hif2a* mice to generate *Nrp1*^{iΔEC/iΔEC}, *Hif2α*^{iΔEC/iΔEC} and control mice. Four week old mice were intraperitoneally (i.p.) treated with tamoxifen at a dose of 250 mg/kg for 10 days, and interrupted for a week after the third and the sixth dose (or at a dose of 150 mg/kg for 6 days, and interrupted for 3 days after the third dose). Deletion of target genes in ECs was corroborated by quantitative polymerase chain reaction (PCR). *Nrp1*^{iΔEC/iΔEC} and *Hif2a*^{iΔEC/iΔEC} mice and age-matched littermate mice were used for mouse lung, liver and kidney injury models. Floxed *Il1a* mouse was generated with CRISPR/Cas9 system by GemPhamatech, Nanjing, China. Cas9 mRNA, sgRNA and donor were coinjected into zygotes. sgRNA directed Cas9 endonuclease cleavage in intron 2-3 and 4-5, and created a double-strand break. Such breaks resulted in loxP sites by homologous recombination, and regions of exon3 and exon4 were floxed. *Il1a* floxed mice were mated with mouse expressing platelet factor 4-Cre. Thus, sequence between two loxP sites were specifically deleted in platelets, and *Il1a* gene was disrupted by frame shift mutation. To stimulate EPCR signaling, mice were i.p. treated with 0.1 mg/kg APC every three days. At the same time, EPCR signaling was blocked by i.p. injection of neutralizing antibody 1560 at 1.5 mg/kg every three days to verify the specificity of the EPCR pathway. To inhibit NRP1 or HIF2α, mice were i.p. injected with 50 mg/kg EG00229 or 25 mg/kg HIF-2α-IN-1 (HY-19949) every three days, respectively.

. In the embodiments of the present invention, the main reason for selecting EG00229 and its derivatives (EG00229 (N²-[ [3-[ (2,1,3-Benzothiadiazol-4-ylsulfonyl)amino]-2-thienyl]carbonyl]-L-arginine) and EG00229 trifluoroacetate (N²-[[3- [(2,1,3-Benzothiadiazol-4-ylsulfonyl)amino] -2-thienyl] carbonyl] -L-arginine trifluoroacetate) and HIF-2α-IN-1 (3-[[(3R)-4-(Difluoromethyl)-2,2-difluoro-3-hydroxy-1,1-dioxo-3H- 1-benzothiophen-5-yl]oxy]-5-fluorobenzonitrile) is that the above inhibitors can effectively inhibit NRP1 and HIF2α in vivo, which can be used to prove the technical effects that can be produced by inhibiting NRP1 and HIF2α. However, it is obvious to those skilled in the art that the technical solution of the present invention is not limited to the use of the above inhibitors. From the experimental results of knocking out NRP1 or HIF2α in FIGs. 1-3, 6-11, 13-14, 17, 19, etc., it can be seen that technical solution that achieve the above inhibitory effects such as a substance that can effectively inhibit NRP1 and HIF2α (including suitable small molecule drugs and antibodies) or editing or knocking out the corresponding genes (for example, NRP1, HIF2α) should be within the scope of the present invention. Similarly, the reason for selecting activated protein C (APC) is that APC can effectively stimulate EPCR signaling and promote the expression of EPCR in vivo. However, it can be seen from the experimental results that the beneficial effects of APC are blocked after EPCR signaling blocked by EPCR neutralizing antibody 1560, the key to the technical solution of the present invention is to activate the EPCR signaling pathway. Therefore, for those skilled in the art, any technical solution that can stimulate EPCR signaling and/or promote EPCR expression should be within the protection scope of the present invention and is not limited to the use of APC. All animal experiments were carried out by protocols approved by the Institutional Animal Care and Use Committee at Sichuan University.

. **Mouse lung regeneration and repair models.** To test mouse lung alveolar regeneration and repair, models of left lung PNX (Reference 19) and lung injury (Reference 7) were adapted. Mice were anesthetized with a cocktail of ketamine (100 mg/kg) and xylazine (10 mg/kg). PNX was carried out when left lung lobe was resected with a suture tied around the hilum. PCECs were purified from 20 months (designated as old) and 3 months old (designated as young) mice after PNX, and total RNA was isolated and subjected to transcriptome analysis as described. Intratracheal injection of 2U/kg bleomycin was employed to induce lung injury. To block EPCR signaling, mice were intraperitoneally injected with EPCR neutralizing antibody 1560 at 1.5 mg/kg (Reference 46) after PNX and Bleo injection. Young *Nrp1*^{iΔEC/iΔEC}, *Hif2α*^{iΔEC/iΔEC}, and control mice were also administered with 20 µg/kg mouse PlGF or same molar amounts of HGF and PDGF-BB (Peprotech) after PNX and PH. In addition, repeated intratracheal hydrochloric acid injection models were employed to induce lung injury (Reference 76). Orotracheal instillation was performed in anesthetized mice, and 20 µl of an iso-osmolar solution of 0.1 M hydrochloric acid (pH 1.0) was instilled. After each injection, mice were observed to ensure the full recovery from anesthesia, and body temperature was maintained using external heat sources. After recovery, mice were transferred to ventilated cages with access to food and water. Mice were administered with 0.1 mg/kg APC, 50 mg/kg NRP1 inhibitor, 25 mg/kg HIF2α inhibitor, and 1.5 mg/kg 1560 every three days after PNX and first Acid injection. At the described time points, pulmonary function and oxygen tension in arterial blood of treated mice were measured as previously described (Reference 7). To measure cell proliferation, 100 mg/kg 5-ethynyl-2'-deoxyuridine (EDU) was i.p. injected into mice one hour before sacrificing, and EDU incorporation was measured by EDU cell proliferation kit. Mice were i.p. supplemented with 1 ml PBS after all surgery procedures.

. **Mouse model of immunotherapy-induced pneumonitis.** PD1 antibody was injected to the mice at the dose of 10 mg/kg dose every four days one month after radiation. Mouse survival was monitored, and lung tissues were harvested from mice three months after radiation. BALF was collected as previously described (Reference 19). To test immunotherapy-induced pneumonitis in tumor-bearing mice, two million Lewis lung carcinoma (LLC) cells were transplanted to old *Thpo*^{*-*/*-*} mice via jugular vein. To measure the contribution of platelet IL1α, recipient mice were transplanted with *Il1a*^{+/+} or *Il1a*^{-/-} platelets after LLC cell injection. Mice were also treated with PD1 antibody and radiation. After platelet transplantation, lung fibrosis, tumor load and animal survival were analyzed in recipient mice at indicated time.

. **TIMP1 treatment of lung fibroblast and liver stellate cell.** Lung and liver fibroblast cells were from ScienceCell Research Laboratories and cultured as described previously (Reference 8). Lung and liver fibroblast cells were treated with 20 ng/ml TIMP1, 10 ng/ml TGFβ1, or 20 ng/ml TIMP1 and 10 ng/ml TGFβ1. To identify the role of β1 integrin in TIMP1-mediated fibroblast activation, β1 integrin was silenced in fibroblast cells by shRNA. After treatment, lung and liver fibroblast cells were retrieved for analysis of Smad2 phosphorylation and SMA protein expression by immunoblot. Smad2 phosphorylation and SMA protein expression were quantified by densitometry of immunoblot.

. **Mouse liver regeneration and repair models.** Mouse liver regeneration was induced by PH. Midline laparotomy was performed in the anaesthetized mice, and three most anterior lobes (right medial, left medial and left lateral lobes) were resected. After opening the abdomen and the exposure of liver, the left lobe to be removed was lifted. A 5-0 silk suture tie was placed under the lobe and positioned to the origin of the lobe. The tied lobe distal to the suture was cut, and this procedure was repeated for the other lobes to finish PH. Following surgical removal of 70% of liver mass, the skin was closed. Injections of CCl4 were used to induce liver injury as previously described (Reference 8). CCl₄ was diluted in oil (Sigma-Aldrich) to yield a concentration of 40% (0.64 mg/ml) and i.p. injected to mice at a dose of 1.6 g/kg. CCl₄ was diluted in oil (Sigma-Aldrich) to yield a concentration of 40% (0.64 mg/ml) and i.p. injected to mice at a dose of 1.6 g/kg. Mice were injected with 0.1 mg/kg APC, 50 mg/kg NRP1 inhibitor, 25 mg/kg HIF2α inhibitor, and 1.5 mg/kg 1560 every three days after PH and first CCl₄ injection. Regeneration of liver function was assessed by measuring serum levels of bilirubin and AST at indicated time points after PH (Reference 8).

. **Mouse kidney regeneration and repair models.** Mouse kidney I/R model was adopted from described (Reference 72). After mice were anesthetized, ischemia was induced by the retroperitoneal approach on both kidneys for 30 min. Sham operation was carried out with exposure of kidneys without inducing ischemia. To generate kidney fibrosis, mice were given a single i.p. injection of folic acid at the dose of 250 mg/kg, as previously described (Reference 74). Mice were treated 0.1 mg/kg APC, 50 mg/kg NRP1 inhibitor, 25 mg/kg HIF2α inhibitor, and 1.5 mg/kg 1560 every three days after kidney I/R and day 40 after folic acid injection. At indicated time, serum creatinine concentration was measured by the picric acid method, and tissue morphology and fibrosis were analyzed by Sirius red and hematoxylin co-stainings.

. **Injection of growth factor and treatment of HDAC and DNMT inhibitors.** Three months old (young) mice were subjected to PNX, PH or kidney I/R and injected with 20 µg/kg PIGF or equal molar amounts ofPDGF-BB, HGF, VEGF165, or TGFβ1 every three days. 3-mon *Nrp1*^{+/+}, *Nrp1*^{iΔEC/iΔEC}, or *Hif2a*^{iΔEC/iΔEC} mice were also treated with PIGF. To test the regulation of PlGF and NRP1, old mice were treated with 10 mg/kg HDAC inhibitor VPA, 0.5 mg/kg DNMT1 inhibitor azacitidine (AZA), or combinatorial treatment of VPA and AZA (VPA+AZA). Expression of EPCR and NRP1 proteins in isolated lung, liver, and kidney ECs were tested by immunoblot, and PIGF amounts in homogenized organs was tested by ELISA.

. **Immunofluorescence (IF) and morphometric analysis.** Mouse lung, liver and kidney tissues were harvested for histological analysis. 10 µm thick tissue cryosections were blocked (5% donkey serum/0.3% Triton X-100) and incubated in primary antibodies at 4°C overnight: anti-VE-cadherin polyclonal antibody (pAb, 2 µg/ml, R&D Systems, MN), anti-SMA antibody (pAb, 2 µg/ml, Abcam, CA), anti-EPCR monoclonal antibody (mAb 1560, 5 µg/ml), anti-Podoplanin (mAb, 5 µg/ml, R&D, MN), anti-aquaporin 5 (pAb, 5µg/ml, Abcam, CA), anti-CD41 (mAb, 5 µg/ml, BD Bioscience). After incubation in fluorophore-conjugated secondary antibodies (2.5 µg/ml, Jackson ImmunoResearch, PA), nuclear staining was carried out with DAPI using Prolong Gold mounting medium (Invitrogen). To determine the immunofluorescent staining signal in the prepared tissue sections, fluorescent cells in each slide were independently evaluated on five different high-power fields and quantified, representing the results for individual specimen.

. **Tissue fibrosis determination.** Lung, liver and kidney fibrotic responses were determined at the indicated time after injury. Tissues were homogenized in tissue lysis buffer. Immunoblot of Collagen I was performed with the obtained tissue lysates. Protein level of Collagen I was compared between different groups. Sirius red and hematoxylin stainings were performed on paraffin-embedded tissue sections to determine the tissue morphology and Collagen deposition and distribution (Reference 8). Sirius red-positive fibrotic parenchyma was determined from five random fields in each section and quantified. Hydroxyproline amount was quantified in the liver and lung to determine the extent of fibrosis.

. **Macrophage/monocyte depletion, isolation, and adoptive transfer.** Clodronate liposome method was used to selectively deplete macrophage/monocyte in mice (Reference 7). 30 µL of clodronate encapsulated in liposomes (Clophosome-A) or empty control liposomes (Formu Max, Palo Alto, CA) was intravenously (i.v.) injected into mice two days before PNX, PH, or Kid I/R and every ten days thereafter to deplete macrophage/monocyte during chronic lung injury. Macrophages/monocytes were isolated from indicated mouse tissues via antibody-coated beads isolation after PNX, PH, or Kid I/R. Expression of genes was examined by quantitative PCR. To examine the contribution of TIMP1-expressing macrophages/monocytes in organ repair, monocytes were isolated from bone marrow of *WT* or *Timp1*^{*-*/*-*} mice. 3 × 10⁶ *WT* and *Timp1*^{*-*/*-*} monocytes were i.v. infused to macrophage-depleted *WT* mice one day after PNX, PH, Kid I/R, or injection of CCl₄, Bleo, or folic acid, respectively. This adoptive transfer of monocyte was repeated after the first transplantation till sacrificing the recipient mice. Pulmonary, hepatic, renal functions and fibrosis were compared between different groups receiving *WT or Timp1*^{*-*/*-*} monocyte. Collagen deposition and morphological features were tested by Sirius red and hematoxylin stainings.

. **Mouse platelet adoptive transfer model.** Strategy to infuse platelets via jugular vein was adopted from previously described (Reference 24). Platelets were isolated and concentrated from mice with platelet-specific deletion of *Il1a* (*Il1a*^{ΔPlt/ΔPlt}) mice to obtain *Il1a*^{*-*/*-*} platelets. Platelets from wild type were utilized as control platelets. In brief, blood was harvested from anesthetized mice with syringes containing 0.5 ml ACD (12.5 g/L Na Citrate, 10.0 g/L D-glucose, and 6.85 g/L citric acid). Collected blood was transferred to 5 ml buffer containing 150 mM NaCl and 20 mM PIPES and then centrifuged at 100 g for 15 min. The supernatant enriched for platelets was collected after centrifugation. Supernatant was centrifuged again at 1000 g for 10 min. Obtained platelet pellet was re-suspended and counted. 2× 10⁹ platelets were infused through exposed jugular vein of recipient mice over a period of 15 minutes.

. **Image acquisition and analysis.** Fluorescent images were recorded on AxioVert LSM710 confocal microscope (Zeiss). Analysis of sections was recorded with Olympus BX51 microscope (Olympus America, NY). Densitometry analysis of immunoblot image was carried out using ImageJ software with calibrated standard curve.

. **Quantification and statistical analysis.** Calculations were carried out with Prism 8 software package (GraphPad). For datasets with more than two groups, one-way ANOVA followed by Tukey's post hoc test was employed to determine significant differences. The statistical details of experiments can be found in the legends. All data are presented as mean ± standard error of mean (SEM). Error bar shows SEM and center shows mean. p values < 0.05 were considered as statistically significant.

### . Embodiment 2: Regeneration to Fibrotic Transition in Old Mouse Lung Associates with Formation of Platelet-Macrophage Rosette and Reprogramming in ECs

. Experimenters of the present invention used pneumonectomy (PNX) to compare the lung alveolar regeneration and fibrosis in mice at different ages (FIG. 1A), including restoration of pulmonary function, epithelial architecture, and amounts of hydroxyproline (FIGs. 1B-C, FIGs. 8A-B). Compared with mice at the age of 2, 3, and 6 months, 20-month-old mice showed marked suppression of alveolar regeneration and increased fibrosis after PNX. Therefore, experimenters of the present invention defined 20-month-old mice as "old" mice and compared the regenerative capacity of old mice with that of mice at 2-month-old (2-mon) or 3-month-old (3-mon) age.

. Platelets and macrophages are important players in organ repair. For this reason, experimenters of the present invention stained the deposition of CD41⁺ platelets and F4/80⁺ macrophages in the mouse lungs after PNX (FIG. 1D). There was little deposition of platelets in the 2-mon and 3-mon mouse lungs after PNX. By contrast, platelets associated with macrophages in the old mouse lung, forming perivascular cellular rosettes (FIG. 8C). These data suggest that the maladapted interaction between platelets and macrophages might contribute to the regeneration to fibrotic transition in the aged lung.

. ECs lining vascular lumen form an anti-inflammatory and anti-thrombotic interface to maintain organ homeostasis. Thus, experimenters of the present invention hypothesized that reprogramming of key node molecules in ECs activate platelets and macrophages to promote fibrosis. To define the contribution of vascular ECs to aging-related fibrosis, experimenters of the present invention isolated pulmonary capillary endothelial cells (PCECs) from 2-month- and 20-month-old mice after PNX. On the basis of the transcription profiles (FIG. 1E), the expression of EPCR is compared, and EPCR is selectively expressed in ECs Neuropilin1 (NRP1) and HIF2α, both of which are induced in ECs by pathological conditions (FIG. 8D). EPCR expression was lower in old PCECs after PNX than in 2-, 3-, and 6-month-old mice. By contrast, expression of NRP1 and HIF2α was elevated in the PCECs of old mice after PNX. Thus, aged lung seems to exhibit changed angiocrine signaling after PNX.

### . Embodiment 3 : Normalizing NRP1-HIF2α-EPCR Circuit in ECs Attenuates Regeneration to Fibrotic Transition in Old Lung

. NRP1 is co-receptor for several cytokines. To dissect NRP1's functional contribution in aging-related fibrosis, experimenters of the present invention generated mice deficient of *Nrp1* specifically in EC (*Nrp1*^{iΔEC/iΔEC})*.* Floxed *Nrp1* mice were bred with mice expressing EC-specific VE-cadherin (Cdh5)-Cre^{ERT2}. Tamoxifen treatment of the offspring deleted *Nrp1* in ECs (FIG. 8E). Old *Nrp1*^{iΔEC/iΔEC} mice were subjected to PNX, and 3-mon (young) and old Nrp1^{+/+} mice served as controls. After PNX, old *Nrp1*^{iΔEC/iΔEC} mice manifested lower *Hif2α* expression and higher *Epcr* level in PCECs than in old control mice did (FIG. 1F, FIG. 8F). Deletion of endothelial *Nrp1* abrogated formation of platelet-macrophage rosette in the pneumonectomized old lung (FIG. 1G, FIG. 8G). Because EPCR expression is upregulated in old *Nrp1*^{iΔEC/iΔEC} mice, experimenters postulated that the decreased formation of perivascular hematopoietic rosette in old *Nrp1*^{iΔEC/iΔEC} mice relies on restoration of EPCR. Indeed, blockage of EPCR by its neutralizing antibody 1560 restored the formation of cellular rosette in old *Nrp1*^{iΔEC/iΔEC} mice, suggesting the epistatic role of NRP1 induction in EPCR suppression and subsequent activation of platelets and macrophages.

. To test this hypothesis, experimenters of the present invention characterized the regenerative and fibrotic responses in the lung of old *Nrp1*^{iΔEC/iΔEC} mice. After PNX, old *Nrp1*^{iΔEC/iΔEC} mice exhibited less collagen deposition, enhanced alveolar regeneration, reduced senescence and fibroblast activation, increased propagation of type 2 alveolar epithelial progenitor cells, and lower number of perivascular neutrophils, compared with those of old control mice (FIG. 1H, FIGs. 8H-I). Antibody 1560 abrogated the beneficial effects observed in old *rp1*^{iΔEC/iΔEC} mice. These data suggest that EPCR normalization in old *Nrp1*^{iΔEC/iΔEC} mice favors regeneration over fibrosis after PNX (FIG. 1I).

### . Embodiment 4: Reprogrammed Hematopoietic-Vascular Niche Subverts Regeneration to Fibrosis in Aged Liver and Kidney

. Liver regeneration was assessed in mice at different ages (FIG. 2A). After partial hepatectomy (PH), old mouse liver displayed fibrotic-like symptoms (FIG. 9A). Platelet-macrophage rosette was also observed in old mouse liver but not 3-month-old mouse liver after PH (FIG. 2B), and there were similar changes in NRP1, HIF2α, and EPCR expression in liver ECs (FIGs. 2C-D). After PH, old *Nrp1*^{iΔEC/iΔEC} mice exhibited increased liver cell proliferation and less fibrosis than old control mice did, which was associated with decreased formation of platelet-macrophage rosette (FIGs. 2E-I and FIG. 9B). Antibody 1560 injection to old *Nrp1*^{iΔEC/iΔEC} mice reversed their phenotype after PH, suggesting that NRP1-dependent suppression of EPCR activates platelets and macrophages in old mouse liver after PH.

. Acute kidney injury was induced in old control and *Nrp1*^{iΔEC/iΔEC} mice by ischemia and reperfusion (I/R) (FIG. 2J). Kidney function was impaired with fibrosis in old mice after I/R, which was accompanied by formation of platelet-macrophage rosette in the kidney of old but not 2-month-old mice (FIG. 2K). I/R similarly increased the expression of NRP1 and HIF2α and downregulated EPCR in old kidney ECs (FIGs. 2L-N). Old *Nrp1*^{iΔEC/iΔEC} mice showed improved kidney repair with higher degree of cell propagation, less fibrosis, and lower amounts of platelet-macrophage rosette than old control mice did (FIGs. 2O-R, FIG. 9C). Antibody 1560 reversed the phenotype of old *Nrp1*^{iΔEC/iΔEC} mice after kidney I/R, implicating that the beneficial effect deletion is dependent on the EPCR.

### . Embodiment 5: Epigenetic Upregulation of P1GF-NRP1 Axis Stimulates HIF2α to Suppress EPCR in ECs

. EPCR was induced in young mouse lung ECs after PNX, which was suppressed in aged lung ECs (FIG. 9D). Deletion of *Nrp1* in ECs restored EPCR induction in old pneumonectomized lung. NRP1 binds to various ligands, including placenta growth factor (PIGF), hepatocyte growth factor (HGF), platelet-derived factor BB (PDGF-BB), and TGF-β1. PIGF protein was upregulated in aged lung and liver after PNX and PH (FIG. 9E). Injection of PlGF but not other NRP1 ligands into young mice suppressed EPCR expression and enhanced HIF2a after PNX, resembling the phenotype of old mice (FIG. 9G). This effect of PlGF was abrogated in *Nrp1*^{iΔEC/iΔEC} mice (FIG. 9G). Deletion of *Hif2a* in EC (*Hif2a*^{iΔEC/iΔEC}) similarly increased EPCR expression in old lung ECs after PNX, and PIGF injection did not suppress EPCR in young *Hif2a*^{iΔEC/iΔEC} mice (FIG. 9G). In the liver lacking endothelial *Nrp1* or *Hif2a,* PlGF did not prohibit EPCR upregulation after PH (FIG. 9H). Thus, it is likely that PlGF-NRP1-Hif2α axis is responsible for EPCR suppression in aged organs after injury.

. Given that epigenetic change contributes to organ fibrosis, experimenters of the present invention tested the influence of histone modification and DNA methylation on endothelial NPR1 and PlGF expression. After PNX, old animals were treated with histone deacetylase (HDAC) inhibitor valproic acid (VPA), DNA methyltransferase (DNMT) inhibitor 5-azacitidine (AZA), or combinatorial treatment of VPA and AZA. VPA treatment in old mice after PNX reduced the expression of NRP1 in lung ECs, which was further decreased by co-treatment with AZA (VPA + AZA) (FIG. 9I). In old mice, VPA + AZA treatment prohibited upregulation of endothelial NRP1 in liver and kidney ECs after PH and kidney I/R (FIG. 9I). VPA + AZA also suppressed PlGF upregulation in old lung, liver, and kidney after PNX, PH, or kidney I/R (FIG. 9J). Thereby, NRP1 and PlGF expression are likely to be epigenetically regulated in aged organs (FIG. 9K).

### . Embodiment 6: NRP1-HIF2α Axis in PCECs Stimulates Stromal-Derived Factor 1 (SDF1) to Recruit Pro-fibrotic Macrophages

. PCECs produce paracrine or angiocrine factors to regulate lung alveolar repair. For this reason, experimenters of the present invention analyzed the expression of factors in lung ECs and fibroblasts from control and *Nrp1*^{iΔEC/iΔEC} mice (FIGs. 10A-B). Chemokine SDF1 showed the most suppression in PCECs of *Nrp1*^{iΔEC/iΔEC} mice. Because SDF1-CXCR4 signaling modulates hematopoietic cell functions and organ repair, experimenters of the present invention investigated the expression pattern of SDF1 in young and old *Sdf1* reporter mice (FIG. 3A). After PNX, SDF1 was induced in PCECs of old mice but not in a younger group, and deletion of *Nrp1* in ECs suppressed SDF1 expression (FIG. 3A).

. Experimenters of the present invention then tested whether HIF2α mediates SDF1 production in old ECs. Old *Hif2a*^{iΔEC/iΔEC} mice displayed lower levels of *Sdf1, Par1*, E-selectin, and VCAM1 after PNX (FIG. 3B). Given that SDF1, E-selectin, and VCAM1 promote EC-macrophage crosstalk, experimenters stained SDF1 receptor CXCR4 and macrophage marker. Compared with the lungs of old control mice, recruitment of CXCR4⁺ macrophages was decreased in old *Hif2a*^{iΔEC/iΔEC} and *Nrp1*^{iΔEC/iΔEC} mouse lungs after PNX, which was reversed by antibody 1560 (FIGs. 3C-D, FIGs. 10C-D). Proliferation of alveolar epithelial progenitor was enhanced in old *Hif2a*^{iΔEC/iΔEC} mice after PNX, and 1560 blocked the increase of cell proliferation (FIG. 3D, FIG. 10E). Old *Nrp1*^{iΔEC/iΔEC} mice showed less fibrosis after PH and kidney I/R (FIG. 10F), which was accompanied by lower expression of SDF1 and inflammatory genes in ECs (FIG. 10G). As a result, enrichment of CXCR4⁺ perivascular macrophages was attenuated in old *Hif2a*^{iΔEC/iΔEC} mice after PH and kidney I/R (FIG. 10H). Hence, recruitment of these perivascular CXCR4⁺ macrophages might contribute to the formation of pro-fibrotic platelet-macrophage rosette in old organs.

### . Embodiment 7: CXCR4⁺ Macrophages Suppress Regeneration and Provoke Fibrosis in Old Organs

. Pulmonary macrophages were isolated from old control and *Hif2a*^{iΔEC/iΔEC} mice after PNX (FIG. 3E). Tissue inhibitor of metalloproteinases 1 (TIMP1) differed markedly between old *Hif2a*^{iΔEC/iΔEC} and control mice. The fibrogenic function of TIMP1 was then defined. TIMP1 was shown to interact with β1 integrin involved in fibroblast activation. Thus, experimenters of the present invention tested the contribution of TIMP1-β1 integrin interaction to fibroblast activation. Incubation with TIMP1 augmented phosphorylation of Smad2 and smooth muscle cell actin (SMA) protein expression, which was further enhanced by co-treatment with TGF-β1. Smad2 phosphorylation and SMA upregulation by TIMP1 were attenuated by silencing of β1 integrin in fibroblast cells. These data imply that TIMP1 stimulates fibroblast activation via β1 integrin.

. The fibrogenic contribution of TIMP1 was tested in old mice lacking *Timp1* (Timp1^{-/-}). After PNX, old Timp1^{-/-} mice exhibited reduced lung fibrosis and enhanced restoration of alveolar epithelial coverage, as compared with that of control mice (FIG. 4A). TIMP1 expression was higher in macrophages from old mouse organs after PNX, PH, or kidney I/R, than in young organs (FIG. 4B, FIGs. 11A-C). Treatment of CXCR4 antagonist AMD3100 attenuated TIMP1 upregulation in the macrophages of mouse old organs. These findings imply that deregulation of NRP1-HIF2α-EPCR pathway in old ECs stimulates SDF1 to recruit CXCR4⁺ macrophages expressing TIMP1 (FIG. 10I).

. To unravel the role of CXCR4⁺TIMP1^{high} macrophages, experimenters of the present invention utilized an adoptive macrophage transfer approach (FIG. 4C). Transplantation of *Timpl*-null monocytes promoted the recovery of pulmonary function and decreased collagen deposition in pneumonectomized old mice (FIGs. 4D-G). After PH and kidney I/R, transfer of Timp1^{-/-} but not Timp1^{+/+} monocytes to old mice enhanced parenchymal function restoration and blocked fibrosis (FIGs. 4H-J). As such, reprogramming of **NRP1-HIF2α-EPCR** pathway in the EC of old organs induces SDF1 to recruit pro-fibrotic CXCR4⁺TIMP1^{high} macrophages, impeding regeneration and stimulating fibrosis (FIG. 4K).

### . Embodiment 8: Platelets Produce IL-1α to Activate Pro-fibrotic TIMP1⁺ Macrophages

. Platelets produce pro-inflammatory cytokine such as interleukins to interact with macrophages. To assess the contribution of platelet IL-1α, experimenters of the present invention generated mice with platelet-specific deletion of IL-1α (*Il1a*^{ΔPlt/ΔPlt}) (FIGs. 5A-B). Given that EPCR-neutralizing antibody 1560 induces fibrotic-like phenotype in young mice after PNX (FIGs. 12A-E), experimenters of the present invention used antibody 1560 to treat 3-mon *Il1a*^{ΔPlt/ΔPlt} mouse to establish the correlation between EPCR and platelet IL-1α. Deleting *Il1a* in platelet blocked the formation of platelet-macrophage rosette and reduced the pulmonary expression of pro-fibrotic TIMP1 in mice treated with EPCR monoclonal antibody (mAb) (FIGs. 5C-G). Transplantation of *Timp1*-deficient macrophages/monocytes to young mice injected with antibody 1560 also blocked lung fibrosis (FIGs. 12F-H). Hence, suppression of EPCR in old ECs leads to platelet IL-1α production and activation of pro-fibrotic TIMP1⁺ macrophages, causing fibrotic phenotypes.

. A platelet adoptive transfer model was used (Reference 52) (FIG. 5H). Old Thpo^{-/-} mice were injected with wild-type (Il1a^{+/+}) or *Il1a*-knockout (I11a^{-/-}) platelets and then underwent PNX, hepatectomy, or kid I/R. Expression of TIMP1 and fibrosis in the lung, liver, and kidney of recipient mice were assessed. Compared with old mice transferred with I11a^{+/+} platelets, old mice injected with I11a^{-/-} platelets exhibited reduced TIMP1 expression and less collagen deposition in tested organs (FIGs. 5I-K). These data imply the contribution of platelet IL-1α to the formation of pro-fibrotic platelet-macrophage rosette and TIMP1 expression in aged organs (FIG. 5L).

### . Embodiment 9: Normalizing Aberrant EC-Platelet Crosstalk Stimulates Regeneration over Fibrosis in Aged Organs

. Experimenters of the present invention used a bleomycin (Bleo) model as a lung fibrosis model. Old *Nrp1*^{iΔEC/iΔEC} and control mice were treated with intratracheal injection of bleomycin (FIG. 6A). PIGF was upregulated in old mouse lungs after Bleo (FIG. 6B), and old *Nrp1*^{iΔEC/iΔEC} mice showed EPCR upregulation and HIF2α suppression than old control mice (FIG. 6C), which was associated with reduced fibrosis, enhanced restoration of alveolar regeneration, decreased cell senescence, and attenuated recruitment of perivascular CXCR4⁺ macrophages (FIGs. 6D-F, FIGs. 13A-C). The phenotypes in old *Nrp1*^{iΔEC/iΔEC} mice were blocked by EPCR-neutralizing antibody, implicating that reprogrammed NRP1-EPCR pathway in ECs promotes lung fibrosis in old mice after Bleo injury.

. Contribution of platelets and macrophages to fibrosis in this Bleo model was tested. Old mice were transplanted with Il1a^{+/+} and Il1a^{-/-} platelets after Bleo injury (FIG. 6G). Transplantation of Il1a^{*-*/*-*} platelets mitigated formation of hematopoietic rosette and TIMP1 expression in the injured recipient lung (FIG. 6H). Antibody 1560 did not alter the effect of Il1a^{-/-} platelets. The differential effects of EPCR antibody on old *Nrp1*^{iΔEC/iΔEC} mice and *Il1a*^{ΔPLT/ΔPLT} mice suggests that NRP1 in aged ECs causes platelet IL-1α production. Old mice transplanted with Timp1^{-/-} monocytes or lacking *Timp1* showed restored lung alveolar regeneration and alleviated fibrosis (FIGs. 6I-K, FIGs. 13D-E). Thereby, reprogrammed **NRP1-HIF2α-EPCR** signaling in aged ECs activates IL-1α⁺ platelets and recruits TIMP1^{high} macrophages to form pro-fibrotic hematopoietic rosette (FIG. 6L).

. Immune checkpoint blockage such as anti-programed cell death protein 1 (PD1) antibody has shown remarkable clinical benefit in multiple cancer types. However, immunotherapy might have potential risk in subset of patients, causing immunotherapy-related adverse effects (IrAEs). Pneumonitis is one of common IrAEs after checkpoint blockage therapy. Experimenters of the present invention tested whether old mice are more susceptible to pulmonary fibrosis after PD1 antibody treatment, especially in conjunction with radiotherapy. Old and young mice were exposed to thoracic radiation with or without treatment of PD1 antibody 1 month after radiation to stimulate immunotherapy-induced pneumonitis (FIG. 7A). PD1 antibody treatment after thoracic radiation caused higher extent of lung fibrosis in old Hif2a^{+/+} mice than either 3-mon *Hif2a*^{+/+} mice or old *Hif2α*^{iΔEC/iΔEC} mice (FIGs. 7B-C). Mortality and lung fibrosis were attenuated in old *Hif2a*^{iΔEC/iΔEC} mice, which was reversed by EPCR antibody (FIGs. 7B-C). Old mice transplanted with Il1a^{*-*/*-*} but not Il1a^{+/+} platelets were protected from death and lung fibrosis (FIGs. 7D-F). By contrast, EPCR antibody did not affect this protection of Il1a^{-/-} platelets, suggesting the pro-fibrotic function of platelet IL-1α after checkpoint blockage is downstream of EPCR suppression. Therefore, the aberrant crosstalk between endothelial NRP1 and platelet IL-1α might contribute to immunotherapy-induced pneumonitis in old patients.

. The fibrogenic role of aberrant EC-platelet crosstalk in immunotherapy-induced pneumonitis was tested in tumor-bearing mice. After injection of lewis lung carcinoma (LLC) cells, old mice were transplanted with Il1a^{+/+} or Il1a^{-/-} platelets and subjected to PD1 antibody treatment (FIG. 7G). Recipient mice with Il1a^{-/-} platelets showed reduced tumor cell load and fibrosis in the lungs, as compared with mice injected with Il1a^{+/+} platelets (FIGs. 7H-I). Survival of recipient mice infused with Il1a^{-/-} platelets was improved compared with those infused with Il1a^{+/+} platelets (FIG. 7J). As such, Il1α from platelets might cause lung fibrosis and exacerbate mortality during immunotherapy for lung tumor.

. Young and old mice were subjected to injection of hepatotoxin carbon tetrachloride (CCl₄) or folic acid (Reference 2) to induce liver or kidney fibrosis (FIG. 7K). After injury, there was upregulation of PlGF in the old injured liver (FIG. 7L). EPCR suppression and HIF2α induction in EC of old control mice were restored in old *Nrp1*^{iΔEC/iΔEC} mice (FIG. 14A), which alleviated parenchymal injury and fibrosis in the injured livers and kidneys (FIGs. 7M-N, FIG. 14B). Transplantation of Il1a^{-/-}platelets similarly reduced formation of pro-fibrotic hematopoietic-vascular rosette and TIMP1 expression in the injured liver and kidney (FIG. 7O, FIG. 14C). There was less fibrosis in old Timp1^{-/-} mice or mice transplanted with Timp1^{-/-} monocytes than in old control mice after liver or kidney injury (FIG. 7P, FIGs. 14D-E). Therefore, normalizing the aberrant crosstalk between platelet-macrophage-vascular cells enables repair without fibrosis in the old lung, liver, and kidney.

### . Embodiment 10:

. Previous studies have shown that lung endothelial cells form a niche to initiate lung regeneration and prevent fibrosis (References 7, 17, 19, 52). Experimenters of the present invention show that in the damaged lung, liver and kidney, aberrant induction of neuropilin-1 in endothelial cells abnormally induces transcription factor HIF2 and suppresses EPCR. Disruption of EPCR protective pathway in endothelial niche causes sustained fibrosis in the injured lung, liver, and kidney. Genetic ablation or pharmacological blockage of Neuropilin-1 restored EPCR expression in the injured endothelial cells in lung, liver, and kidney.

. To reset the undermined pro-regenerative function of endothelial niche, experimenters of the present invention used a "two-hit" strategy by enabling induction of EPCR and injection of its molecular partner, activated protein C (APC). Coadministration of NRP-1/HIF2α inhibitor synergistically acted with APC to 1) abolish Rho activation in endothelial cells, 2) reduce deposition of inflammatory monocytes, and 3) stimulate a fibrosis-free repair in lung, liver, and kidney. This actually increases the expression of EPCR through the upstream pathway, which includes but is not limited to NRP1 and HIF2α. These data suggest the translational value of a "niche-reprogramming therapy" for fibrosis-related disorders, which includes administration of clinically utilized APC and enabling its receptor EPCR in endothelial niche. Through the "two-hit" technical solution proposed by the experimenters of the present invention, on the one hand, it activates EPCR and inhibits NRP1 and/or HIF2α to increase EPCR expression and enhance its efficacy. On the other hand, it can reduce the required dosage of APC (compared with using APC alone to activate the EPCR signaling pathway), thereby reducing the occurrence of side effects.

. The embodiment proposes a model of role of the APC-EPCR pathway in promoting fibrosis-free organ repair (FIG. 15). Summary of the model is: after lung injury, EPCR on lung endothelial cells serves to promote lung regeneration and bypass lung fibrosis. Persistent lung damage activates Neuropilin1 (Nrp1) pathway in endothelial cells to upregulate HIF2α, which suppresses EPCR and upregulates PAR1. Disruption of EPCR pathway in endothelial niche overturns regenerative response to lung fibrosis. Therapeutically, Nrp1 inhibitor or HIF2α inhibitor restores EPCR expression, which acts together with administration of activated protein C (APC) to re-instate protective APC-EPCR pathway. These data implicate that reprogramming a pro-regenerative EPCR⁺ endothelial niche might enable a fibrosis-free repair in the lung, liver, and kidney.

. Contribution of EC reprogramming to lung fibrosis was tested in a repeated lung injury model (Reference 76). Hydrochloric acid (Acid) was instilled into mouse trachea every ten days for five injections (FIG. 16A). The injured lungs recovered gas exchanging function after three Acid injections. By contrast, gas exchanging function recovery was suppressed after the 5^{th} injection (FIG. 16B). Suppressed lung function recovery was associated with persistent fibrosis (FIGs. 16C-D). Notably, EPCR induction in PCECs was associated with lung function restoration, and NRP1, HIF2α and PAR1 were upregulated in PCECs when fibrosis occurred (FIGs. 16E-1). *Nrp1*^{iΔEC/iΔEC} mice showed higher EPCR expression and lower HIF2α and PAR1 expression in PCECs than that of control mice after 5^{th} Acid injection (FIGs. 17A-B). Hence, repeated lung injury leads to reprogramming of NRP1- HIF2α-EPCR signaling in PCECs.

. Subsequently, whether targeting of reprogrammed PCECs by injection ofAPC and EG (APC + EG) will reinstate lung repair and reduce fibrosis was tested (FIG. 17C). APC + EG most efficaciously restored lung function and reduced fibrosis after repeated Acid injury among all tested groups (FIGs. 17D-G). Recruitment of perivascular macrophages was suppressed in PCECs by APC + EG treatment after repeated lung injury (FIGs. 17F-G). APC was previously shown to inhibit Rho signaling in ECs. Indeed, phosphorylation of myosin light chain (pMLC) was blocked in PCECs after APC + EG treatment (FIG.s 17F-G). Therefore, co-injection of APC and NRP1 inhibitor, a double hit strategy, stimulates the normalized EPCR signaling in chronically injured PCECs and decreases recruitment of pro-fibrotic perivascular macrophages.

. Therapeutic effect of this double hit strategy was then tested in mouse liver and kidney chronic injury models (FIG. 18A). Mice were subjected to repeated injection of hepatotoxin carbon tetrachloride (CCl₄) or folic acid injection (Reference 77). EPCR was induced in ECs after first CCl₄ injection or at day 10 after folic acid injection. By contrast, EPCR expression was blocked and NRP1 and HIF2A were stimulated in ECs after 8^{th} CCl₄ injection and 100 days after folic acid injection (FIG. 19A). Treatment of *Nrp1*^{iΔEC/iΔEC} mice with APC suppressed HIF2A induction, enhanced EPCR expression in both liver and kidney ECs after chronic injury (FIG. 19B), suggesting a pro-regenerative function of EPCR in both liver and kidney repair.

. Then, the mice were treated with APC, EG or HIF2α inhibitor HIF-2α-IN-1 (HY-19949), and mice were assessed after 8th CCl₄ injection or 100 days after folic acid injection. APC + EG suppressed expression of *Sdf1*, *Par1*, *Vcam1*, and *Hif2a* in liver and kidney ECs (FIG. 18B) and reduced hepatic fibrosis (FIG. 19C). Co-injection of APC and HY-19949 (APC + HY) blocked recruitment of perivascular CXCR4⁺ macrophages and suppressed parenchymal injury and fibrosis in injured liver and kidney (FIG. 18C, FIGs. 19D-G). Moreover, after fibrotic liver and kidney injury, APC + EG blocked SDF1 expression in ECs of

. SDF1-mCherry reporter mice (FIG. 18D) and enhanced restoration of parenchymal function (FIGs. 18E-F). All these beneficial effects were blocked by injection of EPCR neutralizing antibody 1560. Therefore, targeting of reprogrammed NRP1-HIF2α-EPCR (which can also be understood as targeting reprogrammed ECs in the liver and kidneys) by double hit strategy (APC + NRP1/HIF2α inhibitor) enables fibrosis-free repair in chronically injured liver and kidney. The above experiments show that activation of normalized EPCR signaling in the vascular rosette promotes fibrosis-free repair of multiple organs. It should be noted that from the experimental results of blocking EPCR signals such as FIGs. 17-19, it can be seen that EPCR neutralizing antibody 1560 (a way of blocking EPCR signaling) weakens the beneficial technical effects that APC should achieve. Therefore, activating the EPCR signaling pathway is the key to the technical solution of the present invention. For those skilled in the art, any substance that can stimulate the EPCR signaling and/or promote the expression of EPCR should be within the protection scope of the present invention and is not limited to use APC.

### . Conclusion

. Aged organs are susceptible to fibrosis after injury. In the above-mentioned embodiments, experimenters of the present invention show that aging-reprogramed crosstalk between platelets, macrophages, and vascular ECs results in loss of regenerative capacity and fibrogenesis. Release of IL-1α from platelets was found to stimulate the pro-fibrotic function of macrophages. Changes in hemodynamics, glucose, and lipid can all lead to IL-1α release. Results of research of the present invention suggest that in the injured old organs, activation of platelets and production of IL-1α can be at least partially attributable to suppression of anti-thrombotic EPCR signaling in aged ECs. Therefore, loss of multifunctional EPCR signaling in the vascular niche might be a contributor to aging-related regeneration to fibrotic transition. EPCR has pleiotropic functions in modulating thrombosis, inflammation, and development. Data in the present invention show that EPCR induction is suppressed by aberrantly activated NRP1-HIF2α pathway in aged ECs.

. Platelets can be hierarchically activated to exert distinct pathophysiological effects. Activated platelets supply various mediators to initiate the regeneration of lung and liver after pneumonectomy or hepatectomy. Experimenters of the present invention previously used pneumonectomy and hepatectomy to demonstrate the function of SDF1 in promoting lung and liver regeneration. One important feature of pneumonectomy and hepatectomy models is that these procedures induce physiological regeneration, which does not involve significant inflammation and thrombosis in normal young mice. In physiological regeneration, SDF 1 might mainly stimulate its receptors on vascular endothelial or epithelial cells/hepatocytes, promoting epithelial and endothelial propagation. Therefore, after pneumonectomy or hepatectomy in young mice, SDF1 causes vascular and epithelial/hepatocyte regeneration. By contrast, in aged animals with EPCR suppression, pneumonectomy or hepatectomy procedure is associated with thrombosis and inflammation. Under these conditions, SDF1 enhances recruitment and accumulation of CXCR4⁺ macrophages producing pro-fibrotic TIMP1. As such, in the absence of overt inflammation and thrombosis, SDF1 might mainly act on epithelial and ECs to promote regeneration. In settings with higher levels of inflammation and thrombosis, SDF1 activates CXCR4 in inflammatory cells such as macrophages, producing fibrogenic factors such as TIMP1.

. The cellular mechanism revealed in the present invention might help to normalize the aberrant activation of immune cells and bypass their pro-fibrotic function in clinical settings. For example, this mechanism can be used to combat IrAEs. The present invention found that targeting of platelet IL1α or endothelial HIF2α improved survival and alleviated lung fibrosis in old mice treated with PD1 blockage. These data suggest that pneumonitis and fibrosis might be risk factors in old patients after immunotherapy, especially in conjunction with chemotherapy or radiotherapy.

. The present invention shows that aging-associated aberrant reprogrammed crosstalk between platelets, macrophages, and vascular cells in aged organs to reverse regeneration into fibrosis. The mechanisms leading to organ fibrosis revealed by the present invention, however, are not solely aging-specific. Normalization of the pro-fibrotic hematopoietic-vascular niche may help to restore regenerative capacity under pathological conditions. In reprogrammed ECs, genetic and pharmacological targeting of Neuropilin-1 or HIF2a normalizes EPCR signaling, which synergistically with the EPCR agonist, activator protein C (APC), to inhibit the recruitment of pro-fibrotic macrophages in aged and chronically injured organs. Normalization of the dysregulated vascular and immune niche may restore a certain extent of regenerative capacity in aged or fibrotic organs.

. The embodiments of the present invention are described above with reference to the accompanying drawings, but the present invention is not limited to the aforementioned specific embodiments. The aforementioned embodiments are merely illustrative and not limiting. For those of ordinary skill in the art, many forms can be made under the teaching of present invention without departing from the spirit of the present invention and the scope of the claims, all of which shall fall within the protection scope of the present invention.

### . Reference

1. Organotypic vasculature: From descriptive heterogeneity to functional pathophysiology. 2017, Science 357*.*
2. Methylation determines fibroblast activation and fibrogenesis in the kidney. 2010, Nature medicine 16, 544-550.
3. A vascular niche and a VEGF - Nrp 1 loop regulate the initiation and sternness of skin tumours. 2011, Nature 478, 399-403.
4. Platelets: covert regulators of lymphatic development. 2010, Arteriosclerosis, thrombosis, and vascular biology 30, 2368-2371.
5. Vascular endothelial growth factor-B acts as a coronary growth factor in transgenic rats without inducing angiogenesis, vascular leak, or inflammation. 2010, Circulation 122, 1725-1733.
6. Angiogenesis and vascular functions in modulation of obesity, adipose metabolism, and insulin sensitivity. 2013, Cell Metab 18, 478-489.
7. Targeting of the pulmonary capillary vascular niche promotes lung alveolar repair and ameliorates fibrosis. 2016, Nature medicine 22, 154-162.
8. Targeting the vascular and perivascular niches as a regenerative therapy for lung and liver fibrosis. 2017, Science translational medicine 9.
9. Molecular mechanisms and clinical applications of angiogenesis. 2011, Nature 473, 298- 307.
10. Impeding macrophage entry into hypoxic tumor areas by Sema3A/Nrpl signaling blockade inhibits angiogenesis and restores antitumor immunity 2013, Cancer cell 24, 695-709.
11. Activated protein C blocks p53-mediated apoptosis in ischemic human brain endothelium and is neuroprotective. 2003, Nat Med 9, 338-342.
12. Targeting HIF2 in Clear Cell Renal Cell Carcinoma. 2016, Cold Spring Harbor symposia on quantitative biology 81, 113-121.
13. Role of endothelial cell metabolism in vessel sprouting. 2013, Cell Metab 18, 634-647.
14. Macrophage regulation of tumor responses to anticancer therapies. 2013, Cancer Cell 23, 277-286.
15. Alveolar progenitor and stem cells in lung development, renewal and cancer. 2014, Nature.
16. Translational strategies and challenges in regenerative medicine. 2014, Nature medicine 20, 814-821.
17. Divergent angiocrine signals from vascular niche balance liver regeneration and fibrosis. 2014, Nature 505, 97-102.
18. Inductive angiocrine signals from sinusoidal endothelium are required for liver regeneration. 2010, Nature 468, 310-315.
19. Endothelial-derived angiocrine signals induce and sustain regenerative lung alveolarization. 2011, Cell 147, 539-553.
20. Regulation of hypoxia-inducible factor 2alpha signaling by the stress-responsive deacetylase sirtuin 1. 2009, Science 324, 1289-1293.
21. Distinct mechanisms regulate TIMP-1 expression at different stages of phorbol ester-mediated differentiation of U937 cells 1997, Biochemistry 36, 2492-2500.
22. Molecular circuits in thrombosis and inflammation. 2013, Thromb Haemost 109, 416-420.
23. Therapy for fibrotic diseases: nearing the starting line. 2013, Science translational medicine 5, 167sr161.
24. Infusion of mature megakaryocytes into mice yields functional platelets. 2010, The Journal of clinical investigation 120, 3917-3922.
25. Migrating Platelets Are Mechano-scavengers that Collect and Bundle Bacteria. 2017, Cell 171, 1368-1382.
26. Activated PMN Exosomes: Pathogenic Entities Causing Matrix Destruction and Disease in the Lung. 2019, Cell 176, 113-126.
27. Macrophage heterogeneity in tissues: phenotypic diversity and functions. 2014, Immunol Rev 262, 36-55.
28. Semaphorin 3E and plexin-D1 control vascular pattern independently of neuropilins. 2005, Science 307, 265-268.
29. PARI signaling regulates the retention and recruitment of EPCR-expressing bone marrow hematopoietic stem cells 2015, Nat Med 21, 1307-1317.
30. Reversal of persistent fibrosis in aging by targeting Nox4-Nrf2 redox imbalance. 2014, Science translational medicine 6, 231ra247.
31. Targeting of alphav integrin identifies a core molecular pathway that regulates fibrosis in several organs. 2013, Nature medicine 19, 1617-1624.
32. Heterotypic interactions enabled by polarized neutrophil microdomains mediate thromboinflammatory injury. 2009, Nature medicine 15, 384-391.
33. Repair and regeneration of the respiratory system: complexity, plasticity, and mechanisms of lung stem cell function. 2014, Cell stem cell 15, 123-138.
34. Activated protein C protects against diabetic nephropathy by inhibiting endothelial and podocyte apoptosis. 2007, Nat Med 13, 1349-1358.
35. Cytokine-mediated deployment of SDF-1 induces revascularization through recruitment of CXCR4+ hemangiocytes. 2006, Nature medicine 12, 557-567.
36. Targeting CXCR4-dependent immunosuppressive Ly6Clow monocytes improves antiangiogenic therapy in colorectal cancer. 2017, Proceedings of the National Academy of Sciences of the United States of America 114, 10455- 10460.
37. Vascular and neurogenic rejuvenation of the aging mouse brain by young systemic factors. 2014, Science 344, 630-634.
38. HIF1alpha and HIF2alpha: sibling rivalry in hypoxic tumour growth and progression. 2011, Nat Rev Cancer 12, 9-22.
39. Lung regeneration: mechanisms, applications and emerging stem cell populations. 2014, Nature medicine 20, 822-832.
40. Perivascular Gli1+ progenitors are key contributors to injury-induced organ fibrosis. 2015, Cell stem cell 16, 51-66.
41. Age-dependent modulation of vascular niches for haematopoietic stem cells. 2015, Nature 532, 380-384.
42. Aggravation of viral hepatitis by platelet-derived serotonin. 2008, Nature medicine 14, 756-761.
43. Recruited Monocytes and Type 2 Immunity Promote Lung Regeneration following Pneumonectomy. 2017, Cell Stem Cell 21, 120-134 e127.
44. The lung is a site of platelet biogenesis and a reservoir for haematopoietic progenitors. 2017, Nature 544, 105-109.
45. Platelet-derived serotonin mediates liver regeneration. 2006, Science 312, 104-107.
46. Overexpressing endothelial cell protein C receptor alters the hemostatic balance and protects mice from endotoxin. 2005, J Thromb Haemost 3, 1351- 1359.
47. WNT7b mediates macrophage-induced programmed cell death in patterning of the vasculature. 2005, Nature 437,417-421.
48. Platelet-macrophage partnership in innate immunity and inflammation. 2013, Nature immunology 14, 768-770.
49. Peptidylarginine deiminase 4 promotes age-related organ fibrosis. 2017, The Journal of experimental medicine 214, 439-458.
50. Pulmonary fibrosis: patterns and perpetrators. 2012, The Journal of clinical investigation 122, 2756-2762.
51. Platelet-derived SDF-1 primes the pulmonary capillary vascular niche to drive lung alveolar regeneration. 2015, Nature cell biology 17, 123-136.
52. Functional significance of the platelet immune receptors GPVI and CLEC-2. 2019, The Journal of clinical investigation 129, 12-23.
53. Endothelium-microenvironment interactions in the developing embryo and in the adult. 2007, Developmental cell 12, 181-194.
54. Activation of endothelial cell protease activated receptor 1 by the protein C pathway. 2002, Science 296, 1880-1882.
55. A Paradigm Shift in Cancer Immunotherapy: From Enhancement to Normalization. 2018, Cell 175, 313-326.
56. Platelet-derived nucleotides promote tumor-cell transendothelial migration and metastasis via P2Y2 receptor. 2013, Cancer cell 24, 130-137.
57. Restoring systemic GDF11 levels reverses age-related dysfunction in mouse skeletal muscle. 2014, Science 344, 649-652.
58. Targeting placental growth factor/neuropilin 1 pathway inhibits growth and spread of medulloblastoma. 2013, Cell 152, 1065-1076.
59. VEGF modulates erythropoiesis through regulation of adult hepatic erythropoietin synthesis. 2006, Nature medicine 12, 793-800.
*60.* Dedifferentiation of committed epithelial cells into stem cells in vivo. 2013, Nature 503, 218-223.
61. Timpl interacts with beta-1 integrin and CD63 along melanoma genesis and confers anoikis resistance by activating PI3-K signaling pathway independently of Akt phosphorylation. 2013, Molecular cancer 12, 22.
62. Lineage-negative progenitors mobilize to regenerate lung epithelium after major injury. 2015, Nature 517, 621- 625.
63. Regenerative activity of the lung after epithelial injury. 2013, Biochimica et biophysica acta 1832, 922-930.
64. Hepatocyte TAZ/WWTR1 Promotes Inflammation and Fibrosis in Nonalcoholic Steatohepatitis. 2016, Cell Metab 24, 848-862.
65. Ephrin-B2 controls VEGF-induced angiogenesis and lymphangiogenesis. 2010, Nature 465, 483-486.
66. Endothelial expression of hypoxia-inducible factor 1 protects the murine heart and aorta from pressure overload by suppression of TGF-beta signaling 2012, Proceedings of the National Academy of Sciences of the United States of America 109, E841-850.
67. Hierarchical organization of the hemostatic response to penetrating injuries in the mouse macrovasculature. 2017, Journal of thrombosis and haemostasis: JTH 15, 526-537.
68. FOXO1 couples metabolic activity and growth state in the vascular endothelium. 2016, Nature 529, 216-220.
69. Nucleation of platelets with blood-borne pathogens on Kupffer cells precedes other innate immunity and contributes to bacterial clearance. 2013, Nature immunology 14, 785-792.
70. Macrophage biology in development, homeostasis and disease. 2013, Nature496, 445-455.
71. Mechanisms of fibrosis: therapeutic translation for fibrotic disease. 2012, Nature medicine 18, 1028-1040.
72. Epithelial cell cycle arrest in G2/M mediates kidney fibrosis after injury. 2010, Nature medicine 16, 535-543, 531p following 143.
73. Endothelial-to-mesenchymal transition contributes to cardiac fibrosis. 2007, Nature medicine 13, 952-961.
74. BMP-7 counteracts TGF-beta1-induced epithelial-to-mesenchymal transition and reverses chronic renal injury. 2003, Nat Med 9, 964-968.
75. The metabolic regulation of aging. 2015, Nature medicine 21, 1416-1423.
76. Neutrophils promote alveolar epithelial regeneration by enhancing type II pneumocyte proliferation in a model of acid-induced acute lung injury. American journal of physiology. 2016, Lung cellular and molecular physiology 311, L1062-L1075.
77. Methylation determines fibroblast activation and fibrogenesis in the kidney. 2010, Nat Med 16, 544-550.

## Claims

1. An anti-fibrosis pharmaceutical composition, comprising:
(a) an NRP1 inhibitor and/or an HIF2α inhibitor;
(b) an EPCR pathway activator, the EPCR pathway activator promotes activity of the EPCR pathway.

2. The pharmaceutical composition according to claim 1, wherein the NRP1 inhibitor comprises one or more of a substance for inhibiting NRP1 protein activity, a substance for degrading NRP1 protein activity, and a genetic tool for reducing NRP1 protein level; the HIF2α inhibitor comprises one or more of a substance for inhibiting HIF2α protein activity, a substance for degrading HIF2α protein activity, and a genetic tool for reducing HIF2α protein level.

3. The pharmaceutical composition according to claim 1, wherein the NRP 1 inhibitor comprises EG00229 or a derivative thereof; the HIF2α inhibitor comprises HIF-2α-IN-1 or a derivative thereof.

4. The pharmaceutical composition according to claim 1, wherein the EPCR pathway activator comprises activated protein C.

5. The pharmaceutical composition according to claim 1, wherein the fibrosis comprises organ fibrosis, and the organ fibrosis is optionally aging-related organ fibrosis.

6. The pharmaceutical composition according to claim 5, wherein the organ fibrosis comprises one or more of liver fibrosis, kidney fibrosis, pulmonary fibrosis, and cardiac fibrosis, and the pulmonary fibrosis is optionally fibrosis caused by immunotherapy-induced pneumonitis.

7. The pharmaceutical composition according to claim 2, wherein the genetic tool for reducing NRP1 protein level comprises one or more of RNA interference, microRNA, gene editing, and gene knockout; the genetic tool for reducing HIF2α protein level comprises RNA interference, microRNA, gene editing, and gene knockout.

8. The pharmaceutical composition according to any one of claims 1 to 7, wherein the composition further comprises a pharmaceutically acceptable carrier.

9. Use of the pharmaceutical composition according to any one of claims 1 to 8 in the preparation of an anti-fibrotic agent.

10. The use according to claim 9, wherein the use comprises reducing fibrosis degree of damaged organ and/or promoting regeneration capacity of damaged organ, the regeneration capacity comprises one or more of cell proliferation capacity, tissue repair capacity, and function restoration capacity; the tissue repair capacity is optionally fibrosis-free repair, and the organ optionally comprises one or more of liver, kidney, lung, and heart.

11. The use according to claim 9, wherein the use comprises reducing formation of platelet-macrophage rosette; and/or reducing expression of one or more of platelet IL-1α, SDF1, and TIMP1.

12. The use according to claim 9, wherein the use comprises reducing Rho pathway activity; and/or reducing deposition of inflammatory monocytes; and/or promoting pro-regenerative function of endothelial niche.

13. Use of an EPCR pathway activator in the preparation of an anti-fibrotic agent, the EPCR pathway activator promotes activity of the EPCR pathway.

14. The use according to claim 13, wherein the EPCR pathway activator comprises activated protein C.

15. The use according to claim 13, wherein the fibrosis comprises organ fibrosis, the organ fibrosis is optionally aging-related organ fibrosis.

16. The use according to claim 15, wherein the organ fibrosis comprises one or more of liver fibrosis, kidney fibrosis, pulmonary fibrosis and cardiac fibrosis, and the pulmonary fibrosis is optionally fibrosis caused by immunotherapy-induced pneumonitis.

17. The use according to claim 13, wherein the use comprises reducing fibrosis degree of damaged organ and/or promoting regeneration capacity of damaged organ, the regeneration capacity comprises one or more of cell proliferation capacity, tissue repair capacity, and function restoration capacity.

18. The use according to claim 13, wherein the use comprises reducing formation of platelet-macrophage rosette; and/or reducing expression of one or more of platelet IL-1α, SDF1, and TIMP1.

19. The use according to any one of claims 13 to 18, wherein the EPCR pathway activator is used in combination with an NRP1 inhibitor and/or an HIF2α inhibitor to prepare the anti-fibrotic agent, and the NRP1 inhibitor and/or HIF2α inhibitor are/is used for promoting expression of EPCR.

20. The use according to claim 19, wherein the NRP 1 inhibitor comprises one or more of a substance for inhibiting NRP 1 protein activity, a substance for degrading NRP1 protein activity, and a genetic tool for reducing NRP1 protein level; the HIF2α inhibitor comprises one or more of a substance for inhibiting HIF2α protein activity, a substance for degrading HIF2α protein activity, and a genetic tool for reducing HIF2α protein level.

21. The use according to claim 19, wherein the NRP1 inhibitor comprises EG00229 or a derivative thereof; the HIF2α inhibitor comprises HIF-2α-IN-1 or a derivative thereof.

22. The use according to claim 19, wherein the substance for inhibiting NRP1 protein activity comprises a small molecule drug and/or an antibody; the substance for inhibiting HIF2α protein activity comprises a small molecule drug and/or an antibody.

23. The use according to claim 20, wherein the genetic tool for reducing NRP1 protein level comprises one or more of RNA interference, microRNA, gene editing, and gene knockout; the genetic tool for reducing HIF2α protein level comprises RNA interference, microRNA, gene editing, and gene knockout.
